# EUROPEAN PATENT SPECIFICATION

(11) **EP 3 433 365 B1**
(45) Date of publication and mention of the grant of the patent: **02.08.2023**
(21) Application number: 17770984.7
(22) Date of filing: 21.03.2017
(51) Int. Cl.: C12N 15/113, C12N 15/63, C12N 15/79, C12N 9/16, C12N 9/22

(54) **T-CELL EXHAUSTION STATE-SPECIFIC GENE EXPRESSION REGULATORS AND USES THEREOF**
T-ZELL-ERSCHÖPFUNGSZUSTANDSSPEZIFISCHE GENEXPRESSIONSREGULATOREN UND VERWENDUNGEN DAVON
RÉGULATEURS DE L'EXPRESSION GÉNIQUE SPÉCIFIQUES À L'ÉTAT D'ÉPUISEMENT DES LYMPHOCYTES T ET LEURS UTILISATIONS

(30) Priority: 21.03.2016 US 201662310903 P; 23.09.2016 US 201662398948 P
(43) Date of publication of application: 30.01.2019
(73) Proprietor: Dana-Farber Cancer Institute, Inc., Boston, MA 02215-5450 (US); The Trustees of The University of Pennsylvania, Philadelphia, PA 19104 (US); The Regents of the University of California, Oakland, CA 94607 (US); Haining, William, N., Newton, MA 02459 (US); Sen, Debattama, Cambridge, MA 02139-1255 (US); Wherry, E., John, Havertown, PA 19083 (US); Yosef, Nir, Richmond, CA 94805 (US); Kaminski, James, Berkeley, CA 94702 (US); Pauken, Kristen, Jamaica Plain, MA 02130 (US)
(72) Inventor: HAINING, William, Nicholas, Berkeley, CA 94702 (US); SEN, Debattama, Berkeley, CA 94702 (US); WHERRY, E., John, Berkeley, CA 94702 (US); YOSEF, Nir, Berkeley, CA 94702 (US); KAMINSKI, James, Berkeley, CA 94702 (US); PAUKEN, Kristen, Jamaica Plain, MA 02130 (US)
(74) Representative: Hoefer & Partner Patentanwälte mbB
(86) International application number: PCT/US2017/023404
(87) International publication number: WO 2017/165412

(56) References cited:
- WO-A2-2015/161276
- E. JOHN WHERRY ET AL: "Molecular and cellular insights into T cell exhaustion", NATURE REVIEWS IMMUNOLOGY, vol. 15, no. 8, 24 July 2015 (2015-07-24), pages 486-499, XP055339794, GB ISSN: 1474-1733, DOI: 10.1038/nri3862
- OESTREICH ET AL.: 'NFATcl regulates PD-1 expression upon T cell activation' J IMMUNOL vol. 181, no. 7, 01 October 2008, pages 4832 - 4839, XP055440502
- KAO ET AL.: 'Transcription factor T-bet represses expression of the inhibitory receptor PD-1 and sustains virus-specific CD 8+ T cell responses during chronic infection' NAT IMMUNOL vol. 12, no. 7, 29 May 2011, pages 663 - 671, XP055440503
- SCHUMANN ET AL.: 'Generation of knock-in primary human T cells using Cas9 ribonucleoproteins' PROC NAT ACAD SCI vol. 1112, no. 33, 18 August 2015, pages 10437 - 10442, XP055277999
- SEN ET AL.: 'The epigenetic landscape of T cell exhaustion' SCIENCE vol. 354, no. 6316, 27 October 2016, pages 1165 - 1169, XP005440507

## Description

### Cross-Reference to Related Applications

This application claims the benefit of U.S. Provisional Application No. 62/310,903, filed on 21 March 2016, and U.S. Provisional Application No. 62/398,948, filed on 23 September 2016.

### Statement of Rights

This invention was made with government support under grant numbers AI115712, AI091493, AI082630, MH105979, and HG007910 awarded by The National Institutes of Health. The government has certain rights in the invention.

### Background of the Invention

T cell exhaustion, which is an acquired state of T cell dysfunction, is a hallmark of cancer and chronic viral infection (Wherry et al. (2007) Immunity 27:670-684; Zajac et al. (1998) J. Exp. Med. 188:2205-2213). Recently, treatments to reverse T cell exhaustion in cancer have proven strikingly effective (Barber et al. (2006) Nature 439:682-687; Topalian et al. (2012) New Engl. J. Med. 366:2443-2454). Chimeric antigen receptor (CAR)-T cell therapy has also proven highly effective for hematologic malignancies (Porter et al. (2011) New Engl. J. Med. 365:725-733), but the development of exhaustion in engineered T cells to treat solid tumors remains a significant barrier to its broader use (Long et al. (2015) Nat. Med. 21:581-590). Identifying mechanisms that regulate T cell exhaustion could improve the efficacy immune checkpoint blockade and adoptive T cell therapy for cancer immunotherapy (Barber et al. (2006) Nature 439:682-687; Topalian et al. (2012) New Engl. J. Med. 366:2443-2454; Porter et al. (2011) New Engl. J. Med. 365:725-733). However, current strategies for modulating T cell exhaustion rely on directly modulating expression of effector gene expression products, such as immune checkpoints, and such modulation produces undesired side effects since physiological levels of such effector gene expression products are often required for normal T cell function. In addition, such strategies are vulnerable to drug resistance and can lead to immunopathology. Accordingly, there is a great need in the art to identify compositions and methods useful for modulating expression of effector gene expression products in T cells or cells that affect T cells that preserves physiologically relevant levels of such gene expression products. E.J.Wherry and M.Kurachi (Nat. Rev. Immunol. (2015) 15:486) describe molecular and cellular insights into T cell exhaustion in chronic infections and cancer wherein exhausted T cells lose functions and change their transcriptional programme. The molecular context is described and therapeutic aspects are discussed. The claimd invention is not disclosed nor suggested by this publication.

### Summary of the Invention

The present invention is directed to an engineered mammalian T cell comprising a genomic region that 1) regulates the gene expression of *pdcd1* and 2) is selectively chromatin accessible within the genome of an exhausted CD8+ T cell from the mammal, wherein the genomic region is genetically modified to delete the region corresponding to the nucleic acid sequence of SEQ ID NO: 12, which is tctccccaag ggagccattt ctttcaccct ttgtctctgc tctttctcct cacacttttg tttttgttat ggtctgacac cccccccccc ccaatctctc acccccagta gctgtggccg ttttgtccat gccagccagc tatttcatat cgttgctgta acgtgcttgc cagtcactga cacagagaag tgacttggct caaggatgta ctgaccacat aacctgtttt gttctgtacg ctctgaatgt tctgtatgag gtttgctaat cttaagaaat tccacaaagc tttacgcagg acccatcaaa tcaaacgtca atatgaactg ttatgtctaa aacggcttgg gtcagaccac cgggcaacct ttcctgtgcc tacgtctgag ctcactgtgg tttttgtggt tgacactgaa aaatgctact aataagccaa aaaatatgat tataatatgc atgttctgtt atctcaatgt tctgaaattc ttctgttcac cacccaacca ccaccactct taccttactc agaaccaatc agcttaaagg ttaactgata atatttcacc cagtaaacca acttctcccc tctttaccct ttaaactttt gagcttttct catataaaat gcctacactg agagcagact agtaccatcg ccaggctcct gcagtccctt ctgtggtcct agatgcccag cactatggtg tgcattcaat aaagtattct tgcttaactg agatcagggt ttgtttggtt tgaggggtga ctccctgaac c, that is -23.8kb upstream of the *pdcd1* transcription start site and the genetic modification decreases the expression of *pdcd1.*

The present invention is further directed to an *in vitro* or *ex vivo* method of engineering a mammalian T cell to downregulate expression of *pdcd1* in the mammalian T cell, the method comprising deleting the region corresponding to the nucleic acid sequence of SEQ ID NO: 12, which is tctccccaag ggagccattt ctttcaccct ttgtctctgc tctttctcct cacacttttg tttttgttat ggtctgacac cccccccccc ccaatctctc acccccagta gctgtggccg ttttgtccat gccagccagc tatttcatat cgttgctgta acgtgcttgc cagtcactga cacagagaag tgacttggct caaggatgta ctgaccacat aacctgtttt gttctgtacg ctctgaatgt tctgtatgag gtttgctaat cttaagaaat tccacaaagc tttacgcagg acccatcaaa tcaaacgtca atatgaactg ttatgtctaa aacggcttgg gtcagaccac cgggcaacct ttcctgtgcc tacgtctgag ctcactgtgg tttttgtggt tgacactgaa aaatgctact aataagccaa aaaatatgat tataatatgc atgttctgtt atctcaatgt tctgaaattc ttctgttcac cacccaacca ccaccactct taccttactc agaaccaatc agcttaaagg ttaactgata atatttcacc cagtaaacca acttctcccc tctttaccct ttaaactttt gagcttttct catataaaat gcctacactg agagcagact agtaccatcg ccaggctcct gcagtccctt ctgtggtcct agatgcccag cactatggtg tgcattcaat aaagtattct tgcttaactg agatcagggt ttgtttggtt tgaggggtga ctccctgaac c, that is -23.8kb upstream of the *pdcd1* transcription start site by genetic modification and wherein the genetic modification downregulates the expression of *pdcd1.*

The present invention is based, at least in part, on the discovery that exhausted T cells, such as those resulting from cancer and/or chronic infection, have distinctive patterns of gene expression, including sustained expression of the inhibitory receptor PD-1. For example, the epigenetic landscape in exhausted CD8⁺ T cells contributing to such gene expression patterns is profoundly different from that of functional memory CD8⁺ T cells. CD8⁺ T cell differentiation during infection occurs with a massive reshaping of accessible chromatin regions organized into functional modules of enhancers. Exhausted CD8⁺ T cells acquire unique modules of enhancers not found in functional CD8⁺ T cells, and an extensive network of state-specific transcription factor binding. For example, one enhancer, -23kb from the *Pdcd1* locus, is found only in exhausted T cells and other lymphocytes with sustained PD-1 expression. Genome editing described herein demonstrates that the enhancer is required for high PD-1 expression. Thus, T cell exhaustion occurs with state-specific regulation of genes critical to their dysfunction. These state-specific genomic regulators of gene expression are targets for modulation, such as by genome editing, for altering gene expression preferentially in exhausted CD8⁺ T cells or in cells affecting T cell function. Selectively targeting T cell exhaustion-specific genomic regulatory elements, such as enhancers, suppressors, and promoters, can maintain normal physiological regulation of genes like PD-1, while preventing the development of T cell exhaustion, such as occurs in cancer and/or viral infections.

In one aspect of the disclosure, an engineered mammalian T cell comprising a genomic region that 1) regulates the gene expression of at least one gene and 2) is selectively chromatin accessible within the genome of an exhausted CD8+ T cell from the mammal, wherein the genomic region is genetically modified and the genetic modification modulates the expression of the at least one gene, is provided.

Numerous embodiments according to the disclosure are further provided that can be applied to any aspect of the present invention and/or combined with any other embodiment described herein. For example, in one embodiment according to the disclosure, the genomic gene expression regulatory region activity is upregulated (*e.g*., the engineered genomic gene expression regulatory region results in at least a 5% upregulation of expression of the at least one gene in the mammalian T cell as compared to the expression of the at least one gene in the same T cell type from the mammal without the engineered genomic gene expression regulatory region). In another embodiment according to the disclosure, the genomic gene expression regulatory region activity is downregulated (*e.g.*, the engineered genomic gene expression regulatory region results in at least a 5% downregulation of expression of the at least one gene in the mammalian T cell as compared to the expression of the at least one gene in the same T cell type from the mammal without the engineered genomic gene expression regulatory region). In still another embodiment according to the disclosure, the expression of the at least one gene is transcription or translation of the at least one gene. In yet another embodiment according to the disclosure, the genomic gene expression regulatory region, or a portion thereof, is deleted. In another embodiment according to the disclosure, the genomic gene expression regulatory region, or the portion thereof, is deleted by genome editing, optionally wherein the genome editing is expressed constitutively or inducibly (*e.g.*, such as genome editing selected from the group consisting of CRISPR-Cas9 RNA-guided engineered nucleases (RGENs), zinc finger nucleases (ZFNs), transcription activator-like effectors (TALEs), homing meganucleases, and homologous recombination). In still another embodiment according to the disclosure, the genomic gene expression regulatory region is selected from the group consisting of regulatory regions shown in Tables 1A-1K.

In yet another embodiment according to the disclosure, the mammal is an animal model of an immune disorder, optionally wherein the immune disorder is a chronic immune disorder. In another embodiment according to the disclosure, the animal model is a mouse model. In yet another embodiment according to the disclosure, the mammal is a human. In another embodiment according to the disclosure, the human is afflicted with an immune disorder, optionally wherein the immune disorder is a chronic immune disorder.

In still another embodiment according to the disclosure, the chronic immune disorder is a chronic infection or cancer. In yet another embodiment according to the disclosure, the infection is caused by an agent selected from the group consisting of human immunodeficiency virus (HIV), hepatitis C virus (HCV), hepatitis B virus (HBV), adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyoma virus BK, polyoma virus JC, measles virus, rubella virus, human T cell leukemia virus I, human T cell leukemia virus II, *Leishmania, Toxoplasma, Trypanosoma, Plasmodium, Schistosoma,* and *Encephalitozoon.* In another embodiment according to the disclosure, the chronic infection is not a latent infection. In still another embodiment according to the disclosure, the cancer is a hematological cancer or a solid cancer. In yet another embodiment according to the disclosure, the solid cancer is selected from the group consisting of lung cancer, non-small cell lung cancer (NSCLC), skin cancer, melanoma, cervical cancer, uterine cancer, ovarian cancer, breast cancer, pancreatic cancer, stomach cancer, esophageal cancer, colorectal cancer, liver cancer, prostate cancer, kidney cancer, bladder cancer, head and neck cancer, sarcoma, lymphoma, and brain cancer. In another embodiment according to the disclosure, the exhausted CD8+ T cell expresses a T cell exhaustion biomarker selected from the group consisting of a checkpoint inhibitor, PD-1 (Pdcd1), TIM-3 (Havcr2), LAG-3 (Lag3), CTLA-4 (Ctla4), 2B4 (CD244), CD39 (Entpd1), CD160, eomesodermin (Eomes), T-BET (Tbx21), BATF, BLIMP-1 (Prdm1), NFATC1, NR4A2, MAFB, OCT-2 (Pou2f2), Foxp1, retinoic acid receptor alpha (Rara), and combinations thereof. In still another embodiment according to the disclosure, the T cell is a CD8+ T cell. In yet another embodiment according to the disclosure, the CD8+ T cell is a non-exhausted T cell or an exhausted T cell. In another embodiment according to the disclosure, the non-exhausted CD8+ T cell is a naive, functional effector, or memory cell. In another embodiment according to the disclosure, the exhausted CD8+ T cell expresses a T cell exhaustion biomarker selected from the group consisting of a checkpoint inhibitor, PD-1 (Pdcd1), TIM-3 (Havcr2), LAG-3 (Lag3), CTLA-4 (Ctla4), 2B4 (CD244), CD39 (Entpd1), CD160, eomesodermin (Eomes), T-BET (Tbx21), BATF, BLIMP-1 (Prdm1), NFATC1, NR4A2, MAFB, OCT-2 (Pou2f2), Foxp1, retinoic acid receptor alpha (Rara), and combinations thereof. In still another embodiment according to the disclosure, the T cell is a primary T cell isolated from the mammal, engineered, and returned *ex vivo* to the mammal. In yet another embodiment according to the disclosure, the T cell is present *in vivo* within the mammal or is cultured *in vitro.*

In another aspect according to the disclosure, a method of engineering a mammalian T cell to modulate the expression of at least one gene in the mammalian T cell is provided, the method comprising genetically modifying the gene expression regulatory region of the gene, wherein the gene expression regulatory region is selectively chromatin accessible in an exhausted CD8+ T cell from the mammal and wherein the genetic modification modulates the expression of the at least one gene.

As described above, numerous embodiments according to the disclosure are further provided that can be applied to any aspect of the present invention and/or combined with any other embodiment according to the disclosure described herein. For example, in one embodiment according to the disclosure, the genomic gene expression regulatory region is upregulated (*e.g.*, the engineered genomic gene expression regulatory region results in at least a 5% upregulation of expression of the at least one gene in the mammalian T cell as compared to the expression of the at least one gene in the same T cell type from the mammal without the engineered genomic gene expression regulatory region). In another embodiment according to the disclosure, the genomic gene expression regulatory region is downregulated (*e.g.*, the engineered genomic gene expression regulatory region results in at least a 5% downregulation of expression of the at least one gene in the mammalian T cell as compared to the expression of the at least one gene in the same T cell type from the mammal without the engineered genomic gene expression regulatory region). In still another embodiment according to the disclosure, the expression of the at least one gene is transcription or translation of the at least one gene. In yet another embodiment according to the disclosure, the genomic gene expression regulatory region, or a portion thereof, is deleted. In another embodiment according to the disclosure, the genomic gene expression regulatory region, or the portion thereof, is deleted by genome editing, optionally wherein the genome editing is expressed constitutively or inducibly. In still another embodiment according to the disclosure, the genome editing is selected from the group consisting of CRISPR-Cas9 RNA-guided engineered nucleases (RGENs), zinc finger nucleases (ZFNs), transcription activator-like effectors (TALEs), homing meganucleases, and homologous recombination. In yet another embodiment according to the disclosure, the genomic gene expression regulatory region is selected from the group consisting of regulatory regions shown in Tables 1A-1K. In another embodiment according to the disclosure, the mammal is an animal model of an immune disorder, optionally wherein the immune disorder is a chronic immune disorder. In still another embodiment according to the disclosure, the animal model is a mouse model. In yet another embodiment according to the disclosure, the mammal is a mouse or a human. In another embodiment according to the disclosure, the mammal is a human. In still another embodiment according to the disclosure, the human is afflicted with an immune disorder, optionally wherein the immune disorder is a chronic immune disorder. In yet another embodiment according to the disclosure, the chronic immune disorder is a chronic infection or cancer. In another embodiment according to the disclosure, the infection is caused by an agent selected from the group consisting of human immunodeficiency virus (HIV), hepatitis C virus (HCV), hepatitis B virus (HBV), adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B 19, polyoma virus BK, polyoma virus JC, measles virus, rubella virus, human T cell leukemia virus I, human T cell leukemia virus II, *Leishmania, Toxoplasma, Trypanosoma, Plasmodium, Schistosoma,* and *Encephalitozoon.* In still another embodiment according to the disclosure, the chronic infection is not a latent infection. In yet another embodiment according to the disclosure, the cancer is a hematological cancer or a solid cancer. In another embodiment according to the disclosure, the solid cancer is selected from the group consisting of lung cancer, non-small cell lung cancer (NSCLC), skin cancer, melanoma, cervical cancer, uterine cancer, ovarian cancer, breast cancer, pancreatic cancer, stomach cancer, esophageal cancer, colorectal cancer, liver cancer, prostate cancer, kidney cancer, bladder cancer, head and neck cancer, sarcoma, lymphoma, and brain cancer. In still another embodiment according to the disclosure, the exhausted CD8+ T cell expresses a T cell exhaustion biomarker selected from the group consisting of checkpoint inhibitor, PD-1 (Pdcd1), TIM-3 (Havcr2), LAG-3 (Lag3), CTLA-4 (Ctla4), 2B4 (CD244), CD39 (Entpd1), CD160, eomesodermin (Eomes), T-BET (Tbx21), BATF, BLIMP-1 (Prdm1), NFATC1, NR4A2, MAFB, OCT-2 (Pou2f2), Foxp1, retinoic acid receptor alpha (Rara), and combinations thereof. In yet another embodiment according to the disclosure, the T cell is a CD8+ T cell. In another embodiment according to the disclosure, the CD8+ T cell is a non-exhausted T cell or an exhausted T cell. In still another embodiment according to the disclosure, the non-exhausted CD8+ T cell is a naive, functional effector, or memory cell. In yet another embodiment according to the disclosure, the exhausted CD8+ T cell expresses a T cell exhaustion biomarker selected from the group consisting of a checkpoint inhibitor, PD-1 (Pdcd1), TIM-3 (Havcr2), LAG-3 (Lag3), CTLA-4 (Ctla4), 2B4 (CD244), CD39 (Entpd1), CD160, eomesodermin (Eomes), T-BET (Tbx21), BATF, BLIMP-1 (Prdm1), NFATC1, NR4A2, MAFB, OCT-2 (Pou2f2), Foxp1, retinoic acid receptor alpha (Rara), and combinations thereof. In another embodiment according to the disclosure, the T cell is a primary T cell isolated from the mammal. In still another embodiment according to the disclosure, the T cell is present *in vivo* within the mammal or is cultured *in vitro.* In yet another embodiment according to the disclosure, the at least one gene is at least 2 genes.

In still another aspect according to the disclosure, a method of preventing exhaustion in a non-exhausted CD8+ T cell comprising engineering the non-exhausted CD8+ T cell according to any method described herein, is provided. In one embodiment according to the disclosure, the non-exhausted CD8+ T cell is a naive, functional effector, or memory cell. In still another embodiment according to the disclosure, the method further comprises administering the engineered non-exhausted CD8+ T cell to a subject.

In yet another aspect according to the disclosure, a method of reversing CD8+ T cell exhaustion in an exhausted CD8+ T cell comprising engineering the exhausted CD8+ T cell according to any method described herein, is provided. In one embodiment according to the disclosure, the exhausted CD8+ T cell expresses a T cell exhaustion biomarker selected from the group consisting of checkpoint inhibitor, PD-1 (Pdcd1), TIM-3 (Havcr2), LAG-3 (Lag3), CTLA-4 (Ctla4), 2B4 (CD244), CD39 (Entpd1), CD160, eomesodermin (Eomes), T-BET (Tbx21), BATF, BLIMP-1 (Prdm1), NFATC1, NR4A2, MAFB, OCT-2 (Pou2f2), Foxp1, retinoic acid receptor alpha (Rara), and combinations thereof. In another embodiment according to the disclosure, the method further comprises administering the engineered exhausted CD8+ T cell to a subject.

In another aspect according to the disclosure, a method of treating an immune disorder in a subject, comprising administering engineered T cells as described herein to the subject, is provided. In one embodiment according to the disclosure, the engineered T cells are administered focally or systemically. In another embodiment according to the disclosure, the systemic administration is intravenous, intramuscular, intraperitoneal, or intra-articular. In still another embodiment according to the disclosure, the engineered T cells administered to the subject are autologous, syngeneic, allogeneic, or xenogeneic to the subject. In yet another embodiment according to the disclosure, the engineered T cells administered to the subject are administered in a pharmaceutically acceptable formulation. In another embodiment according to the disclosure, the engineered T cells maintain the at least 5% modulated expression of the at least one gene after administration to the subject. In still another embodiment according to the disclosure, the method further comprises administering to the subject a therapeutically effective amount of a pharmaceutical composition comprising one or more anti-immune disorder agents. In yet another embodiment according to the disclosure, the method further comprises contacting the CD8+ T cells with one or more agents that prevent or reverse CD8+ T cell exhaustion. In another embodiment according to the disclosure, the one or more agents is an immune checkpoint inhibitor. In still another embodiment according to the disclosure, the immune checkpoint is selected from the group consisting of PD-1, PD-L1, PD-L2, LAG-3, TIM-1, CTLA-4, VISTA, B7-H2, B7-H3, B7-H4, B7-H6, 2B4, ICOS, HVEM, CD160, gp49B, PIR-B, KIR family receptors, TIM-1, TIM-4, BTLA, SIRPalpha (CD47), CD48, 2B4 (CD244), B7.1, B7.2, ILT-2, ILT-4, TIGIT, and A2aR. In yet another embodiment according to the disclosure, the mammal is an animal model of an immune disorder, optionally wherein the immune disorder is a chronic immune disorder. In another embodiment according to the disclosure, the animal model is a mouse model. In still another embodiment according to the disclosure, the mammal is a mouse or a human. In yet another embodiment according to the disclosure, the mammal is a human. In another embodiment according to the disclosure, the human is afflicted with an immune disorder, optionally wherein the immune disorder is a chronic immune disorder. In still another embodiment according to the disclosure, the chronic immune disorder is a chronic infection or cancer. In yet another embodiment according to the disclosure, the infection is caused by an agent selected from the group consisting of human immunodeficiency virus (HIV), hepatitis C virus (HCV), hepatitis B virus (HBV), adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyoma virus BK, polyoma virus JC, measles virus, rubella virus, human T cell leukemia virus I, human T cell leukemia virus II, *Leishmania, Toxoplasma, Trypanosoma, Plasmodium, Schistosoma,* and *Encephalitozoon.* In another embodiment according to the disclosure, the chronic infection is not a latent infection. In still another embodiment according to the disclosure, the cancer is a hematological cancer or a solid cancer. In yet another embodiment according to the disclosure, the solid cancer is selected from the group consisting of lung cancer, non-small cell lung cancer (NSCLC), skin cancer, melanoma, cervical cancer, uterine cancer, ovarian cancer, breast cancer, pancreatic cancer, stomach cancer, esophageal cancer, colorectal cancer, liver cancer, prostate cancer, kidney cancer, bladder cancer, head and neck cancer, sarcoma, lymphoma, and brain cancer.

### Brief Description of the Drawings

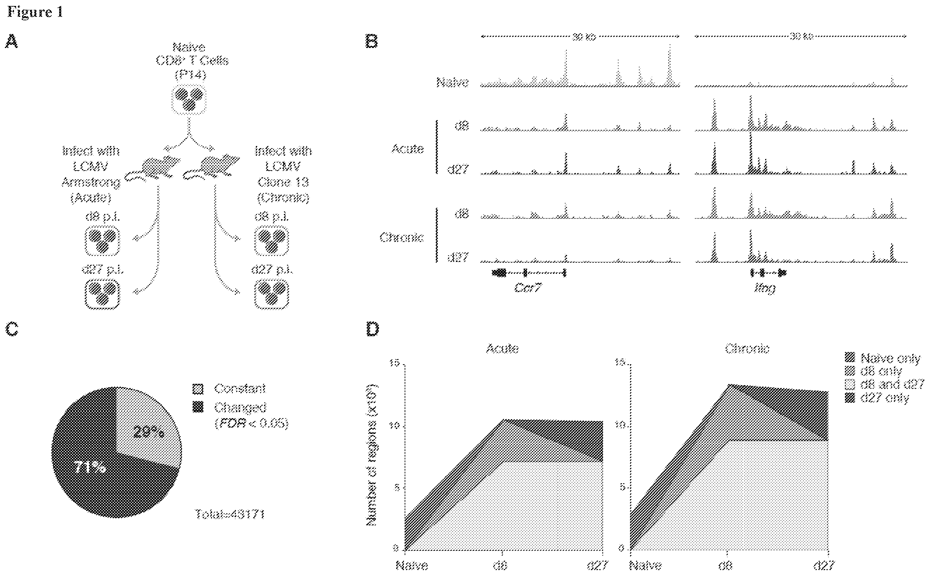

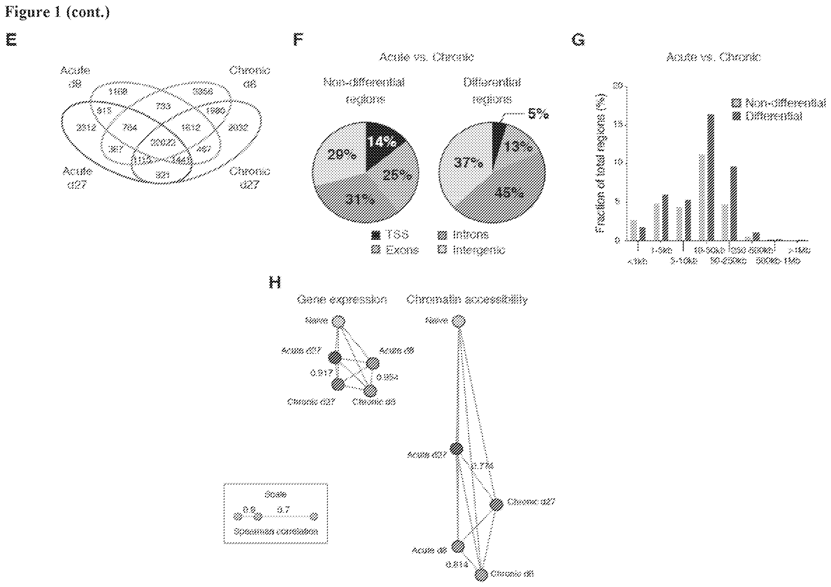

**Figure 1** includes 8 panels, identified as panels A, B, C, D, E, F, G, and H, which show that CD8⁺ T cell exhaustion is associated with extensive changes in accessible chromatin. Panel A shows a schematic diagram of the experiment. Panel B shows representative ATAC-seq tracks at the *Ccr7* and *Ifng* gene loci. Panel C shows the fraction of constant and changing chromatin accessible regions. Panel D shows the developmental trajectory of new regions at each time point. Panel E shows the overlap in chromatin accessible regions between cell states. Panel F shows the distribution of non-differential (left) and differential (right) regions between acute and chronic CD8⁺ T cell states. Panel G shows the distance to nearest TSS for all intergenic regions that are non-differential (light grey) and differential (dark grey). Panel H shows the correlation network of similarity between states measured by gene expression (left) and chromatin accessibility (right). Edge length corresponds to similarity (1-Spearman coefficient).

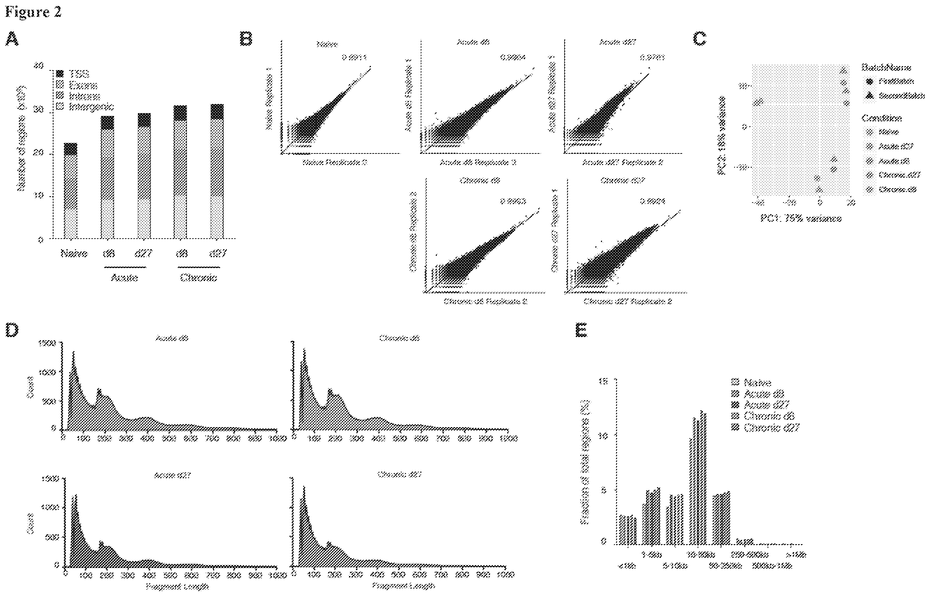

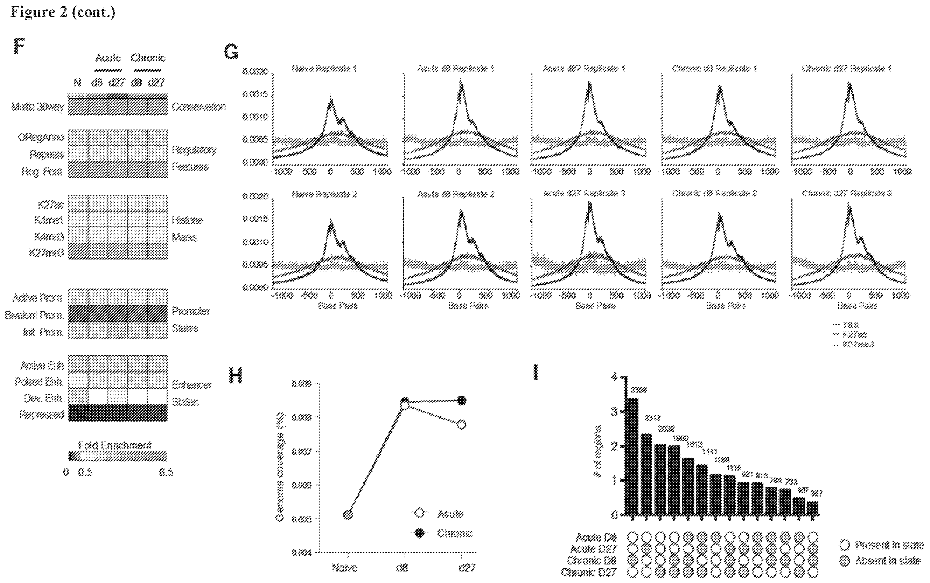

**Figure 2** includes 9 panels, identified as panels A, B, C, D, E, F, G, H, and I, which show characteristics of chromatin accessible regions from mouse CD8+ T cells. Panel A shows the number of chromatin accessible regions per condition, partitioned into those overlapping transcriptional start sites (TSS), exons, introns and intergenic areas as, indicated. Panel B is a scatterplot showing correlation in peak intensity between biological replicates for Naive, Acute d8, Acute d27, Chronic d8 and Chronic d27 states. Panel C shows principal component analysis of biological replicates for Naive, Acute d8, Acute d27, Chronic d8 and Chronic d27 states across all 43,171 ChARs. Panel D shows ATAC-seq fragment sizes from all five cell states. Panel E shows the distance to nearest TSS for all intergenic chromatin accessible regions (as percentage of total), for all five cell states. Panel F shows fold-enrichment of regions annotated for evolutionary conservation, regulatory region status and histone marks within ATAC peaks, for all five cell states. Panel G shows the combined ATAC signal across all TSSs (black), H3K27ac peaks (dark grey) and H3K27me3 (light grey). Panel H shows the fraction of the genome covered by chromatin accessible regions over time, in naive (grey symbol), CD8⁺ T cells and those following Acute (white symbols) or Chronic (black symbols) infection. Panel I shows the number of shared and differential chromatin accessible regions in CD8⁺ T cells from acute and chronic infection at day 8 and day 27 (q < 0.05 for differential) for combinations indicated by the grey/white symbols below.

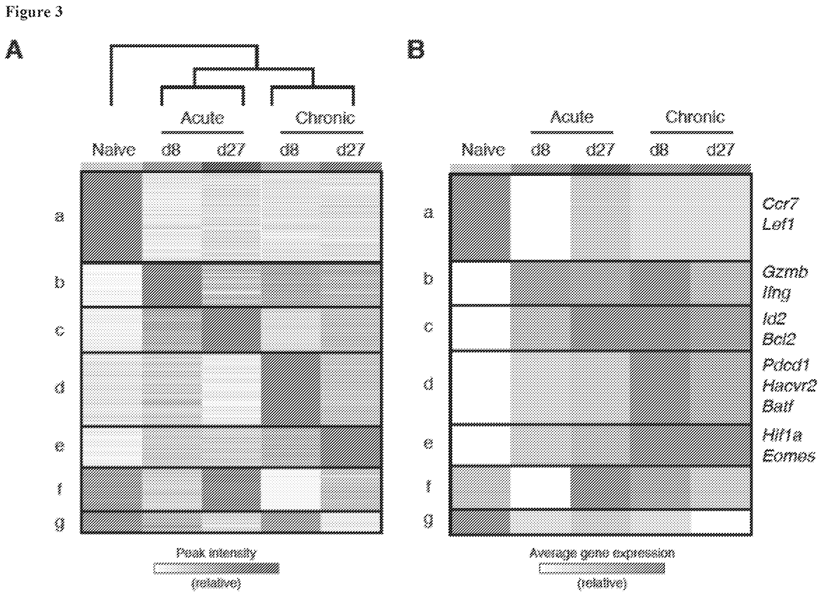

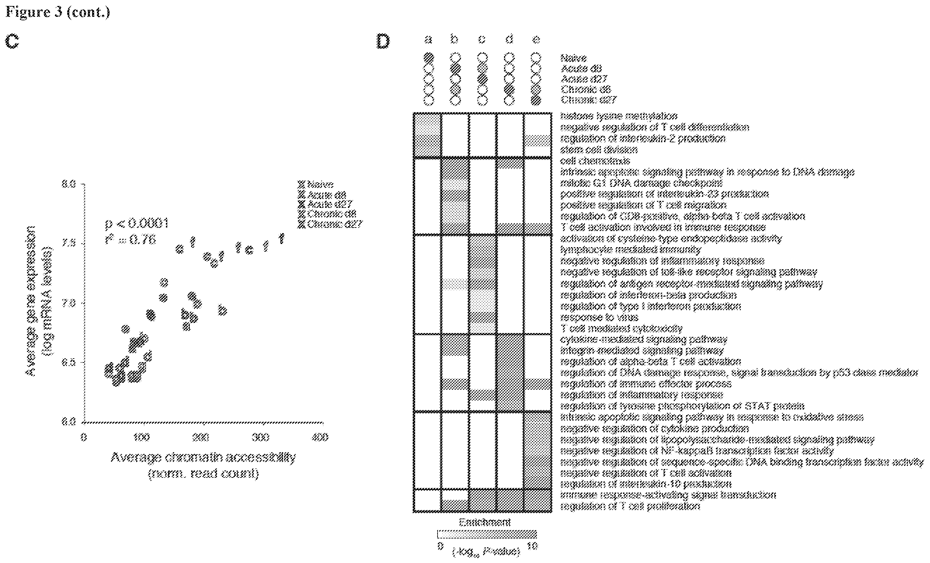

**Figure 3** includes 4 panels, identified as panels A, B, C, and D, which show state-specific enhancers in CD8⁺ T cells form modules that map to functionally distinct classes of genes. Panel A is a heat map of peak showing intensity for all differentially accessible regions (rows) clustered by similarity across cell states (columns). Panel B is a heat map showing row-normalized average mRNA expression of neighboring genes within each module in Panel A in each cell state. Informative genes from each module are shown on the right. Panel C shows the correlation between average gene expression and average chromatin accessibility for each module in each cell state. Letter symbols correspond to modules as indicated in Panels A-B. Panel D is a heat map showing enrichment of Gene Ontology terms (rows) in each module (columns). *P*-values (binomial test) are presented as 1-log10.

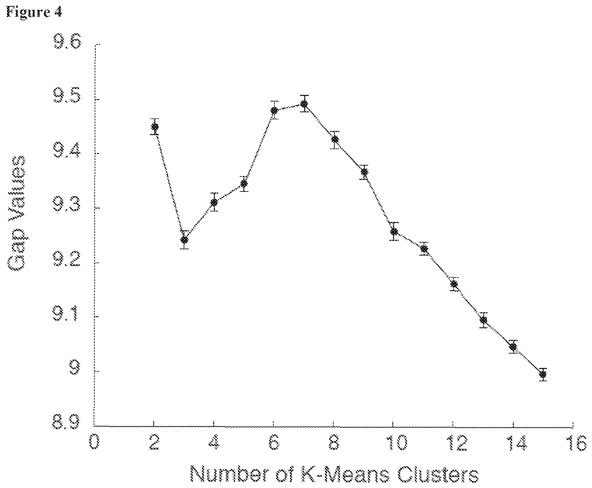

**Figure 4** shows a comparison of differential regions between states. The graph shows the gap statistic using given number of K-means clusters, applied to the signal intensity of all differential regions across the five cell states.

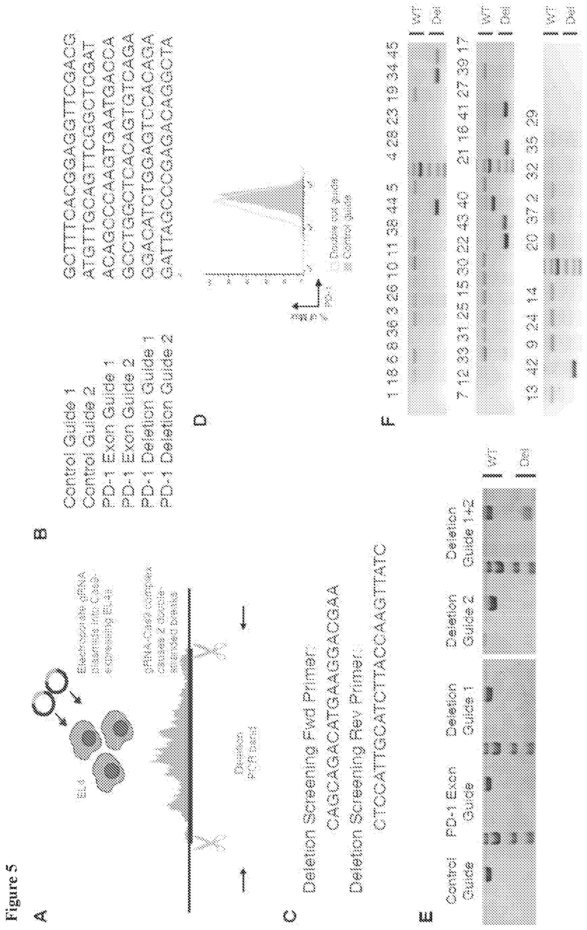

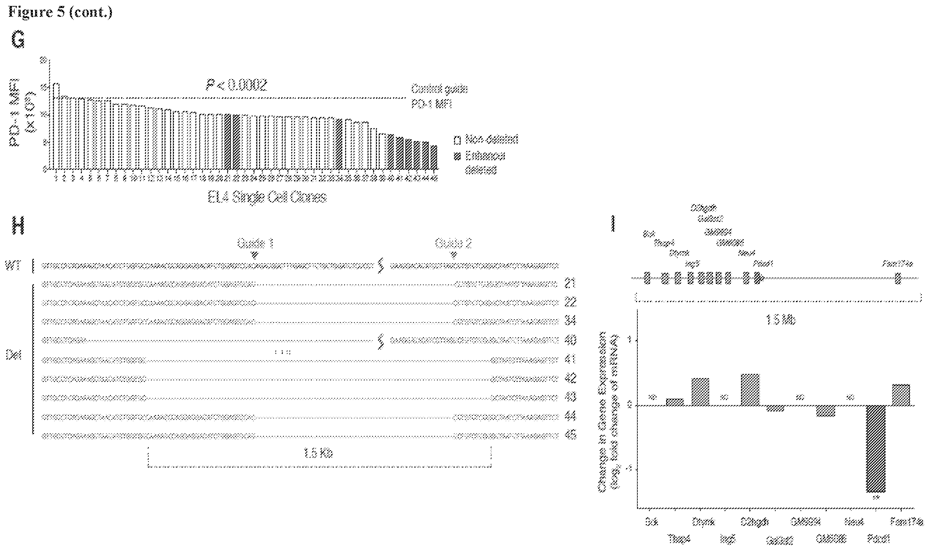

**Figure 5** includes 9 panels, identified as panels A, B, C, D, E, F, G, H, and I, which show the results of CRISPR/Cas9-mediated enhancer editing. Panel A shows a schematic diagram of the genome editing strategy. Panel B shows CRISPR PD-1 targeting and non-targeting sgRNAs used in EL4 cells. Panel C shows PCR primers used for screening genomic deletion and for Sanger sequencing. Panel D shows a histogram of PD-1 expression in EL4 cells transfected with control (light grey) or double cut sgRNAs targeting the -23.8kb region. Panel E shows a gel providing PCR screening results using genomic DNA from control sgRNA, single enhancer sgRNA, and double enhancer sgRNA receiving EL4 cells. Panel F shows a gel providing PCR screening results using genomic DNA from 45 EL4 single cell clones receiving double enhancer targeting sgRNAs. Panel G shows the expression of PD-1 in EL4 single-cell clones that are WT (white bars) or Del for the -23.8 kb enhancer (*p* < 0.0002, Mann-Whitney rank sum test for PD-1 expression). Panel H shows the sequence at the enhancer for representative EL4 single cell clones. Clone numbers correspond to those in Panel A and the arrows indicate the expected cut sites from sgRNAs. Panel I shows relative mRNA expression in WT or Del clones (*p* < 0.005, T-test).

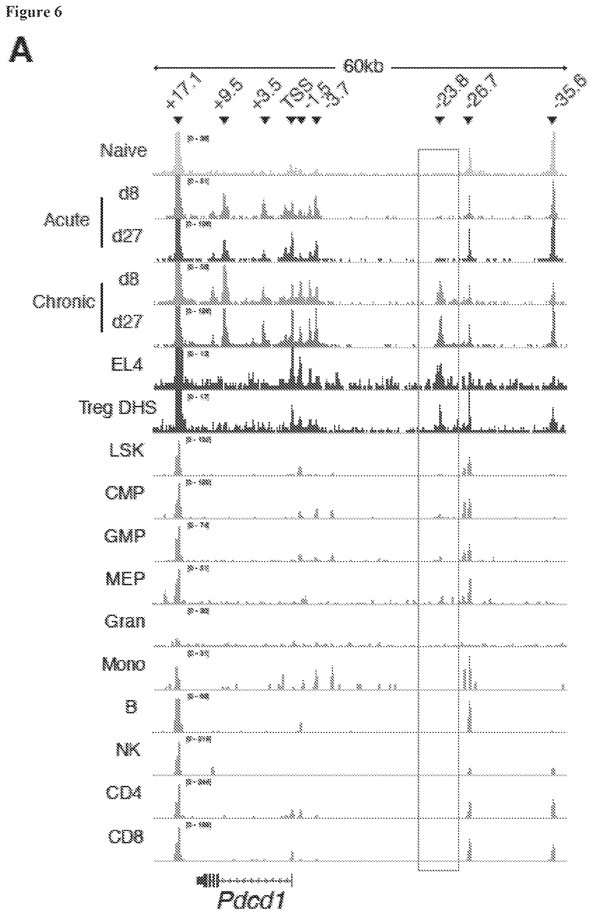

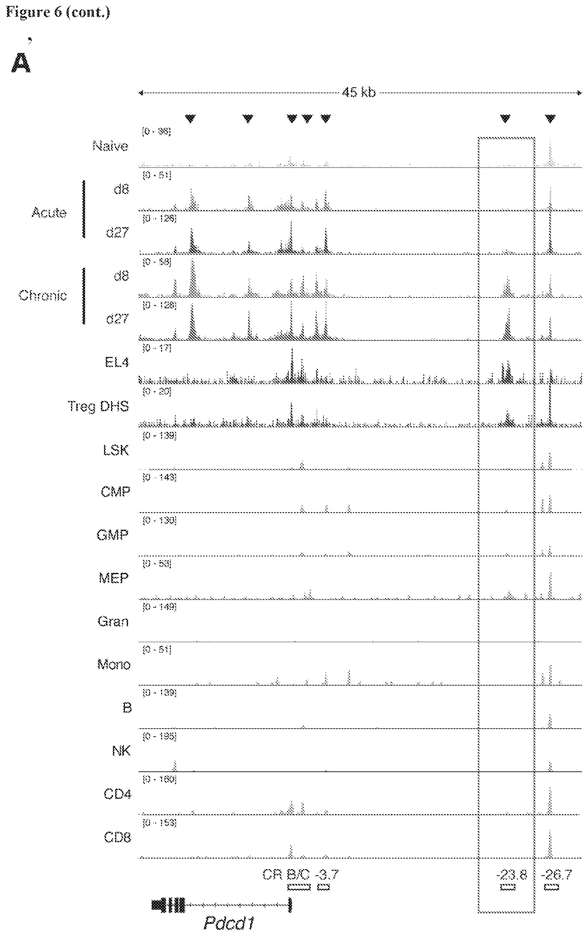

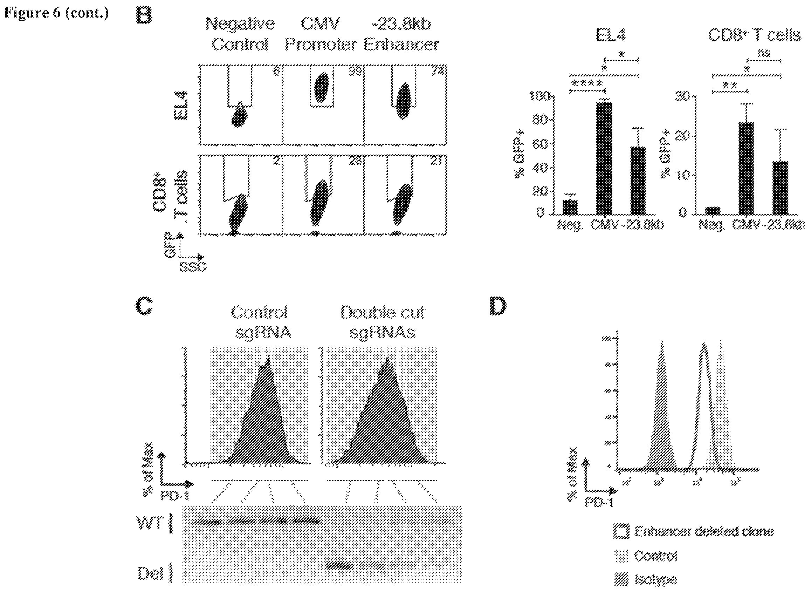

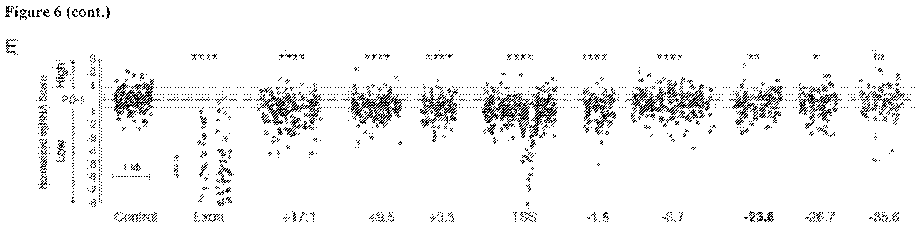

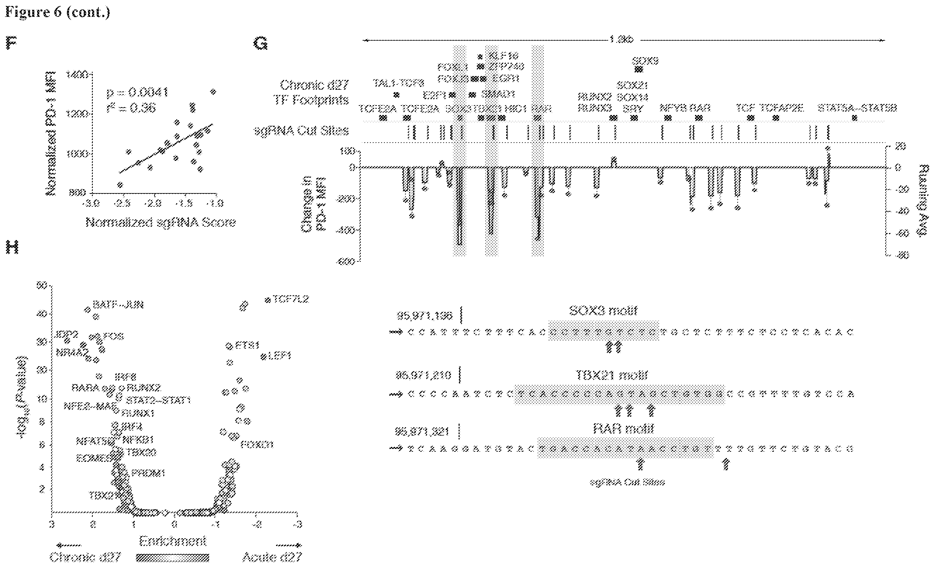

**Figure 6** includes 8 panels, identified as panels A, B, C, D, E, F, G, and H, which show that high resolution functional mapping of an exhaustion-specific enhancer identifies minimal sequences that regulate PD-1. Panel A shows ATAC-seq tracks from CD8⁺ T cells, the EL4 cell line, and hematopoietic lineages as indicated (Lara-Astiaso et al. (2014) Science 345:943-949). The arrows indicate individual ChARs. Note that Panel A is broken into subpanels A and A'. Panel B shows GFP reporter expression in EL4s (upper) and activated CD8⁺ T cells (lower) transduced with a negative control minimal promoter (left), CMV promoter construct (middle), and the *Pdcd1* -23.8 kb enhancer construct (right). Averages of 3-4 replicates are shown in the bar graphs. Panel C shows the results of cell sorting gates (upper panels) and corresponding genomic PCR for the PD-1 enhancer region (lower panels) showing the proportion of wild-type (WT) or deleted (Del) alleles in EL4 cells transfected with control (left) or double-cut sgRNAs (right). Panel D shows the PD-1 expression of representative EL4 wild-type (light grey) or enhancer deleted single cell clones. Panel E shows normalized enrichment of sgRNAs (grey symbols) within PD-1 high and low populations at locations shown. Control non-targeting sgRNAs are pseudo-mapped with 5 bp spacing. The dark circle symbols correspond to the 21 sgRNAs with the largest effect within the -23.8kb enhancer, for which isogenic lines were later produced. Panel F shows the correlation between normalized enrichment score in Panel E and PD-1 MFI of individually-transfected EL4 cells, for 21 top-scoring sgRNAs in the -23.8kb enhancer. Panel G shows the overlap of TF footprints and sgRNA activity within the-23.8kb enhancer. TF footprints with binding probability > 0.9 in chronic d27 are shown on top. Lines represent cut sites of top-scoring sgRNAs. The change in PD-1 MFI relative control guide-transfected populations for each sgRNA (circle symbol, left axis). A 10 bp running average of PD-1 MFI changes caused by sgRNA activity is shown in black (right axis). Panel H is a volcano plot of differential TF footprints in acute d27 v. chronic d27 CD8⁺ T cells.

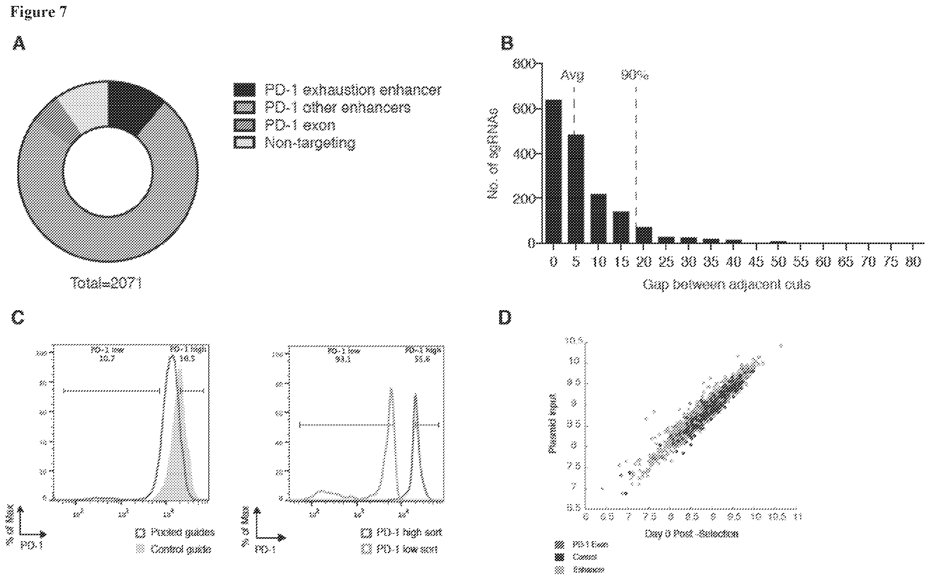

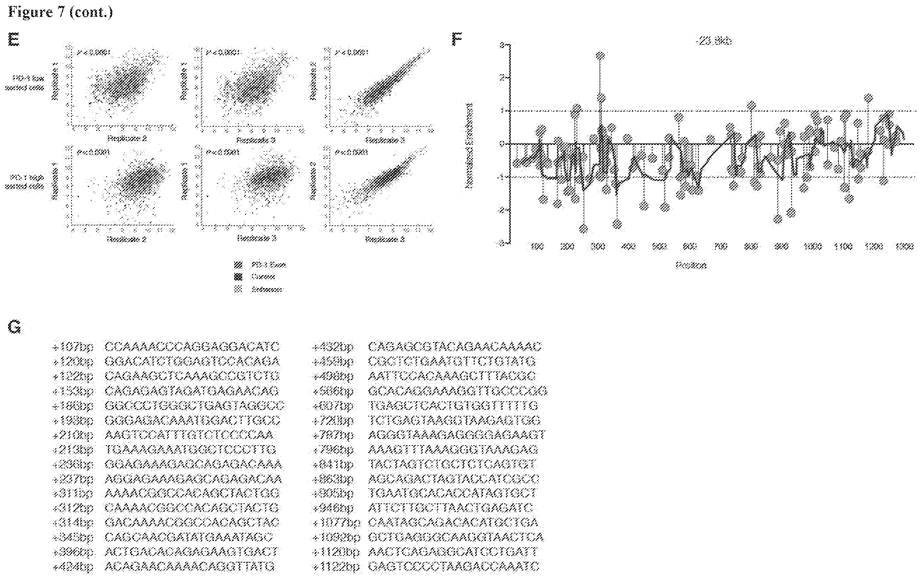

**Figure 7** includes 7 panels, identified as panels A, B, C, D, E, F, and G, which show the results of *in situ* saturation mutagenesis of the PD-1 enhancers. Panel A shows the composition of sgRNAs within the pooled screening library. Panel B shows the distribution of gaps between adjacent sgRNA cut sites. Panel C shows the PD-1 distribution (left) in pooled sgRNA transduced EL4s compared to control-transduced EL4s (grey). Gates indicate PD-1 high and PD-1 low sorted fractions. Post-sort distribution of PD-1 in high and low sorted populations are shown (right). Panel D shows the correlation of sgRNA representation in plasmid pool vs. in post-transduction EL4s. Panel E shows sgRNA enrichment scores within PD-1 high and low populations across 3 replicates. Panel F shows the normalized enrichment of all sgRNAs (grey symbols) within PD-1 high and low populations in -23.8kb enhancer with a 10 bp running average of sgRNA enrichment shown in black. Panel G shows the sequences for 32 sgRNAs enriched in the PD-1 low fraction within the -23.8kb enhancer, which were chosen for individual validation. The relative positions from the start of the -23.8kb enhancer are shown on the left.

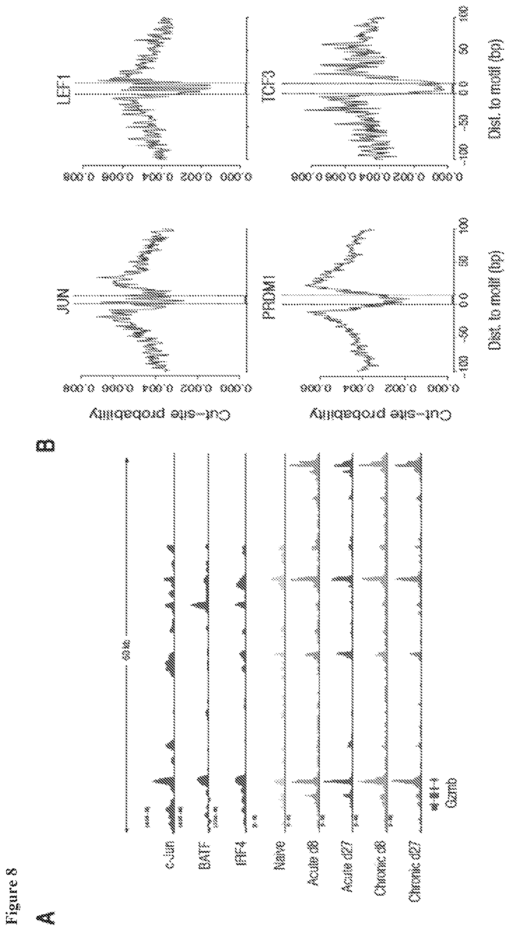

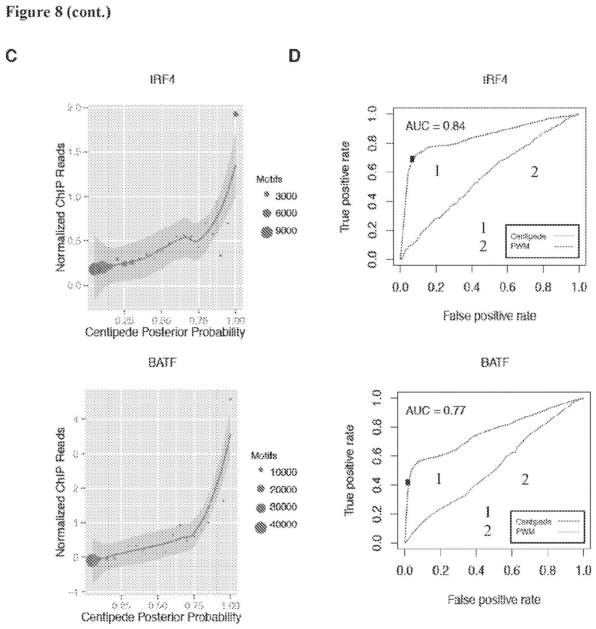

**Figure 8** includes 4 panels, identified as panels A, B, C, and D, which show the results of evaluation of transcription factor footprinting with ChIP-Seq data. Panel A shows IGV tracks showing ATAC-seq peaks for Naive, Acute d8, Acute d27, Chronic d8 and Chronic d27 cell states, as well as c-Jun, Batf and IRF4 ChIP tracks from *in vitro* activated CD8+ T cells, at the *Gzmb* locus. Panel B shows representative transcription factor (TF) footprints of the TFs indicated. Panel C shows the correlation of normalized ChIP-Seq reads and Centipede posterior probability for BATF and IRF4, across the full genome. Panel D shows ROC plots of varying cutoff for Centipede posterior probability used to call presence/absence of IRF4 and BATF binding (ChIP-Seq peaks used as standard for identifying TF binding).

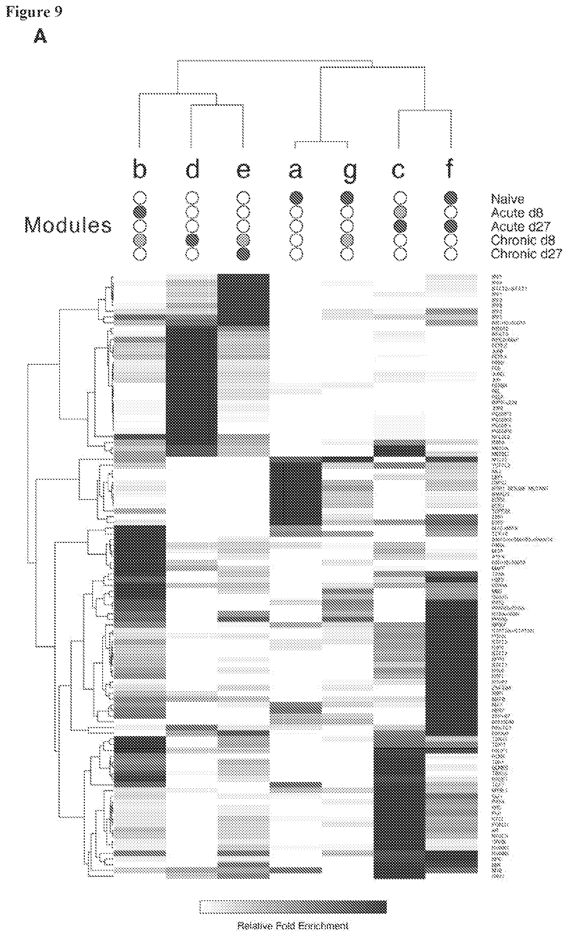

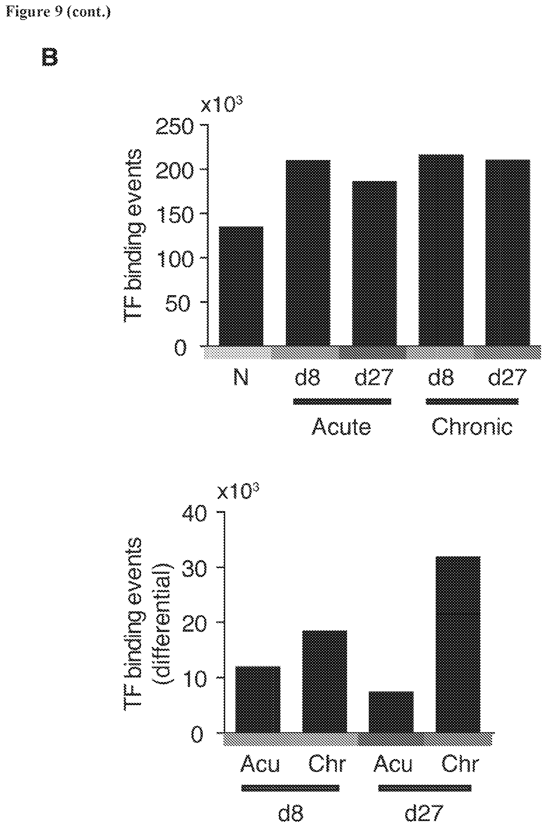

**Figure 9** includes 2 panels, identified as panels A and B, which show transcription factor footprinting in chromatin accessible regions. Panel A shows the relative fold enrichment of 100 transcription factors (TFs) (rows) in the 7 ChAR modules (columns). The rows and columns are hierarchically clustered. TF rankings were determined by weighting differential gene expression and differential TF inferred binding in Acute and Chronic infection. Panel B shows the number of inferred TF binding events in total for all five states (top) or differential between acute and chronic at d8 and d27 (bottom).

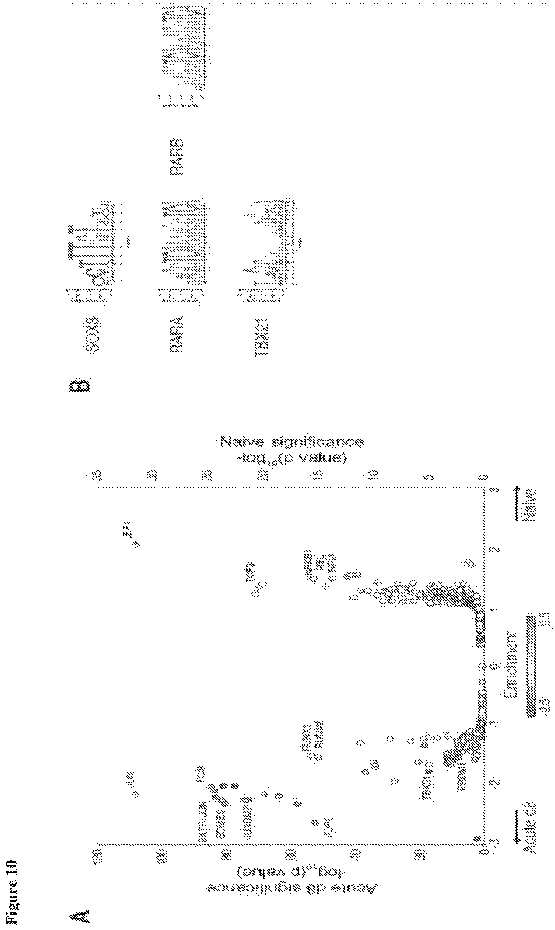

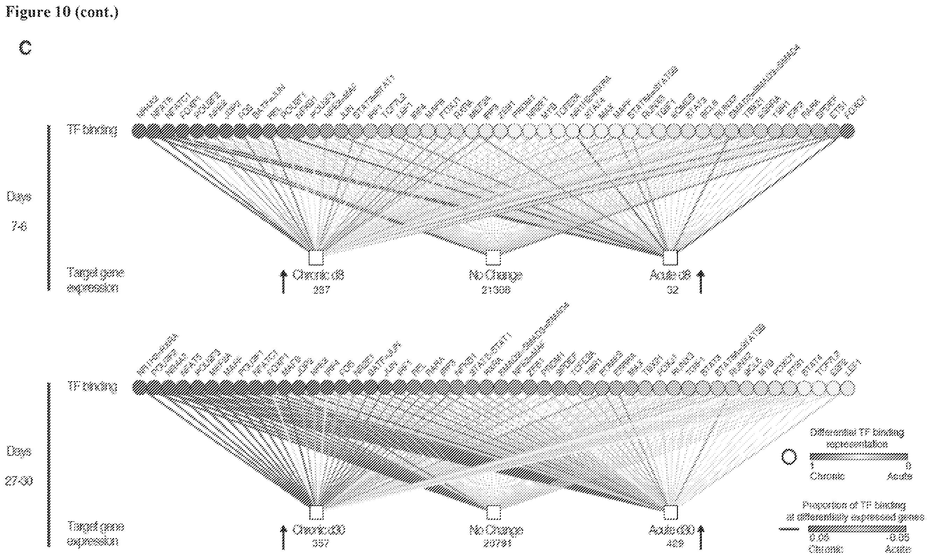

**Figure 10** includes 3 panels, identified as panels A, B, and C, which show the results of transcription factor footprinting in chromatin accessible regions. Panel A is a volcano plot of relative binding activity at TF motifs in naive v. acute d8 CD8⁺ T cells. Panel B shows motifs for Sox3, RAR and TBX21 with footprints in the -23.8kb PD-1 enhancer. Panel C shows a network diagram providing inferred binding at TFs motifs (round nodes) in regions adjacent to genes (square nodes). Genes are partitioned by mRNA expression into those upregulated (left), downregulated (right) or unchanged (center) between acute and chronic infection, at day 7 (top) and day 27 (bottom) post-infection. TF nodes are colored to indicate partiality of state-specific binding in chronic (left) or acute (right) infection. Edges are graded to indicate the relative fraction of state-specific binding events to the 3 classes of genes (upregulated in acute infection, chronic infection or unchanged). Edges denoting binding-events that are the most identified preferentially in chronic infection are most left, while those seen most preferentially in acute infection are most right.

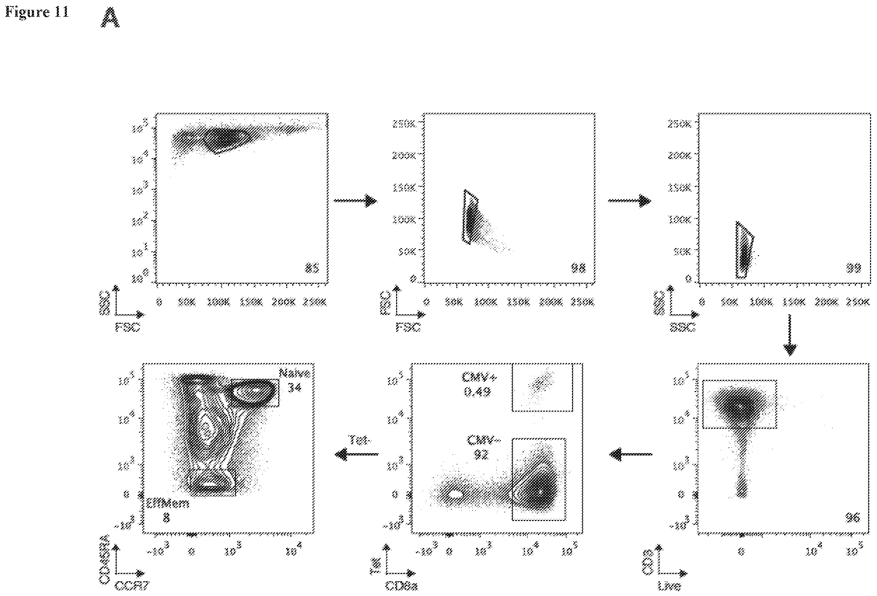

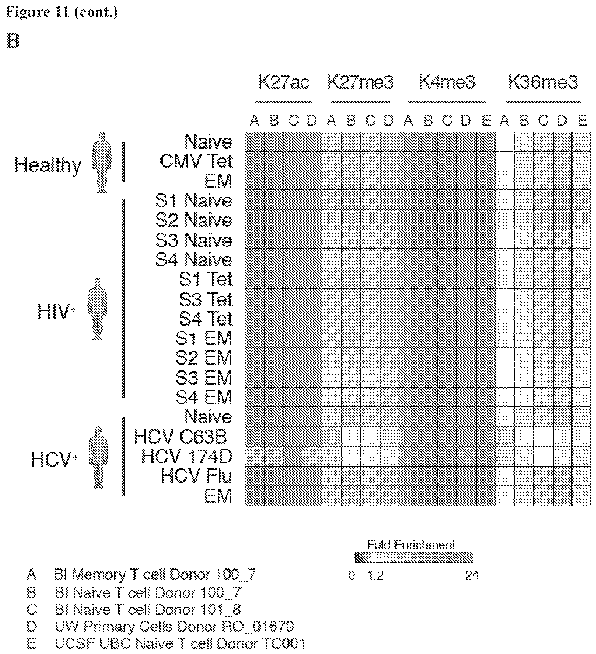

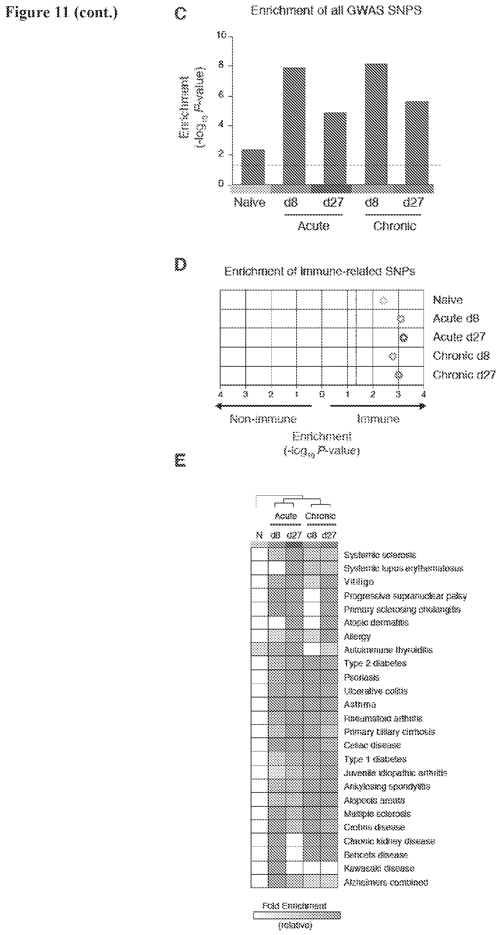

**Figure 11** includes 5 panels, identified as panels A, B, C, D, and E, which show characteristics of human CD8⁺ T cell chromatin accessible regions and mouse orthologous regions. Panel A shows the cell sorting strategy for tetramer⁺, naive, and effector memory populations on a representative human sample. Panel B shows fold enrichment of regions annotated for published histone marks in human primary T cells within ATAC peaks for all human samples. Panel C shows enrichment of all NHGRI GWAS SNPs. The dotted line illustrates *p* = 0.05 (hypergeometric test). Panel D shows enrichment of immune system disease related SNPs. Panel E is a heat map showing fold enrichment of autoimmune diseases SNPs in orthologous regulatory regions relative to randomly selected regions. The columns are hierarchically clustered.

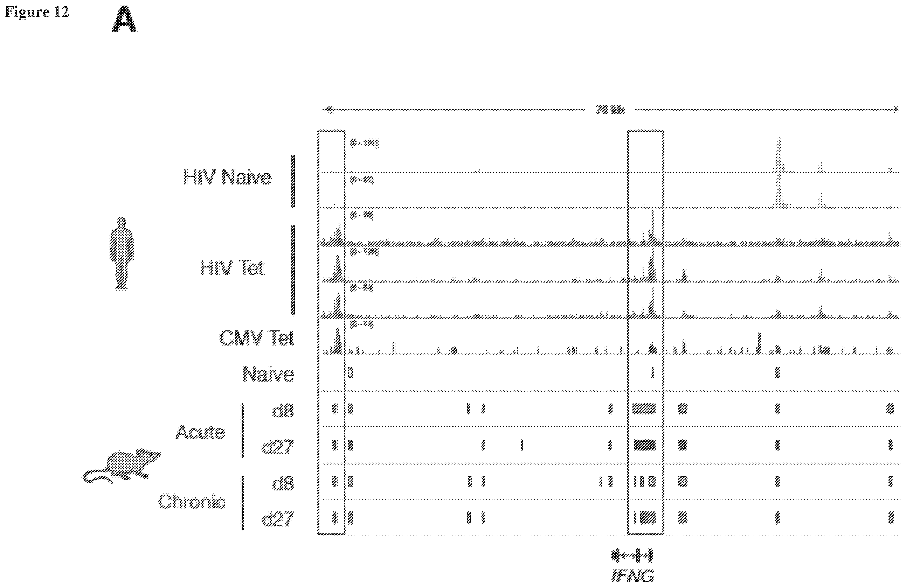

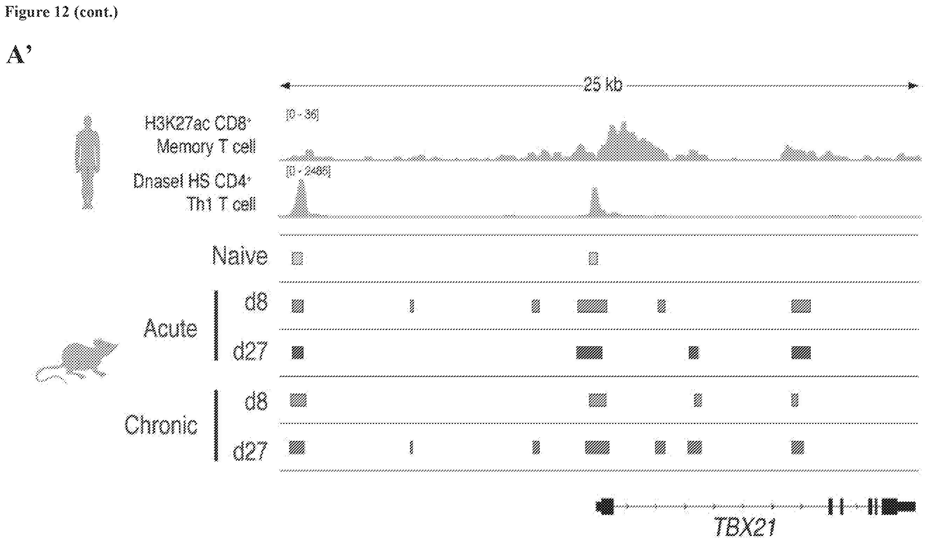

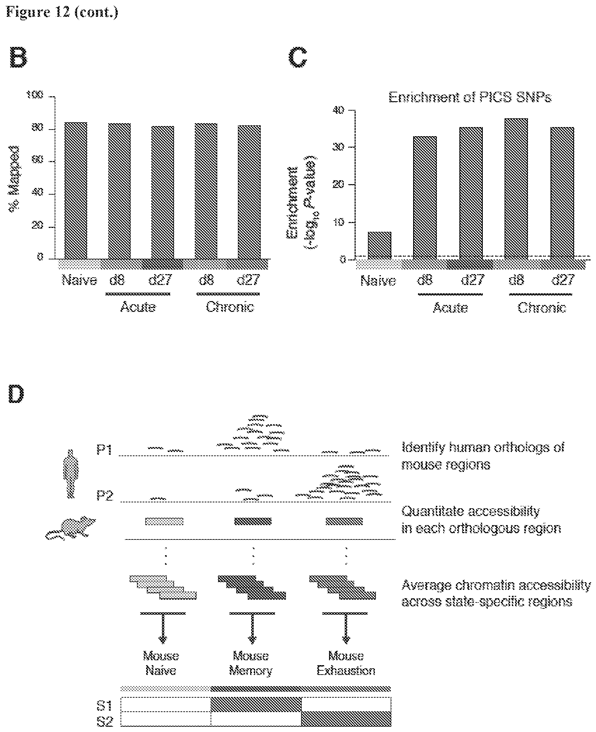

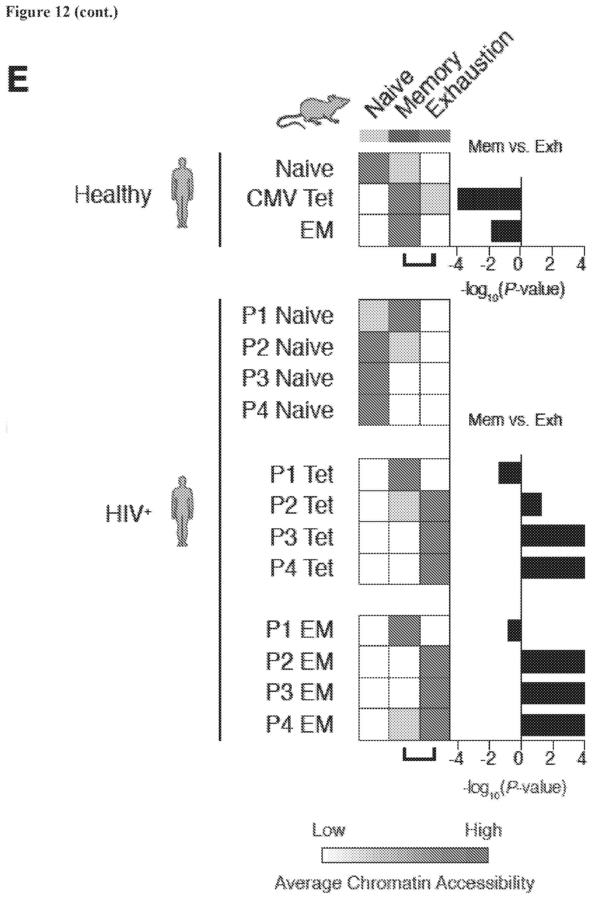

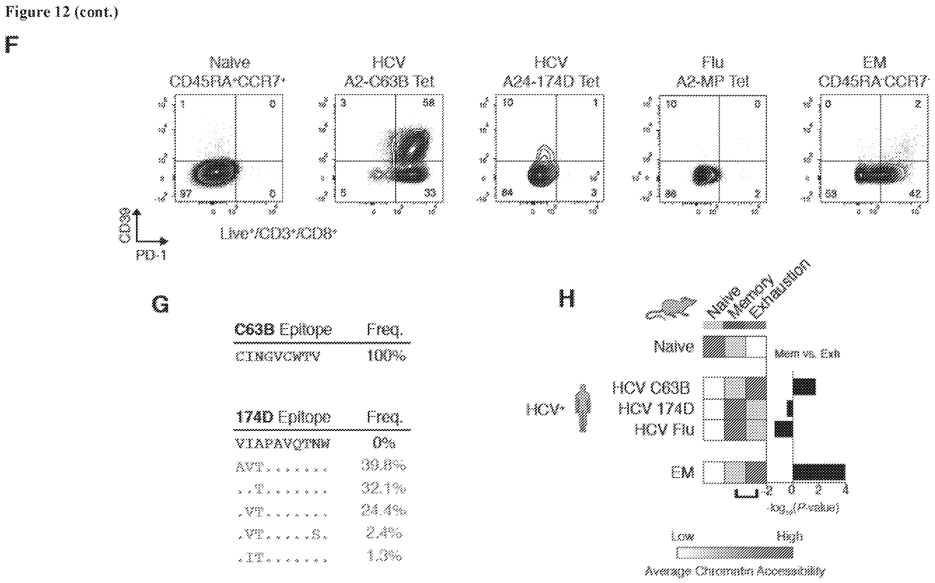

**Figure 12** includes 8 panels, identified as panels A, B, C, D, E, F, G, and H, which show that exhaustion-specific epigenetic profiles in the mouse are conserved in antigen-specific exhausted human T cells in HIV and HCV infection. Panel A shows representative ATAC-seq tracks from Naive, HIV tetramer⁺ and CMV tetramer⁺ samples at the *IFNG* gene locus (top). Mapping of mouse chromatin accessible regions to the human genome at *IFNG* (blocks, bottom) is shown. Note that panel is divided into sub-panels A and A' and sub-panel A' further shows ATAC-seq tracks at the *TBX21* gene locus. Panel B shows the percentage of all peaks entirely/partially mapped to human genome (hg19), for naive, acute d8, acute d27, chronic d8 and chronic d27 states. Panel C shows the enrichment of PICS SNPs in mouse orthologous ChARs. The dotted line illustrates *p* = 0.05 (hypergeometric test). Panel D shows a schematic diagram of mouse and human comparative analysis. Panel E is a heat map showing average chromatin accessibility at regions orthologous to mouse naïve, memory and exhaustion enhancers in the human samples indicated. Panel F shows PD-1 and CD39 expression measured by flow cytometry in naive, HCV C63B tetramer⁺, HCV 174D tetramer⁺, Flu MP tetramer⁺, and effector memory CD8⁺ T cell populations from a single HCV-infected donor. Panel G shows viral sequences encoding C63B and 174D epitopes. Panel H is a heat map showing average chromatin accessibility at regions orthologous to mouse naive, memory and exhaustion enhancers in human samples as indicated from a single HCV-infected donor.

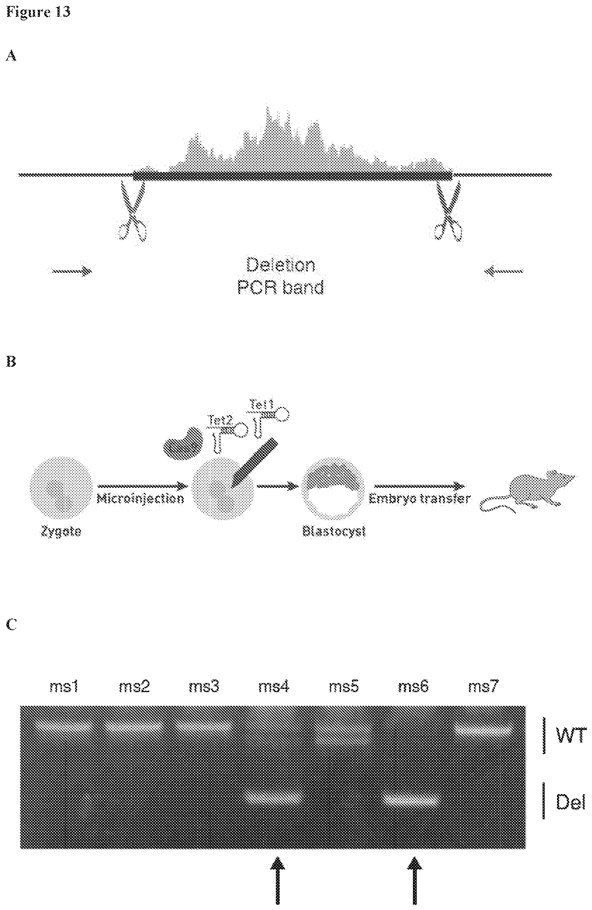

**Figure 13** includes 3 panels, identified as panels A, B, and C, which show the characterization of mice with germ-line deletion of the -23kb enhancer sequence upstream of PD-1. Panel A shows a schematic diagram of germ-line deletion of such enhancer sequence by PCR. Panel B depicts the method of microinjection and implantation into mice used to introduce the germ-line deletion into the genome. Panel C compares the ChAR region containing the -23 kb enhancer sequence among seven viable progeny by gel electrophoresis and demonstrating that 2 *(i.e.,* ms4 and ms6) of 7 progeny had the enhancer sequence deleted.

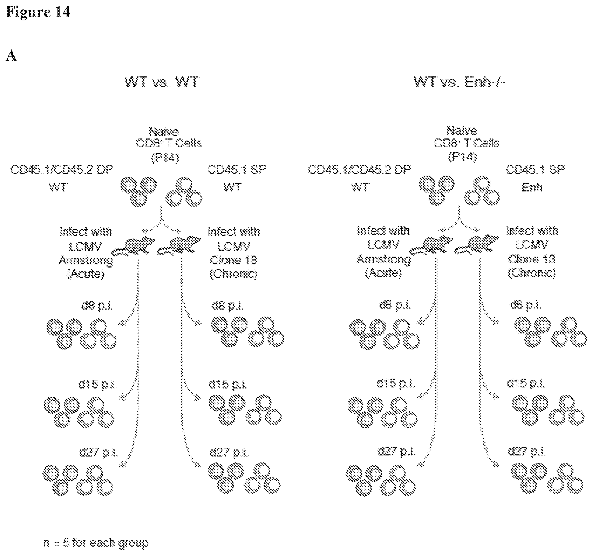

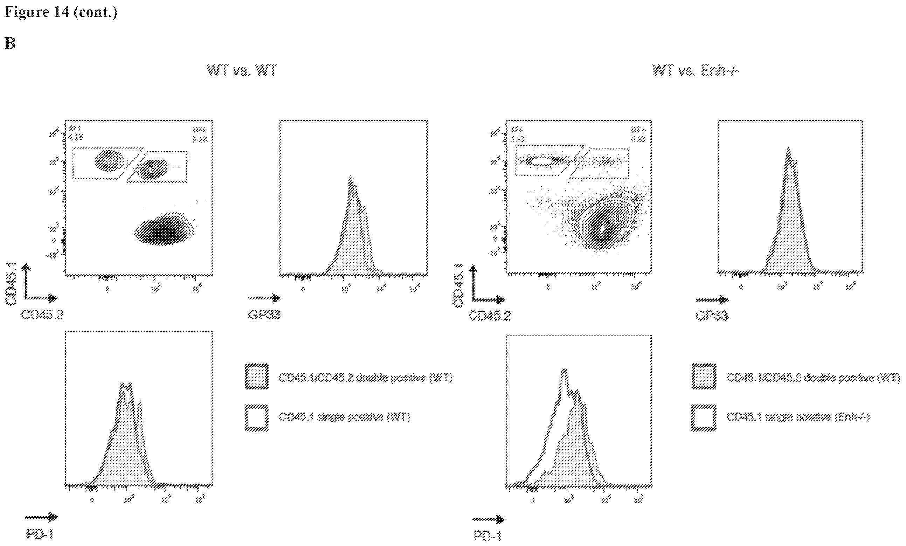

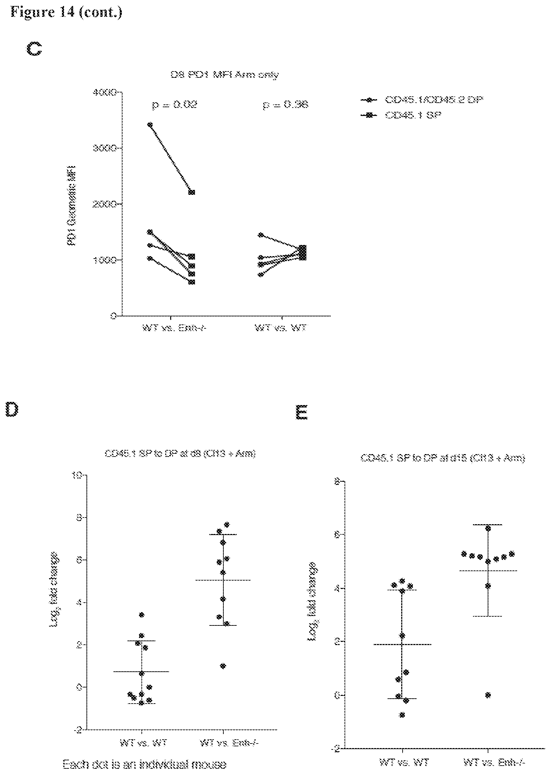

**Figure 14** includes 5 panels, identified as panels A, B, C, D, and E, which compare characteristics of the mice with germ-line deletion of the -23kb PD-1 enhancer versus wild-type mice. Panel A shows a schematic diagram of an adoptive cell transfer strategy to produce two mixes of mice. Panel B shows the characterization of the CD8+ T cells from the two mixes of mice produced in Panel A and results of comparing their cell population and expression levels of transgenic TCR and PD-1 using flow cytometry. Panel C shows the characterization of the CD8+ T cells from the two mixes of mice produced in Panel A and results of comparing their expression level of PD-1 using MFI. Panel D compares the growth rates (as log₂ fold changes) of the CD8+ T cells from the two mixes of mices at d8. Panel E compares the growth rates (as log₂ fold changes) of the CD8+ T cells from the two mixes of mice at d15.

For any figure showing a bar histogram, curve, or other data associated with a legend, the bars, curve, or other data presented from left to right for each indication correspond directly and in order to the boxes from top to bottom of the legend.

### Detailed Description of the Invention

The present invention is based, at least in part, on the discovery that exhausted T cells, such as those resulting from cancer and/or chronic viral infection, have distinctive patterns of gene expression, including sustained expression of the inhibitory receptor PD-1. Chromatin accessible genomic gene regulatory regions *(e.g.,* gene expression enhancers or gene expression suppressors, whether distal to promoters or within promoters themselves) have been identified that are state-specific for exhausted T cells and that are profoundly different from those of functional memory CD8⁺ T cells. Since these regions are chromatin accessible, transcription factors are able to physically bind the region to mediate regulation of gene expression. Such state-specific regulation of genes in exhausted T cells is critical to their dysfunction and represent targets for modulation, such as by genome editing, in order to alter gene expression preferentially in exhausted CD8⁺ T cells or in cells affecting T cell function, are described herein. Exhausted CD8⁺ T cells in a mouse model of chronic viral infection acquire an extensive, state-specific pattern of enhancers, which are organized into functional modules. One enhancer, -23.8kb from the *Pdcd1* locus, is found only in exhausted T cells and other lymphocytes with sustained PD-1 expression. Genome editing showed it to be required for high PD-1 expression. Cas9-mediated *in situ* saturation mutagenesis of the enhancer pinpoints critical minimal sequences that correspond to bound transcription factor motifs for RAR, T-bet and Sox3 in exhausted CD8⁺ T cells. State-specific enhancer profiles identified in mouse exhausted CD8⁺ T cells are conserved in human exhausted antigen-specific CD8⁺ T cells responding to HIV and HCV infection. Detailed functional enhancer maps of T cell exhaustion reveal state-specific regulatory sequences and offer targets for genome editing that could alter gene expression preferentially in exhausted CD8⁺ T cells.

Selectively targeting T cell exhaustion-specific chromatin accessible genomic regulatory elements, such as enhancers, suppressors, and/or promoters, in non-exhausted T cells to prevent T cell exhaustion and/or in exhausted T cells to reverse T cell exhaustion can maintain normal physiological regulation of genes like PD-1, while modulating T cell exhaustion, such as occurs in chronic immune disorders like cancer and/or viral infections. Gene expression modulatory regions proximal to the transcription start site of a gene and that regulates a single gene are typically referred to as promoter enhancing or suppressing gene expression modulatory regions, whereas gene expression modulatory regions distal to the transcription start site of a gene and that may regulate more than a single gene are typically referred to as enhancers and suppressors.

Accordingly, the present invention relates, in part, to compositions and methods for engineering T cells having modulated expression of at least one gene in a mammalian T cell (*e.g*., a non-exhausted or exhausted T cell) by genetic modification of a genomic region that regulates the gene expression of the at least one gene, wherein the genomic region is selectively chromatin accessible within the genome of an exhausted CD8+ T cell. In addition, methods for treating subjects afflicted with a chronic immune disorder (*e.g.*, cancer and/or a chronic viral infection) are provided. Moreover, numerous additional prognostic, diagnostic, and therapeutic methods are also described herein.

### I. Definitions

The articles "a" and "an" are used herein to refer to one or to more than one (i.e. to at least one) of the grammatical object of the article. By way of example, "an element" means one element or more than one element.

The "amount" of a marker, *e.g.*, expression or copy number of a marker, or protein level of a marker, in a subject is "significantly" higher or lower than the normal amount of a marker, if the amount of the marker is greater or less, respectively, than the normal level by an amount greater than the standard error of the assay employed to assess amount, and preferably at least twice, and more preferably three, four, five, ten or more times that amount. Alternately, the amount of the marker in the subject can be considered "significantly" higher or lower than the normal amount if the amount is at least about two, and preferably at least about three, four, or five times, higher or lower, respectively, than the normal amount of the marker.

The term "altered level of expression" of a marker refers to an expression level or copy number of a marker in a test sample *e.g.,* a sample derived from a subject suffering from cancer, that is greater or less than the standard error of the assay employed to assess expression or copy number, and is preferably at least twice, and more preferably three, four, five or ten or more times the expression level or copy number of the marker or chromosomal region in a control sample (*e.g*., sample from a healthy subject not having the associated disease) and preferably, the average expression level or copy number of the marker or chromosomal region in several control samples. The altered level of expression is greater or less than the standard error of the assay employed to assess expression or copy number, and is preferably at least twice, and more preferably three, four, five or ten or more times the expression level or copy number of the marker in a control sample (*e.g*., sample from a healthy subject not having the associated disease) and preferably, the average expression level or copy number of the marker in several control samples.

The term "altered activity" of a marker refers to an activity of a marker which is increased or decreased in a disease state, *e.g.,* in a tumor or autoimmune sample, as compared to the activity of the marker in a normal, control sample. Altered activity of a marker may be the result of, for example, altered expression of the marker, altered protein level of the marker, altered structure of the marker, or, *e.g.,* an altered interaction with other proteins involved in the same or different pathway as the marker, or altered interaction with transcriptional activators or inhibitors.

The term "altered structure" of a biomarker refers to the presence of mutations or allelic variants within a biomarker nucleic acid or protein, *e.g*., mutations which affect expression or activity of a marker nucleic acid or protein, as compared to the normal or wild-type gene or protein. For example, mutations include, but are not limited to substitutions, deletions, or addition mutations. Mutations may be present in the coding or non-coding region of the biomarker nucleic acid.

Unless otherwise specified herein, the terms "antibody" and "antibodies" broadly encompass naturally-occurring forms of antibodies (*e.g*., IgG, IgA, IgM, IgE) and recombinant antibodies such as single-chain antibodies, chimeric and humanized antibodies and multi-specific antibodies, as well as fragments and derivatives of all of the foregoing, which fragments and derivatives have at least an antigenic binding site. Antibody derivatives may comprise a protein or chemical moiety conjugated to an antibody. The properties recited herein for antibodies and antibody fragments also apply to Fc fusion proteins described herein.

The term "antibody" as used herein also includes an "antigen-binding portion" of an antibody (or simply "antibody portion"). The term "antigen-binding portion," as used herein, refers to one or more fragments of an antibody that retain the ability to specifically bind to an antigen *(e.g.,* PD-1 polypeptide or fragment thereof). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent polypeptides (known as single chain Fv (scFv); see *e.g.,* Bird et al. (1988) Science 242:423-426; and Huston et al. (1988) Proc. Natl. Acad. Sci. USA 85:5879-5883; and Osbourn et al. (1998) Nat. Biotechnol. 16: 778). Such single chain antibodies are also intended to be encompassed within the term "antigen-binding portion" of an antibody. Any VH and VL sequences of specific scFv can be linked to human immunoglobulin constant region cDNA or genomic sequences, in order to generate expression vectors encoding complete IgG polypeptides or other isotypes. VH and VL can also be used in the generation of Fab, Fv or other fragments of immunoglobulins using either protein chemistry or recombinant DNA technology. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad. Sci. USA 90:6444-6448; Poljak, R. J., et al. (1994) Structure 2:1121-1123).

Still further, an antibody or antigen-binding portion thereof may be part of larger immunoadhesion polypeptides, formed by covalent or noncovalent association of the antibody or antibody portion with one or more other proteins or peptides. Examples of such immunoadhesion polypeptides include use of the streptavidin core region to make a tetrameric scFv polypeptide (Kipriyanov, S.M., et al. (1995) Human Antibodies and Hybridomas 6:93-101) and use of a cysteine residue, a marker peptide and a C-terminal polyhistidine tag to make bivalent and biotinylated scFv polypeptides (Kipriyanov, S.M., et al. (1994) Mol. Immunol. 31:1047-1058). Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion polypeptides can be obtained using standard recombinant DNA techniques, as described herein.

Antibodies may be polyclonal or monoclonal; xenogeneic, allogeneic, or syngeneic; or modified forms thereof (*e.g.*, humanized, chimeric, etc.). Antibodies may also be fully human. Preferably, antibodies of the invention bind specifically or substantially specifically to PD-1 polypeptides or fragments thereof. They may also be selective for such antigens such that they can distinguish such antigens from closely related antigens, such as other B7 family members. The terms "monoclonal antibodies" and "monoclonal antibody composition", as used herein, refer to a population of antibody polypeptides that contain only one species of an antigen binding site capable of immunoreacting with a particular epitope of an antigen, whereas the term "polyclonal antibodies" and "polyclonal antibody composition" refer to a population of antibody polypeptides that contain multiple species of antigen binding sites capable of interacting with a particular antigen. A monoclonal antibody composition typically displays a single binding affinity for a particular antigen with which it immunoreacts.

As used herein, a "blocking" agent or an "antagonist" is one which inhibits or reduces at least one biological activity of the antigen(s) it binds. For example, an anti-PD-1 antibody binds PD-1 and inhibits the ability of PD-1 to bind one or more ligands, for example, PD-L1 and/or PD-L2. In certain embodiments according to the disclosure, the blocking antibodies or antagonist antibodies or fragments thereof described herein substantially or completely inhibit a given biological activity of the antigen(s). In certain embodiments according to the disclosure, the term "inverse agonist" is used to refer to an agent that promotes the opposite action to normal. For example, a PD-1 inverse agonist can promote co-stimulation as opposed to co-inhibition of immune responses.

The term "biomarker" or "marker" refers to a measurable entity of the present invention that has been determined to be indicative of T cell exhaustion. For example, biomarkers described herein can be genomic regulatory regions that modulate the expression of at least one gene in a T cell. In another embodiment according to the disclosure, biomarkers described herein can be effector genes or products thereof express by T cells and related to T cell activity and/or T cell exhaustion (*e.g*., high sustained PD-1 expression and/or activity in exhausted T cells. Biomarkers can also include, without limitation, cell types (*e.g.,* engineered T cells), cell ratios (*e.g.,* engineered T cells to exhausted T cell ratio), nucleic acids (*e.g.,* genomic nucleic acids and/or transcribed nucleic acids) and proteins, particularly those provided in Table 1. Biomarkers can further include immunological targets or agents that downregulate unwanted immune reactions in order to treat the immune disorder of interest as described further herein. The modulation (*e.g.,* increase or decrease) in biomarker activity can be measured in any number of ways (*e.g.,* according to measures described herein, including using controls, ratios, comparisons to baselines, and the like). For example, a genomic regulatory region selectively chromatin accessible in exhausted CD8+ T cells that is engineered can decrease enhancer activity on at least one gene as measured by a reduction in gene expression (*e.g.*, gene transcription and/or translation) of the at least one gene as compared to the transcription and/or translation of the at least one gene in the same T cell type from the same organism without the engineered genomic regulatory region. The modulation in gene expression can be assessed over time. A modulation can mean a change of at least 1%, 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 200%, 250%, 300%, 350%, 400%, 450%, 500%, 550%, 600%, 650%, 700%, 750%, 800%, 850%, 900%, 950%, 1000%, or more, or any range in between inclusive (*e.g.*, 5% to 100%).

It is to be noted that the biomarkers described herein can be used to refer to any combination of features described herein regarding any individual or combination of such biomarkers. For example, any combination of ortholog across organisms, sequence composition, percentage identity, sequence length, domain structure, functional activity, mutation status, *etc.* can be used to describe a biomarker molecule of the present invention.

A "blocking" antibody or an antibody "antagonist" is one which inhibits or reduces at least one biological activity of the antigen(s) it binds. In certain embodiments according to the disclosure, the blocking antibodies or antagonist antibodies or fragments thereof described herein substantially or completely inhibit a given biological activity of the antigen(s).

The term "body fluid" refers to fluids that are excreted or secreted from the body as well as fluid that are normally not (*e.g*. amniotic fluid, aqueous humor, bile, blood and blood plasma, cerebrospinal fluid, cerumen and earwax, cowper's fluid or pre-ejaculatory fluid, chyle, chyme, stool, female ejaculate, interstitial fluid, intracellular fluid, lymph, menses, breast milk, mucus, pleural fluid, pus, saliva, sebum, semen, serum, sweat, synovial fluid, tears, urine, vaginal lubrication, vitreous humor, and vomit). In certain embodiments according to the disclosure, body fluids comprising lymphocytes, such as T lymphocytes and subpopulations thereof, are used.

The term "bispecific antibody" or "multispecific antibody" refers to an antibody that recognized more than one epitope. Such antibodies are useful for targeting different proteins using the same agent. Methods of making such antibodies are well-known in art (see, at least U.S. Patent 5,798,229; U.S. Patent 5,989,830; and Holliger et al. (2005) Nat. Biotech. 23:1126-1136).

The terms "cancer" or "tumor" or "hyperproliferative disorder" refer to the presence of cells possessing characteristics typical of cancer-causing cells, such as uncontrolled proliferation, immortality, metastatic potential, rapid growth and proliferation rate, and certain characteristic morphological features. Cancer cells are often in the form of a tumor, but such cells may exist alone within an animal, or may be a non-tumorigenic cancer cell, such as a leukemia cell. The term "cancer" includes premalignant, as well as malignant, cancers. The term "pre-malignant lesions" as described herein refers to a lesion that, while not cancerous, has potential for becoming cancerous. It also includes the term "pre-malignant disorders" or "potentially malignant disorders." In particular this refers to a benign, morphologically and/or histologically altered tissue that has a greater than normal risk of malignant transformation, and a disease or a patient's habit that does not necessarily alter the clinical appearance of local tissue but is associated with a greater than normal risk of precancerous lesion or cancer development in that tissue (leukoplakia, erythroplakia, erytroleukoplakia lichen planus (lichenoid reaction) and any lesion or an area which histological examination showed atypia of cells or dysplasia.

Cancers include, but are not limited to, B cell cancer, *e.g.*, multiple myeloma, Waldenström's macroglobulinemia, the heavy chain diseases, such as, for example, alpha chain disease, gamma chain disease, and mu chain disease, benign monoclonal gammopathy, and immunocytic amyloidosis, melanomas, breast cancer, lung cancer, bronchus cancer, colorectal cancer, prostate cancer, pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematologic tissues, and the like. Other non-limiting examples of types of cancers applicable to the methods encompassed by the present invention include human sarcomas and carcinomas, *e.g*., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, colorectal cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, liver cancer, choriocarcinoma, seminoma, embryonal carcinoma, Wilms' tumor, cervical cancer, bone cancer, brain tumor, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, *e.g.,* acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease. In some embodiments according to the disclosure, cancers are epithlelial in nature and include but are not limited to, bladder cancer, breast cancer, cervical cancer, colon cancer, gynecologic cancers, renal cancer, laryngeal cancer, lung cancer, oral cancer, head and neck cancer, ovarian cancer, pancreatic cancer, prostate cancer, or skin cancer. In other embodiments according to the disclosure, the cancer is breast cancer, prostate cancer, lung cancer, or colon cancer. In still other embodiments according to the disclosure, the epithelial cancer is non-small-cell lung cancer, nonpapillary renal cell carcinoma, cervical carcinoma, ovarian carcinoma (*e.g*., serous ovarian carcinoma), or breast carcinoma. The epithelial cancers may be characterized in various other ways including, but not limited to, serous, endometrioid, mucinous, clear cell, Brenner, or undifferentiated.

The term "coding region" refers to regions of a nucleotide sequence comprising codons which are translated into amino acid residues, whereas the term "non-coding region" refers to regions of a nucleotide sequence that are not translated into amino acids (*e.g.*, 5' and 3' untranslated regions).

The term "complementary" refers to the broad concept of sequence complementarity between regions of two nucleic acid strands or between two regions of the same nucleic acid strand. It is known that an adenine residue of a first nucleic acid region is capable of forming specific hydrogen bonds ("base pairing") with a residue of a second nucleic acid region which is antiparallel to the first region if the residue is thymine or uracil. Similarly, it is known that a cytosine residue of a first nucleic acid strand is capable of base pairing with a residue of a second nucleic acid strand which is antiparallel to the first strand if the residue is guanine. A first region of a nucleic acid is complementary to a second region of the same or a different nucleic acid if, when the two regions are arranged in an antiparallel fashion, at least one nucleotide residue of the first region is capable of base pairing with a residue of the second region. Preferably, the first region comprises a first portion and the second region comprises a second portion, whereby, when the first and second portions are arranged in an antiparallel fashion, at least about 50%, and preferably at least about 75%, at least about 90%, or at least about 95% of the nucleotide residues of the first portion are capable of base pairing with nucleotide residues in the second portion. More preferably, all nucleotide residues of the first portion are capable of base pairing with nucleotide residues in the second portion.

The term "control" refers to any reference standard suitable to provide a comparison to the regulatory and/or expression products in the test sample. For efficiency, expression products are described, but the description applies equally to elements that regulate the expression products. In one embodiment according to the disclosure, the control comprises obtaining a "control sample" from which expression product levels are detected and compared to the expression product levels from the test sample. Such a control sample may comprise any suitable sample, including but not limited to a sample from a control immune disorder patient (can be stored sample or previous sample measurement) with a known outcome; normal tissue or cells isolated from a subject, such as a normal patient or the immune disorder patient, cultured primary cells/tissues isolated from a subject such as a normal subject or the immune disorder patient, adjacent normal cells/tissues obtained from the same organ or body location of the immune disorder patient, a tissue or cell sample isolated from a normal subject, or a primary cells/tissues obtained from a depository. In another preferred embodiment according to the disclosure, the control may comprise a reference standard expression product level from any suitable source, including but not limited to housekeeping genes, an expression product level range from normal tissue (or other previously analyzed control sample), a previously determined expression product level range within a test sample from a group of patients, or a set of patients with a certain outcome (for example, survival for one, two, three, four years, etc.) or receiving a certain treatment (for example, standard of care immune disorder therapy). It will be understood by those of skill in the art that such control samples and reference standard expression product levels can be used in combination as controls in the methods of the present invention. In one embodiment according to the disclosure, the control may comprise normal or non-immune disorder cell/tissue sample. In another preferred embodiment according to the disclosure, the control may comprise an expression level for a set of patients, such as a set of immune disorder patients, or for a set of immune disorder patients receiving a certain treatment, or for a set of patients with one outcome versus another outcome. In the former case, the specific expression product level of each patient can be assigned to a percentile level of expression, or expressed as either higher or lower than the mean or average of the reference standard expression level. In another preferred embodiment according to the disclosure, the control may comprise normal cells, cells from patients treated with combination chemotherapy, and cells from patients having an immune disorder that has responded to a treatment of interest. In another embodiment according to the disclosure, the control may also comprise a measured value for example, average level of expression of a particular gene in a population compared to the level of expression of a housekeeping gene in the same population. Such a population may comprise normal subjects, immune disorder patients who have not undergone any treatment (*i.e*., treatment naive), immune disorder patients undergoing standard of care therapy, or patients having an immune disorder that has responded to a treatment of interest. In another preferred embodiment according to the disclosure, the control comprises a ratio transformation of expression product levels, including but not limited to determining a ratio of expression product levels of two cell types and/or genes in the test sample and comparing it to any suitable ratio of the same two cell types and/or genes in a reference standard; determining expression product levels of the two or more cell types and/or genes in the test sample and determining a difference in expression product levels in any suitable control; and determining expression product levels of the two or more cell types and/or genes in the test sample, normalizing their expression to expression of housekeeping cell types and/or genes in the test sample, and comparing to any suitable control. In particularly preferred embodiments according to the disclosure, the control comprises a control sample which is of the same lineage and/or type as the test sample. In another embodiment according to the disclosure, the control may comprise expression product levels grouped as percentiles within or based on a set of patient samples, such as all patients with the immune disorder. In one embodiment according to the disclosure a control expression product level is established wherein higher or lower levels of expression product relative to, for instance, a particular percentile, are used as the basis for predicting outcome. In another preferred embodiment according to the disclosure, a control expression product level is established using expression product levels from immune disorder control patients with a known outcome, and the expression product levels from the test sample are compared to the control expression product level as the basis for predicting outcome. As demonstrated by the data below, the methods of the disclosure are not limited to use of a specific cut-point in comparing the level of expression product in the test sample to the control.

The "copy number" of a biomarker nucleic acid refers to the number of DNA sequences in a cell (*e.g.*, germline and/or somatic) encoding a particular gene product. Generally, for a given gene, a mammal has two copies of each gene. The copy number can be increased, however, by gene amplification or duplication, or reduced by deletion. For example, germline copy number changes include changes at one or more genomic loci, wherein said one or more genomic loci are not accounted for by the number of copies in the normal complement of germline copies in a control *(e.g.,* the normal copy number in germline DNA for the same species as that from which the specific germline DNA and corresponding copy number were determined). Somatic copy number changes include changes at one or more genomic loci, wherein said one or more genomic loci are not accounted for by the number of copies in germline DNA of a control (*e.g*., copy number in germline DNA for the same subject as that from which the somatic DNA and corresponding copy number were determined).

The "normal" copy number (*e.g*., germline and/or somatic) of a biomarker nucleic acid or "normal" level of expression of a biomarker nucleic acid, or protein is the activity/level of expression or copy number in a biological sample, *e.g*., a sample containing tissue, whole blood, serum, plasma, buccal scrape, saliva, cerebrospinal fluid, urine, stool, and bone marrow, from a subject, *e.g*., a human, not afflicted with an immune disorder, or from a corresponding non-immune disorder tissue in the same subject who has an immune disorder.

The term "determining a suitable treatment regimen for the subject" is taken to mean the determination of a treatment regimen *(i.e.,* a single therapy or a combination of different therapies that are used for the prevention and/or treatment of an immune disorder in the subject) for a subject that is started, modified and/or ended based or essentially based or at least partially based on the results of the analysis according to the present invention. One example is determining whether to provide engineered T cells to prevent or reverse T cell exhaustion and/or treat an immune disorder. The determination can, in addition to the results of the analysis according to the present invention such as based on how modulated the gene expression or activity is in the initial and/or administered engineered T cell population, be based on personal characteristics of the subject to be treated. In most cases, the actual determination of the suitable treatment regimen for the subject will be performed by the attending physician or doctor.

The term "expression signature" or "signature" refers to a group of two or more coordinately expressed biomarkers. For example, the genes, proteins, and the like making up this signature may be expressed in a specific cell lineage, stage of differentiation, or during a particular biological response. The biomarkers can reflect biological aspects of the cell types in which they are expressed. Expression data and gene expression levels can be stored on computer readable media, *e.g.,* the computer readable medium used in conjunction with a microarray or chip reading device. Such expression data can be manipulated to generate expression signatures.

A molecule or cell is "fixed" or "affixed" to a substrate if it is covalently or non-covalently associated with the substrate such that the substrate can be rinsed with a fluid (e.g. standard saline citrate, pH 7.4) without a substantial fraction of the molecule or cell dissociating from the substrate.

As used herein, the terms "high," "low," "intermediate," and "negative" in connection with cellular biomarker expression refers to the amount of the biomarker expressed relative to the cellular expression of the biomarker by one or more reference cells. Biomarker expression can be determined according to any method described herein including, without limitation, an analysis of the cellular level, activity, structure, and the like, of one or more biomarker genomic nucleic acids, ribonucleic acids, and/or polypeptides. In one embodiment according to the disclosure, the terms refer to a defined percentage of a population of cells expressing the biomarker at the highest, intermediate, or lowest levels, respectively. Such percentages can be defined as the top 0.1%, 0.5%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10%, 11%, 12%, 13%, 14%, 15% or more, or any range in between, inclusive, of a population of cells that either highly express or weakly express the biomarker. The term "low" excludes cells that do not detectably express the biomarker, since such cells are "negative" for biomarker expression. The term "intermediate" includes cells that express the biomarker, but at levels lower than the population expressing it at the "high" level. In another embodiment according to the disclosure, the terms can also refer to, or in the alternative refer to, cell populations of biomarker expression identified by qualitative or statistical plot regions. For example, cell populations sorted using flow cytometry can be discriminated on the basis of biomarker expression level by identifying distinct plots based on detectable moiety analysis, such as based on mean fluorescence intensities and the like, according to well-known methods in the art. Such plot regions can be refined according to number, shape, overlap, and the like based on well-known methods in the art for the biomarker of interest. In still another embodiment according to the disclosure, the terms can also be determined according to the presence or absence of expression for additional biomarkers.

The term "homologous" refers to nucleotide sequence similarity between two regions of the same nucleic acid strand or between regions of two different nucleic acid strands. When a nucleotide residue position in both regions is occupied by the same nucleotide residue, then the regions are homologous at that position. A first region is homologous to a second region if at least one nucleotide residue position of each region is occupied by the same residue. Homology between two regions is expressed in terms of the proportion of nucleotide residue positions of the two regions that are occupied by the same nucleotide residue. By way of example, a region having the nucleotide sequence 5'-ATTGCC-3' and a region having the nucleotide sequence 5'-TATGGC-3' share 50% homology. Preferably, the first region comprises a first portion and the second region comprises a second portion, whereby, at least about 50%, and preferably at least about 75%, at least about 90%, or at least about 95% of the nucleotide residue positions of each of the portions are occupied by the same nucleotide residue. More preferably, all nucleotide residue positions of each of the portions are occupied by the same nucleotide residue.

As used herein, the term "immune checkpoints" means a group of molecules on the cell surface of CD4+ and CD8+ T cells. These molecules fine-tune immune responses by down-modulating or inhibiting an anti-tumor immune response. Immune checkpoint proteins are well-known in the art and include, without limitation, CTLA-4, PD-1, VISTA, B7-H2, B7-H3, PD-L1, B7-H4, B7-H6, ICOS, HVEM, PD-L2, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, and A2aR (see, for example, WO 2012/177624). Immunotherapeutic agents that can act as immune checkpoint inhibitors useful in the methods of the present invention, include, but are not limited to, Fc fusion proteins having effector function, such as certain classes of antibodies well-known in the art.

The term "anti-immune checkpoint therapy" refers to the use of agents that inhibit immune checkpoint nucleic acids and/or proteins. Inhibition of one or more immune checkpoints can block or otherwise neutralize inhibitory signaling to promote immunomodulation. Exemplary agents useful for inhibiting immune checkpoints include antibodies, small molecules, peptides, peptidomimetics, natural ligands, and derivatives of natural ligands, that can either bind and/or inactivate or inhibit immune checkpoint proteins, or fragments thereof; as well as RNA interference, antisense, nucleic acid aptamers, etc. that can downregulate the expression and/or activity of immune checkpoint nucleic acids, or fragments thereof. Exemplary agents for upregulating an immune response include antibodies against one or more immune checkpoint proteins that block the interaction between the proteins and its natural receptor(s); a non-activating form of one or more immune checkpoint proteins *(e.g.,* a dominant negative polypeptide); small molecules or peptides that block the interaction between one or more immune checkpoint proteins and its natural receptor(s); fusion proteins (*e.g*. the extracellular portion of an immune checkpoint inhibition protein fused to the Fc portion of an antibody or immunoglobulin) that bind to its natural receptor(s); nucleic acid molecules that block immune checkpoint nucleic acid transcription or translation; and the like. Such agents can directly block the interaction between the one or more immune checkpoints and its natural receptor(s) (*e.g.*, antibodies) to prevent inhibitory signaling and upregulate an immune response. Alternatively, agents can indirectly block the interaction between one or more immune checkpoint proteins and its natural receptor(s) to prevent inhibitory signaling and upregulate an immune response. For example, a soluble version of an immune checkpoint protein ligand such as a stabilized extracellular domain can bind to its receptor to indirectly reduce the effective concentration of the receptor to bind to an appropriate ligand. In one embodiment according to the disclosure, anti-PD-1 antibodies, anti-PD-L1 antibodies, and/or anti-PD-L2 antibodies, either alone or in combination, are used to inhibit immune checkpoints. These embodiments are also applicable to specific therapy against particular immune checkpoints, such as the PD-1 pathway (*e.g.*, anti-PD-1 pathway therapy, otherwise known as PD-1 pathway inhibitor therapy).

"PD-1" is an immune checkpoint inhibitor that refers to a member of the immunoglobulin gene superfamily that functions as a co-inhibitory receptor having PD-L1 and PD-L2 as known ligands. PD-1 was previously identified using a subtraction cloning based approach to select for proteins involved in apoptotic cell death. PD-1 is a member of the CD28/CTLA-4 family of molecules based on its ability to bind to PD-L1. Like CTLA-4, PD-1 is rapidly induced on the surface of T-cells in response to anti-CD3 (Agata et al. 25 (1996) Int. Immunol. 8:765). In contrast to CTLA-4, however, PD-1 is also induced on the surface of B-cells (in response to anti-IgM). PD-1 is also expressed on a subset of thymocytes and myeloid cells (Agata *et al.* (1996) *supra;* Nishimura et al. (1996) Int. Immunol. 8:773).

The nucleic acid and amino acid sequences of a representative human PD-1 biomarker is available to the public at the GenBank database under NM _005018.2 and NP_005009.2 (see also Ishida et al. (1992) 20 EMBO J 11:3887; Shinohara et al. (1994) Genomics 23:704; U.S. Patent 5,698,520). PD-1 has an extracellular region containing immunoglobulin superfamily domain, a transmembrane domain, and an intracellular region including an immunoreceptor tyrosine-based inhibitory motif (ITIM) (Ishida et al. (1992) EMBO J. 11:3887; Shinohara et al. (1994) Genomics 23:704; and U.S. Patent 5,698,520). These features also define a larger family of polypeptides, called the immunoinhibitory receptors, which also includes gp49B, PIR-B, and the killer inhibitory receptors (KIRs) (Vivier and Daeron (1997) Immunol. Today 18:286). It is often assumed that the tyrosyl phosphorylated ITIM motif of these receptors interacts with SH2-domain containing phosphatases, which leads to inhibitory signals. A subset of these immunoinhibitory receptors bind to MHC polypeptides, for example the KIRs, and CTLA4 binds to B7-1 and B7-2. It has been proposed that there is a phylogenetic relationship between the MHC and B7 genes (Henry et al. (1999) Immunol. Today 20(6):285-8). Nucleic acid and polypeptide sequences of PD-1 orthologs in organisms other than humans are well known and include, for example, mouse PD-1 (NM_008798.2 and NP_032824.1), rat PD-1 (NM_001106927.1 and NP_001100397.1), dog PD-1 (XM_543338.3 and XP_543338.3), cow PD-1 (NM_001083506.1 and NP_001076975.1), and chicken PD-1 (XM_422723.3 and XP_422723.2).

PD-1 polypeptides are inhibitory receptors capable of transmitting an inhibitory signal to an immune cell to thereby inhibit immune cell effector function, or are capable of promoting costimulation (e.g., by competitive inhibition) of immune cells, *e.g.,* when present in soluble, monomeric form. Preferred PD-1 family members share sequence identity with PD-1 and bind to one or more B7 family members, *e.g.,* B7-1, B7-2, PD-1 ligand, and/or other polypeptides on antigen presenting cells.

The term "PD-1 activity" includes the ability of a PD-1 polypeptide to modulate an inhibitory signal in an activated immune cell, *e.g.,* by engaging a natural PD-1 ligand on an antigen presenting cell. PD-1 transmits an inhibitory signal to an immune cell in a manner similar to CTLA4. Modulation of an inhibitory signal in an immune cell results in modulation of proliferation of, and/or cytokine secretion by, an immune cell. Thus, the term "PD-1 activity" includes the ability of a PD-1 polypeptide to bind its natural ligand(s), the ability to modulate immune cell costimulatory or inhibitory signals, and the ability to modulate the immune response. Agents that modulate PD-1 activity are well-known in the art. Representative examples include, without limitation, antibodies such as MDX-1106, Merck 3475, and CT-011. MDX-1106, also known as MDX-1106-04, ONO-4538 or BMS-936558, is a fully human IgG4 anti-PD-1 monoclonal antibody described in PCT Publ. No. WO 2006/121168 and U.S. Pat. No. 8,0088,449. Merck 3475, also known as SCH-900475 and pembrolizumab, is a humanized IgG4 anti-PD-1 monoclonal antibody described in PCT Publ. No. WO 2009/114335; U.S. Pat. No. 8,354,509; and Hamid et al. (2013) New Engl. J. Med. 369:134-144. Pidilizumab (CT-011; CureTech) is a humanized IgGl monoclonal antibody that binds to PD-1. Pidilizumab and other humanized anti-PD-1 monoclonal antibodies are disclosed in PCT Publ. No. WO 2009/101611. Similarly, AMP-224 (B7-DCIg; Amplimmune) is a PD-L2 Fc fusion soluble receptor that blocks the interaction between PD-1 and PD-L1 and is disclosed in PCT Publ. Nos. WO 2010/027827 and WO 2011/066342. Moreover, many other anti-PD-1 Fc fusion proteins are known in the art as described in U.S. Pat. No. 8,609,089; US Pat. Publ. No. 2010/028330; U.S. Pat. Publ. No. 2012-0114649; and PCT Publ. No. WO 2014/089113.

The term "PD-1 ligand" refers to binding partners of the PD-1 receptor and includes both PD-L1 (Freeman et al. (2000) J. Exp. Med. 192:1027) and PD-L2 (Latchman et al. (2001) Nat. Immunol. 2:261). At least two types of human PD-1 ligand polypeptides exist. PD-1 ligand proteins comprise a signal sequence, and an IgV domain, an IgC domain, a transmembrane domain, and a short cytoplasmic tail. Both PD-L1 (See Freeman et al. (2000) J. Exp. Med. 192:1027 for sequence data) and PD-L2 (See Latchman et al. (2001) Nat. Immunol. 2:261 for sequence data) are members of the B7 family of polypeptides. Both PD-L1 and PD-L2 are expressed in placenta, spleen, lymph nodes, thymus, and heart. Only PD-L2 is expressed in pancreas, lung and liver, while only PD-L1 is expressed in fetal liver. Both PD-1 ligands are upregulated on activated monocytes and dendritic cells, although PD-L1 expression is broader. For example, PD-L1 is known to be constitutively expressed and upregulated to higher levels on murine hematopoietic cells (*e.g*., T cells, B cells, macrophages, dendritic cells (DCs), and bone marrow-derived mast cells) and non-hematopoietic cells (*e.g*., endothelial, epithelial, and muscle cells), whereas PD-L2 is inducibly expressed on DCs, macrophages, and bone marrow-derived mast cells (see, Butte et al. (2007) Immunity 27:111).

PD-1 ligands comprise a family of polypeptides having certain conserved structural and functional features. The term "family" when used to refer to proteins or nucleic acid molecules, is intended to mean two or more proteins or nucleic acid molecules having a common structural domain or motif and having sufficient amino acid or nucleotide sequence homology, as defined herein. Such family members can be naturally or non-naturally occurring and can be from either the same or different species. For example, a family can contain a first protein of human origin, as well as other, distinct proteins of human origin or alternatively, can contain homologues of non-human origin. Members of a family may also have common functional characteristics. PD-1 ligands are members of the B7 family of polypeptides. The term "B7 family" or "B7 polypeptides" as used herein includes costimulatory polypeptides that share sequence homology with B7 polypeptides, *e.g.,* with B7-1 (CD80), B7-2 (CD86), inducible costimulatory ligand (ICOS-L), B7-H3, B7-H4, VISTA, B7-H6, B7h (Swallow et al. (1999) Immunity 11:423), and/or PD-1 ligands (*e.g*., PD-L1 or PD-L2). For example, human B7-1 and B7-2 share approximately 26% amino acid sequence identity when compared using the BLAST program at NCBI with the default parameters (Blosum62 matrix with gap penalties set at existence 11 and extension 1 (see the NCBI website). The term B7 family also includes variants of these polypeptides which are capable of modulating immune cell function. The B7 family of molecules share a number of conserved regions, including signal domains, IgV domains and the IgC domains. IgV domains and the IgC domains are art-recognized Ig superfamily member domains. These domains correspond to structural units that have distinct folding patterns called Ig folds. Ig folds are comprised of a sandwich of two β sheets, each consisting of anti-parallel β strands of 5-10 amino acids with a conserved disulfide bond between the two sheets in most, but not all, IgC domains of Ig, TCR, and MHC molecules share the same types of sequence patterns and are called the C1-set within the Ig superfamily. Other IgC domains fall within other sets. IgV domains also share sequence patterns and are called V set domains. IgV domains are longer than IgC domains and contain an additional pair of β strands.

The term "immune disorders" refers to conditions characterized by an unwanted immune response. In some embodiments according to the disclosure, the immune disorder is such that a desired anti-immune disorder response suppresses immune responses. Such conditions in which downregulation of an immune response is desired are well-known in the art and include, without limitation, situations of tissue, skin and organ transplantation, in graft-versus-host disease (GVHD), inflammation, or in autoimmune diseases, such as systemic lupus erythematosus, multiple sclerosis, allergy, hypersensitivity response, a disorder requiring improved vaccination efficiency, and a disorder requiring increased regulatory T cell production or function, as described further herein. In other embodiments according to the disclosure, the immune disorder is such that a desired response is an increased immune response. Such conditions in which upregulation of an immune response is desired are well-known in the art and include, without limitation, disorders requiring increased CD4+ effector T cell production or function such as combating cancer, infections (*e.g.*, parasitic, bacterial, helminthic, or viral infections), and the like.

The term "acute immune disorder" refers to conditions that can be resolved by an appropriate immune response that eradicates a targeted antigen and host comprising such a targeted antigen, such as a cancer or an infection agent like a virus, bacteria, parasite, mycoplasma, fungus, and the like. Such conditions are relatively brief and last on the order of a few days to a few weeks.

By contrast, the term "chronic immune disorders" refers to those conditions that are not effectively cleared or eliminated by the induction of a host immune response. In chronic immune disorders, a targeted antigen (and/or host comprising the targeted antigen), such as an infectious agent or cancer cell, and the immune response reach equilibrium such that the subject maintains the targeted antigen or host comprising the targeted antigen (*e.g.*, remains infectious or afflicted with cancer) over a long period of time *(i.e.,* a time period of months to years or even a lifetime) without necessarily expressing symptoms. Chronic immune disorders can involve stages of both silent and productive targeted antigen maintenance without rapidly killing or even producing excessive damage of the host cells. Detection of the targeted antigen or host comprising the targeted antigen can be made according to any one of many well-known methods in the art and described, for example, in U.S. Patent Nos. 6,368,832, 6,579,854, and 6,808,710 and U.S. Patent Application Publication Nos. 20040137577, 20030232323, 20030166531, 20030064380, 20030044768, 20030039653, 20020164600, 20020160000, 20020110836, 20020107363, and 200201067. In some embodiments according to the disclosure, chronic immune disorders are the result of infection, such as an infection with a virus including, but not limited to, human immunodeficiency viruses (HIV), hepatitis C viruses (HCV), T-cell leukemia viruses, Epstein-Barr virus, cytomegalovirus, herpesviruses, varicella-zoster virus, measles, papovaviruses, prions, hepatitis viruses, adenoviruses, parvoviruses, papillomaviruses, prions, and the like. Chronic immune disorders include, for example, chronic conditions and latent conditions. As used herein, chronic immune disorders can be limited to chronic conditions, latent conditions, or both.

In a "chronic condition," the targeted antigen can be detected in the subject at all times regardless of whether the signs and symptoms of the disease are present or absent, even for an extended period of time. Non-limiting examples of chronic conditions resulting from infection include hepatitis B (caused by hepatitis B virus (HBV)) and hepatitis C (caused by hepatitis C virus (HCV)) adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyoma virus BK, polyoma virus JC, measles virus, rubella virus, human immunodeficiency virus (HIV), human T cell leukemia virus I, and human T cell leukemia virus II. Parasitic persistent infections can arise as a result of infection by, for example, Leishmania, Toxoplasma, Trypanosoma, Plasmodium, Schistosoma, and Encephalitozoon.

A particular type of chronic condition involving infections is known as a "latent condition," where the infectious agent (such as a virus) is seemingly inactive and dormant such that the subject does not always exhibit signs or symptoms. In a latent viral infection, the virus remains in equilibrium with the host for long periods of time before symptoms again appear; however, the actual viruses cannot typically be detected until reactivation of the disease occurs. Infection latency is the ability of a pathogenic infection agent, such as a virus, to lie dormant within a cell. For example, a latent viral infection is a phase in the life cycle of certain viruses in which after initial infection, virus production ceases. However, the virus genome is not fully eradicated. The result of this is that the virus can reactivate and begin producing large amounts of viral progeny (the lytic part of the viral life cycle) without the host being infected by a new virus. The virus may stay within the host indefinitely. In one embodiment according to the disclosure, virus latency is not identical to clinical latency, in which the virus is undergoing an incubation period but is not dormant. Non-limiting examples of latent infections include infections caused by herpes simplex virus (HSV)-1 (fever blisters), HSV-2 (genital herpes), and varicella zoster virus VZV (chickenpox-shingles).

As used herein, the term "immunotherapeutic agent" can include any molecule, peptide, antibody or other agent which can stimulate a host immune system to promote immunomodulation in the subject. Various immunotherapeutic agents are useful in the compositions and methods described herein.

An "inducible" promoter is a nucleotide sequence which, when operably linked with a polynucleotide which encodes or specifies a gene product, causes the gene product to be produced in a living cell substantially only when an inducer, such as a chemical, regulatory element, or the like, is present in the cell.

The term "inhibit" includes the decrease, limitation, or blockage, of, for example a particular action, function, or interaction. In some embodiments according to the disclosure, an immune disorder is "inhibited" if at least one symptom of the immune disorder is alleviated, terminated, slowed, or prevented. As used herein, an immune disorder is also "inhibited" if recurrence or spread of the immune disorder is reduced, slowed, delayed, or prevented.

The term "interaction", when referring to an interaction between two molecules, refers to the physical contact (*e.g*., binding) of the molecules with one another. Generally, such an interaction results in an activity (which produces a biological effect) of one or both of said molecules.

An "isolated antibody" is intended to refer to an antibody that is substantially free of other antibodies having different antigenic specificities. Moreover, an isolated antibody may be substantially free of other cellular material and/or chemicals.

An "isolated protein" refers to a protein that is substantially free of other proteins, cellular material, separation medium, and culture medium when isolated from cells or produced by recombinant DNA techniques, or chemical precursors or other chemicals when chemically synthesized. An "isolated" or "purified" protein or biologically active portion thereof is substantially free of cellular material or other contaminating proteins from the cell or tissue source from which the antibody, polypeptide, peptide or fusion protein is derived, or substantially free from chemical precursors or other chemicals when chemically synthesized. The language "substantially free of cellular material" includes preparations of a biomarker polypeptide or fragment thereof, in which the protein is separated from cellular components of the cells from which it is isolated or recombinantly produced. In one according to the disclosure, the language "substantially free of cellular material" includes preparations of a biomarker protein or fragment thereof, having less than about 30% (by dry weight) of non-biomarker protein (also referred to herein as a "contaminating protein"), more preferably less than about 20% of non-biomarker protein, still more preferably less than about 10% of non-biomarker protein, and most preferably less than about 5% non-biomarker protein. When antibody, polypeptide, peptide or fusion protein or fragment thereof, *e.g.,* a biologically active fragment thereof, is recombinantly produced, it is also preferably substantially free of culture medium, *i.e*., culture medium represents less than about 20%, more preferably less than about 10%, and most preferably less than about 5% of the volume of the protein preparation.

As used herein, the term "K_{D}" is intended to refer to the dissociation equilibrium constant of a particular antibody-antigen interaction. The binding affinity of antibodies of the disclosed invention may be measured or determined by standard antibody-antigen assays, for example, competitive assays, saturation assays, or standard immunoassays such as ELISA or RIA.

A "kit" is any manufacture (*e.g*. a package or container) comprising at least one reagent, *e.g.* a therapeutic, probe, small molecule, and the like, for specifically detecting and/or therapeutically affecting the expression of a marker of the present invention. The kit may be promoted, distributed, or sold as a unit for performing the methods of the present invention. The kit may comprise one or more reagents necessary to express a composition useful in the methods of the present invention. In certain embodiments according to the disclosure, the kit may further comprise a reference standard, *e.g.,* a nucleic acid encoding a protein that does not affect or regulate signaling pathways controlling immunological responses, cell growth, division, migration, survival, or apoptosis. One skilled in the art can envision many such control proteins, including, but not limited to, common molecular tags (e.g., green fluorescent protein and beta-galactosidase), proteins not classified in any of pathway encompassing cell growth, division, migration, survival or apoptosis by GeneOntology reference, or ubiquitous housekeeping proteins. Reagents in the kit may be provided in individual containers or as mixtures of two or more reagents in a single container. In addition, instructional materials which describe the use of the compositions within the kit can be included.

The term "neoadjuvant therapy" refers to a treatment given before the primary treatment. Examples of neoadjuvant therapy can include chemotherapy, radiation therapy, and hormone therapy. For example, in treating breast cancer, neoadjuvant therapy can allow patients with large breast cancer to undergo breast-conserving surgery.

The "normal" level of expression of a biomarker is the level of expression of the biomarker in relevant cells such as those of a subject, *e.g.,* a human patient, as compared to a control, such as the cells not engineered to modulate a genomic gene expression regulatory region and/or cells from a subject not afflicted with an immune disorder. An "over-expression" or "significantly higher level of expression" of a biomarker refers to an expression level in a test sample that is greater than the standard error of the assay employed to assess expression, and is preferably at least 10%, and more preferably 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more higher than the expression activity or level of the biomarker in a control sample (e.g., sample from a healthy subject not having the biomarker associated disease) and preferably, the average expression level of the biomarker in several control samples. A "significantly lower level of expression" of a biomarker refers to an expression level in a test sample that is at least 10%, and more preferably 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more lower than the expression level of the biomarker in a control sample (*e.g*., sample from a healthy subject not having the biomarker associated disease) and preferably, the average expression level of the biomarker in several control samples. Such "significance" levels can also be applied to any other measured parameter described herein, such as for expression, inhibition, cytotoxicity, cell growth, cell ratios, and the like.

The term "pre-determined" biomarker amount and/or activity measurement(s) may be a biomarker amount and/or activity measurement(s) used to, by way of example only, evaluate a subject that may be selected for a particular treatment, evaluate a response to a treatment, and/or evaluate the disease state. A pre-determined biomarker amount and/or activity measurement(s) may be determined in populations of cells or patients, either with or without an immune disorder. The pre-determined biomarker amount and/or activity measurement(s) can be a single number, equally applicable to every patient, or the pre-determined biomarker amount and/or activity measurement(s) can vary according to specific subpopulations of patients. Age, weight, height, and other factors of a subject may affect the pre-determined biomarker amount and/or activity measurement(s) of the individual. Furthermore, the pre-determined biomarker amount and/or activity can be determined individually for each cell, cell line, subject, and the like. In one embodiment, the amounts determined and/or compared in a method described herein are based on absolute measurements. In another embodiment according to the disclosure, the amounts determined and/or compared in a method described herein are based on relative measurements, such as ratios (*e.g.*, serum biomarker normalized to the expression of a housekeeping or otherwise generally constant biomarker). The pre-determined biomarker amount and/or activity measurement(s) can be any suitable standard. For example, the pre-determined biomarker amount and/or activity measurement(s) can be obtained from the same or a different human for whom a patient selection is being assessed. In one embodiment according to the disclosure, the pre-determined biomarker amount and/or activity measurement(s) can be obtained from a previous assessment of the same patient. In such a manner, the progress of the selection of the patient can be monitored over time. In addition, the control can be obtained from an assessment of another human or multiple humans, e.g., selected groups of humans, if the subject is a human. In such a manner, the extent of the selection of the human for whom selection is being assessed can be compared to suitable other humans, *e.g.,* other humans who are in a similar situation to the human of interest, such as those suffering from similar or the same condition(s) and/or of the same ethnic group.

The term "predictive" includes the use of a biomarker nucleic acid, protein, and/or regulatory status, *e.g.,* over- or under- activity of gene expression regulation, as well as emergence, expression, growth, remission, recurrence or resistance of tumors before, during or after therapy, for determining the likelihood of T cell exhaustion prevention, T cell exhaustion reversal, response of an immune disorder to treatment, such as engineered T cell therapy with or without additional anti-immune disorder therapy such as anti-immune checkpoint inhibitor treatment. Such predictive use of the biomarker may be confirmed by, *e.g.,* (1) increased or decreased copy number (*e.g.*, by FISH, FISH plus SKY, single-molecule sequencing, *e.g.,* as described in the art at least at J. Biotechnol., 86:289-301, or qPCR), overexpression or underexpression of a biomarker nucleic acid (*e.g*., by ISH, Northern Blot, or qPCR), increased or decreased biomarker protein (*e.g*., by IHC) and/or biomarker metabolite, or increased or decreased activity (determined by, for example, modulation of biomarkers, *e.g.,* in more than about 5%, 6%, 7%, 8%, 9%, 10%, 11%, 12%, 13%, 14%, 15%, 20%, 25%, 30%, 40%, 50%, 60%, 70%, 80%, 90%, 95%, 100%, or more of assayed relevant human chronic immune disorder types or samples; (2) its absolute or relatively modulated presence or absence in a biological sample, *e.g.,* a sample containing tissue, whole blood, serum, plasma, buccal scrape, saliva, cerebrospinal fluid, urine, stool, or bone marrow, from a subject, *e.g.* a human, afflicted with the chronic immune disorder; (3) its absolute or relatively modulated presence or absence in clinical subset of patients with the immune disorder (*e.g.,* those responding to a therapy or those developing resistance thereto).

The terms "prevent," "preventing," "prevention," "prophylactic treatment," and the like refer to reducing the probability of developing a disease, disorder, or condition in a subject, who does not have, but is at risk of or susceptible to developing a disease, disorder, or condition.

The term "probe" refers to any molecule which is capable of selectively binding to a specifically intended target molecule, for example, a nucleotide transcript or protein encoded by or corresponding to a biomarker nucleic acid. Probes can be either synthesized by one skilled in the art, or derived from appropriate biological preparations. For purposes of detection of the target molecule, probes may be specifically designed to be labeled, as described herein. Examples of molecules that can be utilized as probes include, but are not limited to, RNA, DNA, proteins, antibodies, and organic molecules.

The term "prognosis" includes a prediction of the probable course and outcome of an immune disorder or the likelihood of recovery from the disease. In some embodiments according to the disclosure, the use of statistical algorithms provides a prognosis of the immune disorder in an individual. For example, the prognosis can be surgery, development of a clinical subtype of the immune disorder (*e.g.*, cancer or chronic infection), development of one or more clinical factors, or recovery from the disease.

The term "resistance" refers to an acquired or natural resistance of an immune disorder sample or a mammal to an anti-immune disorder therapy *(i.e.,* being nonresponsive to or having reduced or limited response to the therapeutic treatment), such as having a reduced response to a therapeutic treatment by 25% or more, for example, 30%, 40%, 50%, 60%, 70%, 80%, or more, to 2-fold, 3-fold, 4-fold, 5-fold, 10-fold, 15-fold, 20-fold or more. The reduction in response can be measured by comparing with the same immune disorder sample or mammal before the resistance is acquired, or by comparing with a different immune disorder sample or a mammal who is known to have no resistance to the therapeutic treatment. A typical acquired resistance to chemotherapy is called "multidrug resistance." The multidrug resistance can be mediated by P-glycoprotein or can be mediated by other mechanisms, or it can occur when a mammal is infected with a multidrug-resistant microorganism or a combination of microorganisms. The determination of resistance to a therapeutic treatment is routine in the art and within the skill of an ordinarily skilled clinician, for example, can be measured by cell proliferative assays and cell death assays as described herein as "sensitizing." In some embodiments according to the disclosure, the term "reverses resistance" means that the use of a second agent in combination with a primary anti-immune disorder therapy (*e.g.*, anti-immune checkpoint inhibitor, chemotherapeutic, and/or radiation therapy) is able to produce a significant decrease in immune disordered tissue at a level of statistical significance (*e.g.*, p<0.05) when compared to immune disordered tissue in the circumstance where the primary therapy alone is unable to produce a statistically significant decrease. For example, this generally applies to tumor volume measurements made at a time when the untreated tumor is growing logarithmically.

The term "response to therapy" relates to any response of an immune disorder to therapy, such as engineered T cell therapy, preferably to a change in symptoms, such as reduced infection or viral load, tumor mass and/or volume after initiation of neoadjuvant or adjuvant chemotherapy, and the like. T cell function, such as CD4+ and/or CD8+ effector function, as well as antigen-specific function thereof, can be assessed according to numerous assays well-known in the art and/or described herein. Hyperproliferative disorder response may be assessed, for example for efficacy or in a neoadjuvant or adjuvant situation, where the size of a tumor after systemic intervention can be compared to the initial size and dimensions as measured by CT, PET, mammogram, ultrasound or palpation. Responses may also be assessed by caliper measurement or pathological examination of the tumor after biopsy or surgical resection. Response may be recorded in a quantitative fashion like percentage change in tumor volume or in a qualitative fashion like "pathological complete response" (pCR), "clinical complete remission" (cCR), "clinical partial remission" (cPR), "clinical stable disease" (cSD), "clinical progressive disease" (cPD) or other qualitative criteria. Assessment of hyperproliferative disorder response may be done early after the onset of neoadjuvant or adjuvant therapy, *e.g.,* after a few hours, days, weeks or preferably after a few months. A typical endpoint for response assessment is upon termination of neoadjuvant chemotherapy or upon surgical removal of residual tumor cells and/or the tumor bed. This is typically three months after initiation of neoadjuvant therapy. In some embodiments according to the disclosure, clinical efficacy of the therapeutic treatments described herein may be determined by measuring the clinical benefit rate (CBR). The clinical benefit rate is measured by determining the sum of the percentage of patients who are in complete remission (CR), the number of patients who are in partial remission (PR) and the number of patients having stable disease (SD) at a time point at least 6 months out from the end of therapy. The shorthand for this formula is CBR=CR+PR+SD over 6 months. In some embodiments according to the disclosure, the CBR for a particular cancer therapeutic regimen is at least 25%, 30%, 35%, 40%, 45%, 50%, 55%, 60%, 65%, 70%, 75%, 80%, 85%, or more. Additional criteria for evaluating the response to cancer therapies are related to "survival," which includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point *(e.g.,* time of diagnosis or start of treatment) and end point *(e.g.,* death, recurrence or metastasis). In addition, criteria for efficacy of treatment can be expanded to include response to chemotherapy, probability of survival, probability of metastasis within a given time period, and probability of tumor recurrence. For example, in order to determine appropriate threshold values, a particular cancer therapeutic regimen can be administered to a population of subjects and the outcome can be correlated to biomarker measurements that were determined prior to administration of any cancer therapy. The outcome measurement may be pathologic response to therapy given in the neoadjuvant setting. Alternatively, outcome measures, such as overall survival and disease-free survival can be monitored over a period of time for subjects following cancer therapy for whom biomarker measurement values are known. In certain embodiments according to the disclosure, the doses administered are standard doses known in the art for cancer therapeutic agents. The period of time for which subjects are monitored can vary. For example, subjects may be monitored for at least 2, 4, 6, 8, 10, 12, 14, 16, 18, 20, 25, 30, 35, 40, 45, 50, 55, or 60 months. Biomarker measurement threshold values that correlate to outcome of a cancer therapy can be determined using well-known methods in the art, such as those described in the Examples section.

An "RNA interfering agent" as used herein, is defined as any agent which interferes with or inhibits expression of a target biomarker gene by RNA interference (RNAi). Such RNA interfering agents include, but are not limited to, nucleic acid molecules including RNA molecules which are homologous to the target biomarker gene of the invention, or a fragment thereof, short interfering RNA (siRNA), and small molecules which interfere with or inhibit expression of a target biomarker nucleic acid by RNA interference (RNAi).

"RNA interference (RNAi)" is an evolutionally conserved process whereby the expression or introduction of RNA of a sequence that is identical or highly similar to a target biomarker nucleic acid results in the sequence specific degradation or specific post-transcriptional gene silencing (PTGS) of messenger RNA (mRNA) transcribed from that targeted gene (*see* Coburn, G. and Cullen, B. (2002) J. of Virology 76(18):9225), thereby inhibiting expression of the target biomarker nucleic acid. In one embodiment according to the disclosure, the RNA is double stranded RNA (dsRNA). This process has been described in plants, invertebrates, and mammalian cells. In nature, RNAi is initiated by the dsRNA-specific endonuclease Dicer, which promotes processive cleavage of long dsRNA into double-stranded fragments termed siRNAs. siRNAs are incorporated into a protein complex that recognizes and cleaves target mRNAs. RNAi can also be initiated by introducing nucleic acid molecules, e.g., synthetic siRNAs, shRNAs, or other RNA interfering agents, to inhibit or silence the expression of target biomarker nucleic acids. As used herein, "inhibition of target biomarker nucleic acid expression" or "inhibition of marker gene expression" includes any decrease in expression or protein activity or level of the target biomarker nucleic acid or protein encoded by the target biomarker nucleic acid. The decrease may be of at least 30%, 40%, 50%, 60%, 70%, 80%, 90%, 95% or 99% or more as compared to the expression of a target biomarker nucleic acid or the activity or level of the protein encoded by a target biomarker nucleic acid which has not been targeted by an RNA interfering agent.

The term "sample" used for detecting or determining the presence or level of at least one biomarker is typically whole blood, plasma, serum, saliva, urine, stool *(e.g.,* feces), tears, and any other bodily fluid *(e.g.,* as described above under the definition of "body fluids"), or a tissue sample (*e.g.*, biopsy) such as a small intestine, colon sample, or surgical resection tissue. In certain instances, the method of the present invention further comprises obtaining the sample from the individual prior to detecting or determining the presence or level of at least one marker in the sample. In some embodiments according to the disclosure, any sample comprising T lymphocytes or subsets thereof are useful according to the present invention.

The term "sensitize" means to alter immune disordered cells, such as infected or cancer cells, in a way that allows for more effective treatment of the associated immune disorder with a therapy (*e.g.*, engineered T cell therapy). In some embodiments according to the disclosure, normal cells are not affected to an extent that causes the normal cells to be unduly injured by the therapy. An increased sensitivity or a reduced sensitivity to a therapeutic treatment is measured according to a known method in the art for the particular treatment and methods described herein below, including, but not limited to, cell proliferative assays (Tanigawa N, Kern D H, Kikasa Y, Morton D L, Cancer Res 1982; 42: 2159-2164), cell death assays (Weisenthal L M, Shoemaker R H, Marsden J A, Dill P L, Baker J A, Moran E M, Cancer Res 1984; 94: 161-173; Weisenthal L M, Lippman M E, Cancer Treat Rep 1985; 69: 615-632; Weisenthal L M, In: Kaspers G J L, Pieters R, Twentyman P R, Weisenthal L M, Veerman A J P, eds. Drug Resistance in Leukemia and Lymphoma. Langhorne, P A: Harwood Academic Publishers, 1993: 415-432; Weisenthal L M, Contrib Gynecol Obstet 1994; 19: 82-90). The sensitivity or resistance may also be measured in animal by measuring chronic immune disorder symptom reduction over a period of time, for example, 6 months for a human and 4-6 weeks for a mouse. A composition or a method sensitizes response to a therapeutic treatment if the increase in treatment sensitivity or the reduction in resistance is 25% or more, for example, 30%, 40%, 50%, 60%, 70%, 80%, or more, to 2-fold, 3-fold, 4-fold, 5-fold, 10-fold, 15-fold, 20-fold or more, compared to treatment sensitivity or resistance in the absence of such composition or method. The determination of sensitivity or resistance to a therapeutic treatment is routine in the art and within the skill of an ordinarily skilled clinician. It is to be understood that any method described herein for enhancing the efficacy of a cancer therapy can be equally applied to methods for sensitizing hyperproliferative or otherwise cancerous cells (*e.g.*, resistant cells) to cancer therapy.

"Short interfering RNA" (siRNA), also referred to herein as "small interfering RNA" is defined as an agent which functions to inhibit expression of a target gene, *e.g.,* by RNAi. An siRNA may be chemically synthesized, may be produced by *in vitro* transcription, or may be produced within a host cell. In one embodiment according to the disclosure, siRNA is a double stranded RNA (dsRNA) molecule of about 15 to about 40 nucleotides in length, preferably about 15 to about 28 nucleotides, more preferably about 19 to about 25 nucleotides in length, and more preferably about 19, 20, 21, or 22 nucleotides in length, and may contain a 3' and/or 5' overhang on each strand having a length of about 0, 1, 2, 3, 4, or 5 nucleotides. The length of the overhang is independent between the two strands, *i.e.,* the length of the overhang on one strand is not dependent on the length of the overhang on the second strand. Preferably the siRNA is capable of promoting RNA interference through degradation or specific post-transcriptional gene silencing (PTGS) of the target messenger RNA (mRNA). In another embodiment according to the disclosure, an siRNA is a small hairpin (also called stem loop) RNA (shRNA). In one embodiment according to the disclosure, these shRNAs are composed of a short *(e.g.,* 19-25 nucleotide) antisense strand, followed by a 5-9 nucleotide loop, and the analogous sense strand. Alternatively, the sense strand may precede the nucleotide loop structure and the antisense strand may follow. These shRNAs may be contained in plasmids, retroviruses, and lentiviruses and expressed from, for example, the pol III U6 promoter, or another promoter (*see, e.g.,* Stewart, et al. (2003) RNA Apr; 9(4):493-501). RNA interfering agents, *e.g.,* siRNA molecules, may be administered to a subject having or at risk for having immune disorder, to inhibit expression of a marker gene of the invention, *e.g.,* a marker gene whose expression or source must be reduced in immune disorder (such as the markers listed in Table 1) and thereby treat, prevent, or inhibit an immune disorder in the subject.

The term "small molecule" is a term of the art and includes molecules that are less than about 1000 molecular weight or less than about 500 molecular weight. In one embodiment according to the disclosure, small molecules do not exclusively comprise peptide bonds. In another embodiment according to the disclosure, small molecules are not oligomeric. Exemplary small molecule compounds which can be screened for activity include, but are not limited to, peptides, peptidomimetics, nucleic acids, carbohydrates, small organic molecules *(e.g.,* polyketides) (Cane et al. (1998) Science 282:63), and natural product extract libraries. In another embodiment according to the disclosure, the compounds are small, organic non-peptidic compounds. In a further embodiment according to the disclosure, a small molecule is not biosynthetic.

The term "subject" refers to any healthy animal, mammal or human, or any animal, mammal or human afflicted with an immune disorder. The term "subject" is interchangeable with "patient."

The term "survival" includes all of the following: survival until mortality, also known as overall survival (wherein said mortality may be either irrespective of cause or tumor related); "recurrence-free survival" (wherein the term recurrence shall include both localized and distant recurrence); disease free survival (wherein the term disease shall include immune disorders and diseases associated therewith). The length of said survival may be calculated by reference to a defined start point (*e.g.* time of diagnosis or start of treatment) and end point (*e.g.* death, recurrence or metastasis). In addition, criteria for efficacy of treatment can be expanded to include response to therapy, probability of survival, probability of recurrence within a given time period, and the like.

The term "synergistic effect" refers to the combined effect of two or more anti-immune disorder agents or therapies that can be greater than the sum of the separate effects of each such agent or therapy alone. In some embodiments according to the disclosure, it can provide for similar efficacy of monotherapy but with other unexpected improvements relative to monotherapy, such as reducing unwanted side effects.

The term "therapeutic effect" refers to a local or systemic effect in animals, particularly mammals, and more particularly humans, caused by a pharmacologically active substance. The term thus means any substance intended for use in the diagnosis, cure, mitigation, treatment or prevention of disease or in the enhancement of desirable physical or mental development and conditions in an animal or human. The phrase "therapeutically-effective amount" means that amount of such a therapy or substance that produces some desired local or systemic effect at a reasonable benefit/risk ratio applicable to any treatment. In certain embodiments according to the disclosure, a therapeutically effective amount of a compound will depend on its therapeutic index, solubility, and the like. For example, certain compounds discovered by the methods of the present invention may be administered in a sufficient amount to produce a reasonable benefit/risk ratio applicable to such treatment.

The terms "therapeutically-effective amount" and "effective amount" as used herein means that amount of a compound, material, or composition comprising a compound of the present invention which is effective for producing some desired therapeutic effect in at least a sub-population of cells in an animal at a reasonable benefit/risk ratio applicable to any medical treatment. Toxicity and therapeutic efficacy of subject compounds may be determined by standard pharmaceutical procedures in cell cultures or experimental animals, *e.g.,* for determining the LD₅₀ and the ED₅₀. Compositions that exhibit large therapeutic indices are preferred. In some embodiments according to the disclosure, the LD₅₀ (lethal dosage) can be measured and can be, for example, at least 10%, 20%, 30%, 40%, 50%, 60%, 70%, 80%, 90%, 100%, 200%, 300%, 400%, 500%, 600%, 700%, 800%, 900%, 1000% or more reduced for the agent relative to no administration of the agent. Similarly, the ED₅₀ (*i.e.,* the concentration which achieves a half-maximal inhibition of symptoms) can be measured and can be, for example, at least 10%, 20%, 30%, 40%, 50%, 60%, 70%, 80%, 90%, 100%, 200%, 300%, 400%, 500%, 600%, 700%, 800%, 900%, 1000% or more increased for the agent relative to no administration of the agent. Also, similarly, the IC₅₀ *(i.e.,* the concentration which achieves a half-maximal effect, such as cytotoxic or cytostatic effect on cancer cells or inhibition of viral replication or load) can be measured and can be, for example, at least 10%, 20%, 30%, 40%, 50%, 60%, 70%, 80%, 90%, 100%, 200%, 300%, 400%, 500%, 600%, 700%, 800%, 900%, 1000% or more increased for the agent relative to no administration of the agent. In some embodiments according to the disclosure, an effect in an assay can be inhibited by at least about 10%, 15%, 20%, 25%, 30%, 35%, 40%, 45%, 50%, 55%, 60%, 65%, 70%, 75%, 80%, 85%, 90%, 95%, or even 100%. In another embodiment according to the disclosure, at least about a 10% , 15%, 20%, 25%, 30%, 35%, 40%, 45%, 50%, 55%, 60%, 65%, 70%, 75%, 80%, 85%, 90%, 95%, or even 100% decrease in a malignancy or viral load can be achieved.

A "tissue-specific" promoter is a nucleotide sequence which, when operably linked with a polynucleotide which encodes or specifies a gene product, causes the gene product to be produced in a living human cell substantially only if the cell is a cell of the tissue type corresponding to the promoter. For example, Foxp3, CD25, or other Treg-selective or Treg-specific promoter can be used to express a polynucleotide selective or specifically within Tregs.

A "transcribed polynucleotide" or "nucleotide transcript" is a polynucleotide (*e.g*. an mRNA, hnRNA, a cDNA, or an analog of such RNA or cDNA) which is complementary to or homologous with all or a portion of a mature mRNA made by transcription of a biomarker nucleic acid and normal post-transcriptional processing (*e.g.* splicing), if any, of the RNA transcript, and reverse transcription of the RNA transcript.

As used herein, the term "unresponsiveness" or "tolerance" includes refractivity of immune cells to stimulation, *e.g.,* stimulation via an activating receptor or a cytokine. Unresponsiveness can occur, *e.g.,* because of exposure to immunosuppressants or exposure to high doses of antigen. As used herein, the term "anergy" or "tolerance" includes refractivity to activating receptor-mediated stimulation. Such refractivity is generally antigen-specific and persists after exposure to the tolerizing antigen has ceased. For example, anergy in T cells (as opposed to unresponsiveness) is characterized by lack of cytokine production, *e.g.,* IL-2. T cell anergy occurs when T cells are exposed to antigen and receive a first signal (a T cell receptor or CD-3 mediated signal) in the absence of a second signal (*e.g*., a costimulatory signal). Under these conditions, reexposure of the cells to the same antigen (even if reexposure occurs in the presence of a costimulatory polypeptide) results in failure to produce cytokines and, thus, failure to proliferate. Anergic T cells can, however, proliferate if cultured with cytokines (*e.g.*, IL-2). For example, T cell anergy can also be observed by the lack of IL-2 production by T lymphocytes as measured by ELISA or by a proliferation assay using an indicator cell line. Alternatively, a reporter gene construct can be used. For example, anergic T cells fail to initiate IL-2 gene transcription induced by a heterologous promoter under the control of the 5' IL-2 gene enhancer or by a multimer of the AP1 sequence that can be found within the enhancer (Kang et al. (1992) Science 257:1134).

As used herein, the term "vector" refers to a nucleic acid capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g*., bacterial vectors having a bacterial origin of replication and episomal mammalian vectors). Other vectors (*e.g.*, non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" or simply "expression vectors". In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (*e.g*., replication defective retroviruses, adenoviruses and adeno-associated viruses), which serve equivalent functions.

### II. T cell chromatin accessible genomic gene expression regulatory regions

"Chromatin accessible" genomic regions identify gene expression regulatory regions because they provide physical space for transcription factors to bind and mediate regulation of gene expression. As described further below in the Examples, various sets of chromatin accessible regulatory regions of different types of T cells (*e.g.*, naive CD8+ T cells, highly functional effector CD8+ T cells, memory CD8+ T cells, and exhausted CD8+ T cells) were analyzed and compared against one another. Many chromatin accessible genomic gene expression regulatory regions were determined to be selectively or specifically present within a given T cell type of interest. Tables 1A-1K provided below describe the chromatin accessible genomic gene expression regulatory regions that are selective and/or specific to exhausted CD8+ T cells and are useful according to the embodiments of the present disclosure described herein. These regions are selectively and/or specifically available in exhausted CD8+ T cells for transcription factors to bind and mediate regulation of gene expression, such as overexpression of PD-1.

The term "specific" refers to an exclusionary action or function. In one example, a genomic gene expression regulatory region specifically present within the genome of an exhausted T cell," such as an exhausted CD8+ T cell, is not present within the genome of a naive T cell, a highly functional effector T cell, or a memory T cell. In another example, specific modulation of a gene's expression in a cell refers to the exclusive modulation of the gene's expression and/or activity in the cell population and not in other cell populations. In still another example, specific binding of an antibody to a predetermined antigen refers to the ability of the antibody to bind to the antigen of interest without binding to other antigens. Typically, the antibody binds with an affinity (K_{D}) of approximately less than 1 × 10⁻⁷ M, such as approximately less than 10⁻⁸ M, 10⁻⁹ M, 10⁻¹⁰ M, 10⁻¹¹ M, or even lower when determined by surface plasmon resonance (SPR) technology in a BIACORE^{®} assay instrument using an antigen of interest as the analyte and the antibody as the ligand, and binds to the predetermined antigen with an affinity that is at least 1.1-, 1.2-, 1.3-, 1.4-, 1.5-, 1.6-, 1.7-, 1.8-, 1.9-, 2.0-, 2.5-, 3.0-, 3.5-, 4.0-, 4.5-, 5.0-, 6.0-, 7.0-, 8.0-, 9.0-, or 10.0-fold or greater than its affinity for binding to a non-specific antigen (e.g., BSA, casein) other than the predetermined antigen or a closely-related antigen. In addition, K_{D} is the inverse of K_{A}. The phrases "an antibody recognizing an antigen" and "an antibody specific for an antigen" are used interchangeably herein with the term "an antibody which binds specifically to an antigen."

By contrast, the term "selective" refers to a preferential action or function. Selective biomarkers also encompass specific markers for purposes of the present invention unless otherwise stated. For example, a genomic gene expression regulatory region "selectively present within the genome of an exhausted T cell," such as an exhausted CD8+ T cell, is not present within the genome of one or more of a naive T cell, a highly functional effector T cell, or a memory T cell. As another example, selective binding is a relative term referring to the ability of an antibody to preferentially discriminate the binding of one antigen over another. Bispecific or multispecific antibodies can selectively target certain antigens or cell populations based upon the increased binding affinities for multiple antigen targets expressed at a single location due to the increased local effective concentration of the multiple antigen targets. The term "selective" can be quantified in terms of the preferential effect in a particular target of interest relative to other targets. For example, a measured variable (e.g., PD-1 expression in engineered T cells versus PD-1 expression in non-engineered T cell) can be 20%, 25%, 30%, 35%, 40%, 45%, 50%, 55%, 60%, 65%, 70%, 75%, 80%, 85%, 90%, 95%, 1-fold, 1.5-fold, 2-fold, 2.5-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, 10-fold, 11-fold, 12-fold, 13-fold, 14-fold, 15-fold, 16-fold, 17-fold, 18-fold, 19-fold, 20-fold, 25-fold, 30-fold, 35-fold, 40-fold, 45-fold, 50-fold, 55-fold, 60-fold, 70-fold, 80-fold, 90-fold, 100-fold, or greater or any range in between inclusive *(e.g.,* 50% to 16-fold), different in a target of interest versus unintended or undesired targets. The same fold analysis can be used to confirm the magnitude of an effect in a given tissue, cell population, measured variable, measured effect, and the like, such as cellular ratios, hyperproliferative cell growth rate or volume, T cell function or rate of proliferation, and the like.

Generally, the term "nucleic acid molecule" is intended to include DNA molecules *(i.e.,* cDNA or genomic DNA) and RNA molecules *(i.e.,* mRNA) and analogs of the DNA or RNA generated using nucleotide analogs. "Genomic nucleic acid" will generally have the same nucleic acid type of the host genome (*e.g.*, double-stranded DNA for human genomic DNA, single-stranded RNA for hepatitis C viral genomic RNA, etc.). The nucleic acid molecule can be single-stranded or double-stranded, but preferably is double-stranded DNA. An "isolated" nucleic acid molecule is one which is separated from other nucleic acid molecules which are present in the natural source of the nucleic acid. Preferably, an "isolated" nucleic acid is free of sequences which naturally flank the nucleic acid (*i.e.,* sequences located at the 5' and 3' ends of the nucleic acid) in the genomic DNA of the organism from which the nucleic acid is derived. For example, in various embodiments according to the disclosure, the isolated nucleic acid molecules corresponding to the one or more biomarkers listed in Table 1 or described herein can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived *(i.e.,* a T cell). Moreover, an "isolated" nucleic acid molecule can be substantially free of other cellular material, or culture medium when produced by recombinant techniques, or chemical precursors or other chemicals when chemically synthesized.

As described above, the present invention contemplates, in addition to the genomic nucleic acid regions shown in Table, genomic nucleic acid sequences comprising the listed regions, or an ortholog thereof within the genome of a mammal other than mice, including humans. In addition, the listed regions, or ortholog thereof, further comprising about 1 bp, 2 bp, 3 bp, 4 bp, 5 bp, 10 bp, 15 bp, 20 bp, 25 bp, 30 bp, 35 bp, 40 bp, 45 bp, 50 bp, 55 bp, 60 bp, 65 bp, 70 bp, 75 bp, 80 bp, 85 bp, 90 bp, 95 bp, 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, 2000 bp, 2100 bp, 2200 bp, 2300 bp, 2400 bp, 2500 bp, 2600 bp, 2700 bp, 2800 bp, 2900 bp, or 3000 bp, or any range in between, inclusive (e.g., 1 bp to 222 bp), on the 5' end, on the 3' end, or on both the 5' and the 3' ends. In addition, any portion or fragment thereof is contemplated. Further contemplated is any genomic nucleic acid sequence comprising a nucleic acid sequence having at least 80%, 81%, 82%, 83%, 84%, 85%, 86%, 87%, 88%, 89%, 90%, 91%, 92%, 93%, 94%, 95%, 96%, 97%, 98%, 99%, 99.5%, or more identity across their full length with such a region, or portion/fragment thereof. The described nucleic acid molecules can have a function of the nucleic acids of the listed regions.

Such regions can be isolated using standard molecular biology techniques and the sequence information provided herein. For example, a human cDNA can be isolated from a human cell line using all or portion of the nucleic acid molecule, or fragment thereof, as a hybridization probe and standard hybridization techniques (*i.e*., as described in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989). Moreover, a nucleic acid molecule of the present invention can be isolated by the polymerase chain reaction using oligonucleotide primers designed based upon the sequence of the one or more biomarkers listed in Table 1, or fragment thereof, or the homologous nucleotide sequence. Synthetic oligonucleotide primers for PCR amplification can be designed according to well-known methods in the art. A nucleic acid of the present invention can be amplified using genomic DNA as a template and appropriate oligonucleotide primers according to standard PCR amplification techniques. The nucleic acid so amplified can be cloned into an appropriate vector and characterized by DNA sequence analysis. Furthermore, oligonucleotides corresponding to the nucleotide sequence of one or more biomarkers listed in Table 1 can be prepared by standard synthetic techniques, *i.e.*, using an automated DNA synthesizer.

Probes based on the nucleotide sequences of one or more biomarkers listed in Table 1 can be used to detect or confirm the desired transcripts or genomic sequences encoding the same or homologous proteins. In preferred embodiments according to the disclosure, the probe further comprises a label group attached thereto, *i.e.,* the label group can be a radioisotope, a fluorescent compound, an enzyme, or an enzyme co-factor. Such probes can be used as a part of a diagnostic test kit for identifying cells or tissue which express one or more biomarkers listed in Table 1, such as by measuring a level of one or more biomarkers nucleic acid in a sample of cells from a subject, *i.e.,* detecting mRNA levels of one or more biomarkers listed in Table 1.

It will be appreciated by those skilled in the art that DNA sequence polymorphisms that lead to changes in the amino acid sequences of the one or more biomarkers listed in Table 1 may exist within a population *(e.g.,* a mammalian and/or human population). Such genetic polymorphisms may exist among individuals within a population due to natural allelic variation. As used herein, the terms "gene" and "recombinant gene" refer to nucleic acid molecules comprising an open reading frame encoding one or more biomarkers listed in Table 1, preferably a mammalian, *e.g.,* human, protein. Such natural allelic variations can typically result in 1-5% variance in the nucleotide sequence of the one or more biomarkers listed in Table 1. Any and all such nucleotide variations and resulting amino acid polymorphisms in the one or more biomarkers listed in Table 1 that are the result of natural allelic variation and that do not alter the functional activity of the one or more biomarkers listed in Table 1 are intended to be within the scope of the invention. Moreover, nucleic acid molecules encoding one or more biomarkers listed in Table 1 from other species.

In addition to naturally-occurring allelic variants of the one or more biomarkers listed in Table 1 that may exist in the population, the skilled artisan will further appreciate that changes can be introduced by mutation into the nucleotide sequence, or fragment thereof, thereby leading to changes in the nucleic acid sequence if the biomarkers listed in Table 1, without altering the functional ability of the biomarkers listed in Table 1.

The comparison of sequences and determination of percent homology between two sequences can be accomplished using a mathematical algorithm. Preferably, the alignment can be performed using the Clustal Method. Multiple alignment parameters include GAP Penalty =10, Gap Length Penalty = 10. For DNA alignments, the pairwise alignment parameters can be Htuple=2, Gap penalty=5, Window=4, and Diagonal saved=4.

Finally, nucleic acid and amino acid sequence information for the loci and biomarkers of the present invention (*e.g*., biomarkers listed in Table 1) are well-known in the art and readily available on publicly available databases, such as the National Center for Biotechnology Information (NCBI) and as described in the Examples.

### III. T cells and genetic modification of T cell genomic gene expression regulatory regions

As used herein, the term "immune cell" refers to cells that play a role in the immune response. Immune cells are of hematopoietic origin, and include lymphocytes, such as B cells and T cells; natural killer cells; myeloid cells, such as monocytes, macrophages, eosinophils, mast cells, basophils, and granulocytes. For example, antigen-reactive T cells are T cells that selectively bind to an antigen of interest and modulate immunological responses based upon the recognition of antigen. Immune cells can be found in the peripheral blood. The term "peripheral blood cell subtypes" refers to cell types normally found in the peripheral blood including, but is not limited to, eosinophils, neutrophils, T cells, monocytes, NK cells, granulocytes, and B cells. Some immune cells are "antigen presenting cells," include professional antigen presenting cells *(e.g.,* B lymphocytes, monocytes, dendritic cells, Langerhans cells), as well as other antigen presenting cells (*e.g*., keratinocytes, endothelial cells, astrocytes, fibroblasts, and oligodendrocytes).

Immune cells mediated immune responses. The term "immune response" refers to a response by a cell of the immune system, such as a B cell, T cell (CD4 or CD8), regulatory T cell, antigen-presenting cell, dendritic cell, monocyte, macrophage, NKT cell, NK cell, basophil, eosinophil, or neutrophil, to a stimulus. In one embodiment according to the disclosure, the response is specific for a particular antigen (an "antigen-specific response"), and refers to a response by a CD4 T cell, CD8 T cell, or B cell via their antigen-specific receptor. In another embodiment according to the disclosure, an immune response is a T cell response, such as a CD4+ response or a CD8+ response. Such responses by these cells can include, for example, cytotoxicity, proliferation, cytokine or chemokine production, trafficking, or phagocytosis, and can be dependent on the nature of the immune cell undergoing the response. In still another embodiment according to the disclosure, an immune response is an effector T cell response, such as occurs when a cytotoxic CD8+ cell produces an antigen-specific response. The term "immune response" includes T cell-mediated and/or B cell-mediated immune responses. Exemplary immune responses include T cell responses, *e.g.,* cytokine production and cellular cytotoxicity. In addition, the term immune response includes immune responses that are indirectly effected by T cell activation, *e.g.,* antibody production (humoral responses) and activation of cytokine responsive cells, *e.g.,* macrophages.

T cells are a class of immune cell and are generally divided into two subclasses, regulatory T cells (Tregs) and conventional T cells (Tconv).

Tregs are naturally occurring CD4+CD25+FOXP3+ T lymphocytes that comprise ∼5-10% of the circulating CD4+ T cell population, act to dominantly suppress autoreactive lymphocytes, and control innate and adaptive immune responses (Piccirillo and Shevach (2004) Semin. Immunol. 16:81-88; Fehervari and Sakaguchi (2004) Curr. Opin. Immunol. 16:203-208; Azuma et al. (2003) Cancer Res. 63:4516-4520; Cederbom et al. (2000) Eur. J. Immunol. 30: 1538-1543; Maloy et al. (2003) J. Exp. Med. 197: 111-119; Serra et al. (2003) Immunity 19:877-889; Thornton and Shevach (1998) J. Exp. Med. 188:287-296; Janssens et al. (2003) J. Immunol. 171:4604-4612; Gasteiger et al. (2013) J. Exp. Med. 210:1167-1178; Sitrin et al. (2013) J. Exp. Med. 210:1153-1165; Schmitt and Williams (2013) Front. Immunol. 4:1-13). Natural Tregs also express low amounts of CD127, develop in the thymus, express GITR and CTLA-4. Induced Tregs are CD4+ T cells that acquire CD25 expression outside of the thymus in the periphery (*e.g.,* mucosa-associated lymphoid tissue (MALT)), express low levels of CD45RB and do not natively express Foxp3 or CD25. Induced Tregs acquire Foxp3, CD25, CTLA-4, and GITR/AITR expression based on the influence of TGFbeta on CD4+ naive conventional T cells in the periphery. Tregs achieve this suppression, at least in part, by inhibiting the proliferation, expansion, and effector activity of conventional T cells (Tcons). Tregs suppress effector T cells from destroying their (self-target, either through cell-cell contact by inhibiting T cell help and activation, through release of immunosuppressive cytokines such as IL-10 or TGF-β, through production of cytotoxic molecules such as Granzyme B, through depleting IL-2 levels, or by changing nutrients in tissues. Depletion of Tregs was shown to enhance IL-2 induced anti-tumor immunity (Imai et al. (2007) Cancer Sci. 98:416-23).

By contrast, conventional T cells, also known as Tconv or Teffs, have effector functions (*e.g.,* cytokine secretion, cytotoxic activity, anti-self-recognition, and the like) to increase immune responses by virtue of their expression of one or more T cell receptors. Tcons or Teffs are generally defined as any T cell population that is not a Treg and include, for example, naive T cells, activated T cells, memory T cells, resting Tcons, or Tcons that have differentiated toward, for example, the Th1 or Th2 lineages. In some embodiments according to the disclosure, Teffs are a subset of non-Treg T cells. In some embodiments according to the disclosure, Teffs are CD4+ Teffs or CD8+ Teffs, such as CD4+ helper T lymphocytes (*e.g.,* Th0, Th1, Tfh, or Th17) and CD8+ cytotoxic T lymphocytes.

"Naive Tcons" are CD4⁺ T cells or CD8+ T cells that have differentiated in bone marrow, and successfully underwent a positive and negative processes of central selection in a thymus, but have not yet been activated by exposure to an antigen. Naive Tcons are commonly characterized by surface expression of L-selectin (CD62L), absence of activation markers, such as CD25, CD44 or CD69, and absence of memory markers, such as CD45RO. Naive Tcons are therefore believed to be quiescent and non-dividing, requiring interleukin-7 (IL-7) and interleukin-15 (IL-15) for homeostatic survival (see, at least WO 2010/101870). The presence and activity of such cells are undesired in the context of suppressing immune responses.

Unlike Tregs, "effector Tcons" are not anergic and can proliferate in response to antigen-based T cell receptor activation (Lechler et al. (2001) Philos. Trans. R. Soc. Lond. Biol. Sci. 356:625-637). Effector Tcons can be CD4+ or CD8+ T cells. They recognize antigens associated with MHC class I or II molecules, respectively, generally express activation markers, such as CD25, CD44 or CD69, but generally do not express memory markers, such as CD45RO. Generally, increasing the number of Tregs, increasing Treg activity, and/or decreasing Treg cell death (*e.g.,* apoptosis) is useful for suppressing unwanted immune reactions associated with a range of immune disorders (*e.g.,* cGVHD). Tregs are also important in suppressing inflammation as well. In the context of ongoing inflammation, treatments can preferentially enhance Tregs without activating Tcons or other effectors that may worsen GVHD. Effective augmentation of Tregs *in vivo* is also directly relevant to other disorders of impaired peripheral tolerance (*e.g.,* autoimmune diseases like SLE, T1D, MS, psoriasis, RA, IBD, vasculitis), where Treg dysfunction is increasingly implicated (Grinberg-Bleyer et al. (2010) J. Exp. Med. 207:1871-1878; Buckner (2010) Nat. Rev. Immunol. 10:849-859; Humrich et al. (2010) Proc. Natl. Acad. Sci. U.S.A. 107:204-209; Carbone et al. (2014) Nat. Med. 20:69-74).

"Memory Tcons" are antigen-experienced T cells *(i.e.,* T cells that have previously been exposed to and responded to an antigen) representated by at least three distinct subpopulations of T cells. Memory Tcons can reproduce quickly and elicit a stronger immune response when re-exposed to the antigen. Memory Tcons subpopulationcs can be differentiated based on the differential expression of the chemokine receptor, CCR7, and L-selection (CD62L) (Sallusto et al. (2000) Curr. Top. Microbiol. Immunol. 251:167-171). For example, stem memory T cells (Tscm), like naive cells, are CD45RO-, CCR7+, CD45RA+, CD62L+ (L-selectin), CD27+, CD28+, and IL-7Rα+, but they also express large amounts of CD95, IL-2Rβ, CXCR3, and LFA-1, and show numerous functional attributes distinctive of memory cells (Gattinoni et al. (2011) Nat. Med. 17:1290-1297). Central memory cells (Tcm) express L-selectin and the CCR7 and secrete IL-2, but not IFNγ or IL-4. Effector memory cells (Tern) do not express L-selectin or CCR7, but produce effector cytokines like IFNγ and IL-4.

"Exhausted Tcons" are T cells that have progressively lost T-cell function. "Exhaustion" or "unresponsiveness" refers to a state of a cell where the cell does not perform its usual function or activity in response to normal input signals, and includes refractivity of immune cells to stimulation, such as stimulation via an activating receptor or a cytokine. Such a function or activity includes, but is not limited to, proliferation or cell division, entrance into the cell cycle, cytokine production, cytotoxicity, trafficking, phagocytotic activity, or any combination thereof. Normal input signals can include, but are not limited to, stimulation via a receptor (*e.g.,* T cell receptor, B cell receptor, co-stimulatory receptor, and the like).

Exhausted immune cells can have a reduction of at least 10%, 15%, 20%, 25%, 30%, 35%, 40%, 45%, 50%, 55%, 60%, 65%, 70%, 75%, 80%, 85%, 90%, 95%, 99% or more in cytotoxic activity, cytokine production, proliferation, trafficking, phagocytotic activity, or any combination thereof, relative to a corresponding control immune cell of the same type. In one embodiment according to the disclosure, a cell that is exhausted is a CD8+ T cell (*e.g.,* an effector CD8+ T cell that is antigen-specific). CD8 cells normally proliferate (*e.g.,* clonally expand) in response to T cell receptor and/or co-stimulatory receptor stimulation, as well as in response to cytokines such as IL-2. Thus, an exhausted CD8 T cell is one which does not proliferate and/or produce cytokines in response to normal input signals. It is well known that the exhaustion of effector functions can be delineated according to several stages, which eventually lead to terminal or full exhaustion and, ultimately, deletion (Yi et al. (2010) Immunol. 129:474-481; Wherry and Ahmed (2004) J. Virol. 78:5535-5545). In the first stage, functional T cells enter a "partial exhaustion I" phase characterized by the loss of a subset of effector functions, including loss of IL-2 production, reduced TNFα production, and reduced capacity for proliferation and/or *ex vivo* lysis ability. In the second stage, partially exhausted T cells enter a "partial exhaustion II" phase when both IL-2 and TNFα production ceases following antigenic stimulation and IFNγ production is reduced. "Full exhaustion" or "terminal exhaustion" occurs when CD8+ T cells lose all effector functions, including the lack of production of IL-2, TNFα, and IFNγ and loss of *ex vivo* lytic ability and proliferative potential, following antigenic stimulation. A fully exhausted CD8+ T cell is one which does not proliferate, does not lyse target cells (cytotoxicity), and/or does not produce appropriate cytokines, such as IL-2, TNFα, or IFNγ, in response to normal input signals. Such lack of effector functions can occur when the antigen load is high and/or CD4 help is low. This hierarchical loss of function is also associated with the expression of co-inhibitor immune receptors, such as PD-1, TIM-3, LAG-3, and the like (Day et al. (2006) Nature 443:350-4; Trautmann et al. (2006) Nat. Med. 12:1198-202; and Urbani et al. (2006) J. Virol. 80:1398-1403). Other molecular markers distinguish the hierarchical stages of immune cell exhaustion, such as high eomesodermin (EOMES) and low TBET expression as a marker of terminally exhausted T cells (Paley et al. (2012) Science 338:1220-1225). Additional markers of exhausted T cells, such as the reduction of Bcl-b and the increased production of BLIMP-1 (Pdrm1).

In addition, Tregs and/or Teffs can be obtained from a single source or a plurality of sources (*e.g.,* a single subject or a plurality of subjects). A plurality refers to at least two (*e.g.,* more than one). In still another embodiment according to the disclosure, the non-human mammal is a mouse. The animals from which cell types of interest are obtained may be adult, newborn (*e.g.,* less than 48 hours old), immature, or *in utero.* Cell types of interest may be primary cells, stem cells, established cancer cell lines, immortalized primary cells, and the like.

In certain embodiments according to the disclosure, the T cells of interest can be obtained from particular sources. For example, a mammalian animal model of an immune disorder can be used as the source of T cells of interest. In another example, the immune systems of host subjects can be engineered or otherwise elected to be immunological compatible with transplanted cells. For example, in one embodiment according to the disclosure, the subject may be "humanized" in order to be compatible with human cells. The term "immune-system humanized" refers to an animal, such as a mouse, comprising human HSC lineage cells and human acquired and innate immune cells, survive without being rejected from the host animal, thereby allowing human hematopoiesis and both acquired and innate immunity to be reconstituted in the host animal. Acquired immune cells include T cells and B cells. Innate immune cells include macrophages, granulocytes (basophils, eosinophils, and neutrophils), DCs, NK cells and mast cells. Representative, non-limiting examples include SCID-hu, Hu-PBL-SCID, Hu-SRC-SCID, NSG (NOD-SCID IL2r-gamma(null) lack an innate immune system, B cells, T cells, and cytokine signaling), NOG (NOD-SCID IL2r-gamma(truncated)), BRG (BALB/c-Rag2(null)II,2r-gamma(null)), and H2dRG (Stock-H2d-Rag2(null)IL2r-gamma(null)) mice (see, for example, Shultz et al. (2007) Nat. Rev. Immunol. 7:118; Pearson et al. (2008) Curr. Protocol. Immunol. 15:21; Brehm et al. (2010) Clin. Immunol. 135:84-98; McCune et al. (1988) Science 241:1632-1639, U.S. Pat. 7,960,175, and U.S. Pat. Publ. 2006/0161996), as well as related null mutants of immune-related genes like Rag1 (lack B and T cells), Rag2 (lack B and T cells), TCR alpha (lack T cells), perform (cD8+ T cells lack cytotoxic function), FoxP3 (lack functional CD4+ T regulatory cells), IL2rg, or Prfl, as well as mutants or knockouts of PD-1, PD-L1, Tim3, and/or 2B4, allow for efficient engraftment of human immune cells in and/or provide compartment-specific models of immunocompromised animals like mice (see, for example, PCT Publ. WO2013/062134). In addition, NSG-CD34+ (NOD-SCID IL2r-gamma(null) CD34+) humanized mice are useful for studying human gene and tumor activity in animal models like mice.

As used herein, "obtained" from a biological material source means any conventional method of harvesting or partitioning a source of biological material from a donor. For example, biological material may be obtained from a solid tumor, a blood sample, such as a peripheral or cord blood sample, or harvested from another body fluid, such as bone marrow or amniotic fluid. Methods for obtaining such samples are well-known to the ordinarily skilled artisan. In the present invention, the samples may be fresh (*i.e.*, obtained from a donor without freezing). Moreover, the samples may be further manipulated to remove extraneous or unwanted components prior to expansion. The samples may also be obtained from a preserved stock. For example, in the case of cell lines or fluids, such as peripheral or cord blood, the samples may be withdrawn from a cryogenically or otherwise preserved bank of such cell lines or fluid. Such samples may be obtained from any suitable donor.

Well-known immune cell characteristics can be used to purify, enrich, and/or isolate T cells of interest. "Enriched T cells" refer to a composition comprising a desired T cell population (*e.g.,* engineered T cells of the present invention) to other cells and/or T cells in a proportion where the composition has at least a 1:2, 1:1.9, 1:1.8, 1:1.7, 1:1.6, 1:1.5, 1:1.4, 1:1.3, 1:1.2, 1:1.1, 1:1, 1:0.9, 1:0.8, 1:0.7, 1:0.6, 1:0.5, 1:0.4, 1:0.3, 1:0.2, 1:0.1, or more, or any range in between or any value in between, ratio of desired T cells to other cells. Such ratios can be achieved by purifying a composition comprising T cells with various methodologies. For example, purification of Tregs can be performed using CD8+ and CD19+ co-depletion in combination with positive selection for CD25+ cells. Such enriched Tregs can further be defined in terms of cell markers and/or viability. For example, an enriched Tregs cell composition can have greater than 40%, 45%, 50%, 55%, 60%, 65%, 70%, 75%, 80%, 85%, 90%, 95%, 99%, or more, or any range in between or any value in between, total cell viability. It can comprise greater than 40%, 45%, 50%, 55%, 60%, 65%, 70%, 75%, 80%, 85%, 90%, 95%, 99%, or more, or any range in between or any value in between, CD4+CD25+ cells. It can comprise greater than 40%, 45%, 50%, 55%, 60%, 65%, 70%, 75%, 80%, 85%, 90%, 95%, 99%, or more, or any range in between or any value in between, FoxP3+ cells. Tregs can be administered in any suitable route as described herein, such as by infusion. Tregs can also be administered before, concurrently with, or after, other immunomodulatory agents. Such methodologies and metrics can be adapted to any T cell population of interest using well-known methods in the art.

In one embodiment according to the disclosure, fluorescence activated cell sorting (FACS), also referred to as flow cytometry, is used to sort and analyze the different cell populations. Cells having a cellular marker or other specific marker of interest are tagged with an antibody, or typically a mixture of antibodies, that bind the cellular markers. Each antibody directed to a different marker is conjugated to a detectable molecule, particularly a fluorescent dye that may be distinguished from other fluorescent dyes coupled to other antibodies. A stream of tagged or "stained" cells is passed through a light source that excites the fluorochrome and the emission spectrum from the cells detected to determine the presence of a particular labeled antibody. By concurrent detection of different fluorochromes, also referred to in the art as multicolor fluorescence cell sorting, cells displaying different sets of cell markers may be identified and isolated from other cells in the population. Other FACS parameters, including, by way of example and not limitation, side scatter (SSC), forward scatter (FSC), and vital dye staining (*e.g.,* with propidium iodide) allow selection of cells based on size and viability. FACS sorting and analysis of HSC and related lineage cells is well-known in the art and described in, for example, U.S. Pat. Nos. 5,137,809; 5,750,397; 5,840,580; 6,465,249; Manz et al. (202) Proc. Natl. Acad. Sci. U.S.A. 99:11872-11877; and Akashi et al. (200) Nature 404:193-197. General guidance on fluorescence activated cell sorting is described in, for example, Shapiro (2003) Practical Flow Cytometry, 4th Ed., Wiley-Liss (2003) and Ormerod (2000) Flow Cytometry: A Practical Approach, 3rd Ed., Oxford University Press.

Another method of isolating useful cell populations involves a solid or insoluble substrate to which is bound antibodies or ligands that interact with specific cell surface markers. In immunoadsorption techniques, cells are contacted with the substrate (*e.g.,* column of beads, flasks, magnetic particles, etc.) containing the antibodies and any unbound cells removed. Immunoadsorption techniques may be scaled up to deal directly with the large numbers of cells in a clinical harvest. Suitable substrates include, by way of example and not limitation, plastic, cellulose, dextran, polyacrylamide, agarose, and others known in the art (*e.g.,* Pharmacia Sepharose 6 MB macrobeads). When a solid substrate comprising magnetic or paramagnetic beads is used, cells bound to the beads may be readily isolated by a magnetic separator (*e.g.,* Kato and Radbruch (1993) Cytometry 14:384-92). Affinity chromatographic cell separations typically involve passing a suspension of cells over a support bearing a selective ligand immobilized to its surface. The ligand interacts with its specific target molecule on the cell and is captured on the matrix. The bound cell is released by the addition of an elution agent to the running buffer of the column and the free cell is washed through the column and harvested as a homogeneous population. As apparent to the skilled artisan, adsorption techniques are not limited to those employing specific antibodies, and may use nonspecific adsorption. For example, adsorption to silica is a simple procedure for removing phagocytes from cell preparations.

FACS and most batch-wise immunoadsorption techniques may be adapted to both positive and negative selection procedures (see, *e.g.,* U.S. Pat. No. 5,877,299). In positive selection, the desired cells are labeled with antibodies and removed away from the remaining unlabeled/unwanted cells. In negative selection, the unwanted cells are labeled and removed. Another type of negative selection that may be employed is use of antibody/complement treatment or immunotoxins to remove unwanted cells.

It is to be understood that the purification or isolation of cells also includes combinations of the methods described above. A typical combination may comprise an initial procedure that is effective in removing the bulk of unwanted cells and cellular material, for example leukapharesis. A second step may include isolation of cells expressing a marker common to one or more of the progenitor cell populations by immunoadsorption on antibodies bound to a substrate. An additional step providing higher resolution of different cell types, such as FACS sorting with antibodies to a set of specific cellular markers, may be used to obtain substantially pure populations of the desired cells.

Cells of interest can be genetically modified according to the present invention, wherein the genome of an organism or cell can be engineered to modify the expression and/or activity of a biomarker of the present invention. "Engineered T cells" according to the present invention, for example, are T cells of interest genetically modified to comprise a genomic region that 1) regulates the gene expression of at least one gene and 2) is selectively chromatin accessible within the genome of the T cell of interest from a mammal (*e.g.,* an exhausted CD8+ T cell from the mammal) and wherein the genomic region is genetically modified and the genetic modification modulates the expression of the at least one gene. For example, CD8+ T cells can be genetically modified using recombinant techniques in order to modulate the expression and/or activity of PD-1 expressed at high levels by exhausted CD8+ T cells, by modifying (*e.g.,* deleting) the exhausted CD8+ T cell-selective PD-1 genomic gene expression enhancer that is selectively chromatin accessible in the exhausted CD8+ T cell, described in the Examples, or an ortholog thereof in another mammal.

In one embodiment according to the disclosure, the genetic modification is a deletion of all or a portion of the genomic gene expression regulatory region. The deletion can be a *per se* deletion or an effective deletion by inserting a sequence not present in the genomic gene expression regulatory region prior to genetic modification. As described in the Examples, transcription factors are known to bind the chromatin accessible genomic gene expression regulatory region and actual or constructive deletion of the region or a portion thereof disrupts the ability of the transcription factors to bind and mediate gene expression regulation of a gene of interest. Deletion of any portion of the region is believed to modulate gene expression regulation of a gene of interest. As described below, assays for determining the amount of modulated gene expression caused by the genetic modification are routine in the art. Deletion of an enhancer genomic region will reduce transcription of the gene of interest, whereas deletion of a suppressor genomic region will increase transcription of the gene of interest. Similarly, genomic modifications that stabilize the enhancer genomic region, such as strengthening points of contact with transcription factors by optimizing the sequence of the transcription factor binding motif, will reduce transcription of the gene of interest for enhancer genomic regions and decrease transcription of the gene of interest in suppressor genomic regions.

Genome editing methods are well-known in the art. For example, targeted or untargeted gene knockout methods can be used, such as to recombinantly engineer subject Tregs *ex vivo* prior to infusion into the subject. For example, the target DNA in the genome can be manipulated by deletion, insertion, and/or mutation using retroviral insertion, artificial chromosome techniques, gene insertion, random insertion with tissue specific promoters, gene targeting, transposable elements and/or any other method for introducing foreign DNA or producing modified DNA/modified nuclear DNA.

Other modification techniques include deleting DNA sequences from a genome and/or altering nuclear DNA sequences. Nuclear DNA sequences, for example, may be altered by site-directed mutagenesis using homologous recombination. Such methods generally use host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. Vector DNA can be introduced into prokaryotic or eukaryotic cells via conventional transformation or transfection techniques.

As used herein, the terms "transformation" and "transfection" are intended to refer to a variety of art-recognized techniques for introducing foreign nucleic acid into a host cell, including calcium phosphate or calcium chloride co-precipitation, DEAE-dextran-mediated transfection, lipofection, or electroporation. Suitable methods for transforming or transfecting host cells can be found in Sambrook, *et al.* (*supra*)*,* and other laboratory manuals. For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g.,* for resistance to antibiotics) is generally introduced into the host cells along with the gene of interest. Preferred selectable markers include those which confer resistance to drugs, such as G418, hygromycin and methotrexate. Cells stably transfected with the introduced nucleic acid can be identified by drug selection (*e.g.,* cells that have incorporated the selectable marker gene will survive, while the other cells die).

Similarly, the CRISPR-Cas system can be used for precise editing of genomic nucleic acids (*e.g.,* for creating non-functional or null mutations). In such embodiments according to the disclosure, the CRISPR guide RNA and/or the Cas enzyme may be expressed. For example, a vector containing only the guide RNA can be administered to an animal or cells transgenic for the Cas9 enzyme. Similar strategies may be used (*e.g.,* designer zinc finger, transcription activator-like effectors (TALEs) or homing meganucleases). Such systems are well-known in the art (see, for example, U.S. Pat. No. 8,697,359; Sander and Joung (2014) Nat. Biotech. 32:347-355; Hale et al. (2009) Cell 139:945-956; Karginov and Hannon (2010) Mol. Cell 37:7; U.S. Pat. Publ. 2014/0087426 and 2012/0178169; Boch et al. (2011) Nat. Biotech. 29:135-136; Boch et al. (2009) Science 326:1509-1512; Moscou and Bogdanove (2009) Science 326:1501; Weber et al. (2011) PLoS One 6:e19722; Li et al. (2011) Nucl. Acids Res. 39:6315-6325; Zhang et al. (2011) Nat. Biotech. 29:149-153; Miller et al. (2011) Nat. Biotech. 29:143-148; Lin et al. (2014) Nucl. Acids Res. 42:e47). Such genetic strategies can use constitutive expression systems or inducible expression systems according to well-known methods in the art.

The obtained populations of cells may be used directly or frozen for use at a later date. A variety of mediums and protocols for cryopreservation are known in the art. Generally, the freezing medium will comprise DMSO from about 5-10%, 10-90% serum albumin, and 50-90% culture medium. Other additives useful for preserving cells include, by way of example and not limitation, disaccharides such as trehalose (Scheinkoniget al. (2004) Bone Marrow Transplant. 34:531-536), or a plasma volume expander, such as hetastarch (*i.e*., hydroxyethyl starch). In some embodiments according to the disclosure, isotonic buffer solutions, such as phosphate-buffered saline, may be used. An exemplary cryopreservative composition has cell-culture medium with 4% HSA, 7.5% dimethyl sulfoxide (DMSO), and 2% hetastarch. Other compositions and methods for cryopreservation are well-known and described in the art (see, *e.g.,* Broxmeyer et al. (2003) Proc. Natl. Acad. Sci. U.S.A. 100:645-650). Cells are preserved at a final temperature of less than about -135°C.

### IV. Analyzing gene expression and function of engineered T cells

Modulation of gene expression of at least one gene of interest, as well as T cell function, can be determined according to well-known methods in the art and as exemplified in the Examples. For example, T cell activity, proliferation, apoptosis, cytokine production repertoire, cell surface marker expression, and the like can be analyzed. Moreover, phenotypic analyses of lymphocyte subsets, functional assays of immunomodulation leading to reduced immune responses, plasma cytokines, and the like can be analyzed as described further herein. In particular, methods for determining the results of the methods described herein, such as modulation of immune responses, metastasis, disease remission, disease relapse, tumor recurrence, death, autoimmunity, allergy (*e.g.,* asthma, atopic dermatitis, allergic conjunctivitis, pollen allergy, food allergy, etc.), vaccination response, immune tolerance, immune exhaustion, immunological memory, immunological epitope responses, cytokine responses, relative representation of cells, genetic perturbations, and/or other immunologic effects are well-known in the art and as described herein. For example, determination of target nucleic acid gene expression and/or sequences of interest can be performed using variety of sequencing methods known in the art. In preferred embodiments according to the disclosure, a particular genetic perturbation is characterized by a measure of a nucleic acid or product thereof (*e.g.,* mRNA). Marker expression may be monitored in a variety of ways, including by detecting mRNA levels, protein levels, or protein activity, any of which may be measured using standard techniques (see, *e.g.,* Ausubel et al., ed., Current Protocols in Molecular Biology, John Wiley & Sons, New York 1987-1999). Detection may involve quantification of the level of gene expression (*e.g.,* genomic DNA, cDNA, mRNA, protein, or enzyme activity), or, alternatively, may be a qualitative assessment of the level of gene expression, in particular in comparison with a control level. The type of level being detected will be clear from the context. Various amplification and detection methods may also be used. For example, it is within the scope of the present invention to reverse transcribe mRNA into cDNA followed by polymerase chain reaction (RT-PCR); or, to use a single enzyme for both steps as described in U.S. Pat. No. 5,322,770, or reverse transcribe mRNA into cDNA followed by symmetric gap ligase chain reaction (RT-AGLCR), real time PCR, NASBA, ligase chain reaction (Barany, 1991, Proc. Natl. Acad. Sci. USA, 88:189-193), self-sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi et al., 1988, Bio/Technology 6:1197), rolling circle replication (Lizardi et al., U.S. Patent No. 5,854,033), target-mediated amplification, self-sustained sequence replication (SSR), transcription amplification, and the like. Many techniques are known in the state of the art for determining absolute and relative levels of gene expression, commonly used techniques suitable for use in the present invention include in situ hybridization, microarray, chip array, serial analysis of gene expression (SAGE), Northern analysis, RNase protection assays (RPA), microarrays and PCR-based techniques, such as quantitative PCR and differential display PCR. For example, Northern blotting involves running a preparation of RNA on a denaturing agarose gel, and transferring it to a suitable support, such as activated cellulose, nitrocellulose or glass or nylon membranes. Radiolabeled cDNA or RNA is then hybridized to the preparation, washed and analyzed by autoradiography.

In certain embodiments according to the disclosure, nucleic acid detection can be accomplished using methods including, but not limited to, sequencing by hybridization (SBH), sequencing by ligation (SBL), quantitative incremental fluorescent nucleotide addition sequencing (QIFNAS), stepwise ligation and cleavage, fluorescence resonance energy transfer (FRET), molecular beacons, TaqMan^{®} reporter probe digestion, pyrosequencing, fluorescent *in situ* sequencing (FISSEQ), FISSEQ beads (U.S. Pat. No. 7,425,431), wobble sequencing (PCT/US05/27695), multiplex sequencing (U.S. Ser. No. 12/027,039, filed Feb. 6, 2008; Porreca et al. (2007) Nat. Methods 4:931), polymerized colony (POLONY) sequencing (U.S. Pat. Nos. 6,432,360, 6,485,944 and 6,511,803, and PCT/US05/06425); nanogrid rolling circle sequencing (ROLONY) (U.S. Ser. No. 12/120,541, filed May 14, 2008), allele-specific oligo ligation assays (*e.g.,* oligoligation assay (OLA), single template molecule OLA using a ligated linear probe and a rolling circle amplification (RCA) readout, ligated padlock probes, and/or single template molecule OLA using a ligated circular padlock probe and a rolling circle amplification (RCA) readout) and the like. High-throughput sequencing methods, *e.g.,* on cyclic array sequencing using platforms such as Roche 454, Illumina Solexa or MiSeq or HiSeq, AB-SOLiD, Helicos, Polonator platforms and the like, can also be utilized. High-throughput sequencing methods are described in U.S. Ser. No. 61/162,913, filed Mar. 24, 2009. A variety of light-based sequencing technologies are known in the art (Landegren et al. (1998) Genome Res. 8:769-76; Kwok (2000) Pharmocogenom. 1:95-100; and Shi (2001) Clin. Chem. 47:164-172) (see, for example, U.S. Pat. Publ. Nos. 2013/0274117, 2013/0137587, and 2011/0039304).

Similarly, polypeptides and/or cells of interest can be distinguished according to many well-known methods in the art including, but not limited to, flow cytometry, fluorescence activated cell sorting (FACS), fluorescence microscopy, detectable cell barcode technology (U.S. Pat. Publ. 2011/0263457), immunodiffusion, immunoelectrophoresis, radioimmunoassay (RIA), enzyme-linked immunosorbent assays (ELISAs), immunofluorescent assays, Western blotting, binder-ligand assays, immunohistochemical techniques, agglutination, complement assays, high performance liquid chromatography (HPLC), thin layer chromatography (TLC), hyperdiffusion chromatography, and the like (*e.g.,* "Basic and Clinical Immunology," Sites and Terr, eds., Appleton and Lange, Norwalk, Conn. Pp. 217-262, 1991,). Preferred are binder-ligand immunoassay methods including reacting antibodies with an epitope or epitopes and competitively displacing a labeled polypeptide or derivative thereof.

In addition, T cell function of the engineered T cells can be assessed according to well-known methods in the art as described further in the Examples. For example, engineered T cells can be assessed for "reduced exhaustion" or "reduced unresponsiveness," which refers to a given treatment or set of conditions that leads to increased T cell activity, responsiveness, and/or ability or receptiveness, with regards to activation. T cell activity can be measured by contacting T cells with recall antigen, anti-CD3 in the absence of costimulation, and/or ionomycin. Also, proliferation of T cells can be measured in the presence of a relevant antigen assayed, e.g. by a ³H-thymidine incorporation assay or cell number. Markers of T cell activation after exposure to the relevant antigen can also be assayed, e.g. flow cytometry analysis of cell surface markers indicative of T cell activation (*e.g.,* CD69, CD30, CD25, and HLA-DR) and/or T cell exhaustion. In some embodiments according to the disclosure, the assays can be *in vivo* assays, such as through challenging immune cells with antigen *in vivo.* For example, animal models expressing homogeneous populations of T cells from TCR transgenic and other transgenic mice can be transferred into hosts that constitutively express an antigen recognized by the transferred T cells, e.g., the H-Y antigen TCR transgenic; pigeon cytochrome C antigen TCR transgenic; or hemagglutinin (HA) TCR transgenic. In such models, T cells expressing the TCR specific for the antigen constitutively or inducibly expressed by the recipient mice typically undergo an immediate expansion and proliferative phase, followed by a period of unresponsiveness, which is reversed when the antigen is removed and/or antigen expression is inhibited. Accordingly, if the T cells proliferate or expand, show cytokine activity, etc. significantly more in an assay (*e.g.,* with or without additional treatment of immunomodulatory agents) than control T cells, then T cell exhaustion is reduced. Such measurements of proliferation can occur *in vivo* using T cells labeled with BrDU, CFSE or another intravital dye that allows tracking of proliferation prior to transferring to a recipient animal expressing the antigen, or cytokine reporter T cells, or using *ex vivo* methods to analyze cellular proliferation and/or cytokine production, such as thymidine proliferation assays, ELISA, cytokine bead assays, and the like. Moreover, reduction of immune cell exhaustion can be assessed by examination of tumor infiltrating lymphocytes or T lymphocytes within lymph nodes that drain from an established tumor. Such T cells exhibit features of exhaustion through expression of cell surface molecules, such as immunoinhibitory receptors described above, for example, and decreased secretion of cytokines, such as those described above. Accordingly, if increased quantities and/or activities of T cells are observed with, for example, 1) antigen specificity for tumor associated antigens (*e.g.,* as determined by major histocompatibility complex class I or class II tetramers which contain tumor associated peptides) and/or 2) that are capable of secreting high levels of appropriate cytokines and cytolytic effector molecules such as granzyme-B, then T cell exhaustion has been reduced.

### V. Therapeutic methods of modulating T cell exhaustion and treating immune disorders in a subject

It is demonstrated herein that genetically engineering T cells to comprise a genomic region that 1) regulates the gene expression of at least one gene and 2) is present within the genome of an exhausted CD8+ T cell from the mammal, wherein the genomic region is genetically modified and the genetic modification modulates the expression of the at least one gene. Modulating such T cell exhaustion state-specific gene expression can modulate (*e.g.,* prevent or reverse) T cell exhaustion. Modulating T cell exhaustion can be used to treat immune disorders in the subject. The genetically engineered T cells can be used either alone or in combination with other T cell exhaustion and/or immune disorder modulatory agents described herein, including antibodies, small molecules, peptides, peptidomimetics, natural ligands, small non-coding RNAs such as miRNAs, pre-miRNAs, pri-miRNAs, miRNA*, anti-miRNA, a miRNA binding site, and the like, RNA interference, antisense, nucleic acid aptamers, ribozymes, and the like.

### a. Subjects

In one embodiment according to the disclosure, the subject is a mammal (*e.g.,* mouse, rat, primate, non-human mammal, and domestic animals, such as dog, cat, cow, and horse, etc.), and is preferably a human. Adult subjects, as well as pediatric subjects, are contemplated. Pediatric subjects can be treated as described herein, as well as using doses of therapeutic agents up to those used for adult subjects. In some embodiments according to the disclosure, the subject is a mammal presenting an animal model of a disorder of interest, such as a mouse model of an immune disorder.

In another embodiment of the methods of the present disclosure, the subject has not undergone treatment, such as anti-immune disorder therapy. In still another embodiment according to the disclosure, the subject has undergone such treatment. In yet another embodiment according to the disclosure, the subject is immunocompetent or immune-incompetent.

"Immunocompetent" subjects are those subjects comprising immune cells and immune function required to establish a normal or desired immune response following exposure to an antigen. In one embodiment according to the disclosure, the immunocompetent subject is a wild type subject having a completely intact immune system. In another embodiment, the immunocompetent subject is a wild type subject having a completely intact, yet maturing, immune system (*e.g.,* a juvenile subject). In still another embodiment according to the disclosure, the immunocompetent subject has an intact immune system required for mediating immune responses using a specific arm of the immune system (*e.g.,* acquired vs. innate and/or humoral vs. cytotoxic).

"Immuno-incompetent" subjects are those subjects lacking one or more immune cell types or lacking an immune function thereof to establish a normal or desired level of at least one immune response following exposure to an antigen. Immuno-incompetent subjects are more susceptible to opportunistic infections, for example viral, fungal, protozoan, or bacterial infections, prion diseases, and certain neoplasms. "Immunodeficient" subjects are subjects in which no native host immune response may be mounted, such as is the case with severe combined immunodeficiency (SCID) mice. "Immunocompromised" subjects have at least one substantially reduced immunological function relative to immunocompetent subjects. In either case, reduction in or absence of immunological function and/or cell types can arise from many different and well-known manners. For example, hematopoietic stem cells (HSCs) that give rise to all immune cells are any project thereof can be negatively affected in development, function, differentiation, survival, and the like.

Immuno-incompetent subjects can be generated in many different ways well-known in the art. They can result from modulating the function and/or number of various parameters in numerous combinations. For example, immune cell populations can be targeted for modulation that are resting, mitotic, terminally differentiated, post-mitotic, unactivated, activated, and the like, in order to effect a desired immune-incompetency. "Resting" cells refer to a non-cycling cell in a non-replicative state. Although resting cells may have the ability to replicate and divide upon activation, they are quiescent since they are non-cycling. Thus, "resting" cells are not simply manipulated immune cells that have been stimulated to divide and then engineered to revert to a quiescent, non-dividing phase. Resting cells can be "naive," which means that they are immune cells that have differentiated in bone marrow, successfully undergone positive and negative selection in the thymus, and are mature, but have not been activated and are not memory cells. Naive T cells are commonly characterized by the surface expression of L-selectin (CD62L); the absence of the activation markers, CD25, CD44, or CD69; and the absence of memory CD45RO isoform. They also express functional IL-7 receptors, consisting of subunits IL-7 receptor-α, CD127, and common-γ chain, CD132. In the naive state, T cells are thought to be quiescent and non-dividing, requiring the common-gamma chain cytokines IL-7 and IL-15 for homeostatic survival mechanisms. By contrast, activated T cells express or upregulate expression of surface markers, CD25, CD44, CD62L^{low}, and CD69 and may further differentiate into memory T cells. Naive B cells have not been exposed to antigen since they would either become a memory B cell or a plasma cell that secretes antibodies. In one embodiment according to the disclosure, a resting cell becomes "activated" when it is triggered to enter into a state of reproduction or doubling and may include a cell entering the cell cycle, cell division, or mitosis. In another embodiment according to the disclosure, a resting cell may also become "activated" when it encounters an external signal, such as an antigen or a cytokine, that initiates the activity of terminally differentiated, mature immunological cells to generate an immune response (*e.g.,* T cell or B cell function).

In one embodiment according to the disclosure, such subjects are obtained through defined or undefined genetic modifications. Representative, non-limiting examples of such genetic modifications are described above regarding immunocompromised animals. Moreover, the term "severe combined immune deficiency (SCID)" refers to a condition characterized by absence of T cells and lack of B cell function. Common forms of SCID caused by genetic modification include: X-linked SCID which is characterized by gamma chain gene mutations in the IL2RG gene and the lymphocyte phenotype T(-) B(+) NK(-); and autosomal recessive SCID characterized by Jak3 gene mutations and the lymphocyte phenotype T(-) B(+) NK(-), ADA gene mutations and the lymphocyte phenotype T(-) B(-) NK(-), IL-7R alpha-chain mutations and the lymphocyte phenotype T(-) B(+) NK(+), CD3 delta or epsilon mutations and the lymphocyte phenotype T(-) B(+) NK(+), RAG1/RAG2 mutations and the lymphocyte phenotype T(-) B(-) NK(+), Artemis gene mutations and the lymphocyte phenotype T(-) B(-) NK(+), CD45 gene mutations and the lymphocyte phenotype T(-) B(+) NK(+). In another example, genetically modified subjects that are immunodeficient have the severe combined immunodeficiency mutation, Prkdc^{scid}, commonly referred to as the *scid* mutation (see, for example, Bosma et al. (1989) Immunogenet. 29:54-56). Mice homozygous for the scid mutation are characterized by an absence of functional T cells and B cells, lymphopenia, hypoglobulinemia and a normal hematopoietic microenvironment. The *scid* mutation may be detected, for example, by detection of markers for the *scid* mutation using well-known methods.

In another embodiment according to the disclosure, such subjects are obtained through non-genetic ablation of immune cell function or numbers. Other agents can be used to ablate immune cell function or numbers. For example, they may be conditioned with sub-lethal irradiation or lethal irradiation with high frequency electromagnetic radiation. The radiation can be ionizing radiation. Radiation therapy can also be gamma rays, X-rays, or proton beams. Examples of radiation therapy include, but are not limited to, external-beam radiation therapy, interstitial implantation of radioisotopes (I-125, palladium, iridium), radioisotopes such as strontium-89, thoracic radiation therapy, intraperitoneal P-32 radiation therapy, and/or total abdominal and pelvic radiation therapy. For a general overview of radiation therapy, see Hellman, Chapter 16: Principles of Cancer Management: Radiation Therapy, 6th edition, 2001, DeVita et al., eds., J. B. Lippencott Company, Philadelphia. The radiation therapy can be administered as external beam radiation or teletherapy wherein the radiation is directed from a remote source. The radiation treatment can also be administered as internal therapy or brachytherapy wherein a radioactive source is placed inside the body close to cancer cells or a tumor mass. Also encompassed is the use of photodynamic therapy comprising the administration of photosensitizers, such as hematoporphyrin and its derivatives, Vertoporfin (BPD-MA), phthalocyanine, photosensitizer Pc4, demethoxy-hypocrellin A; and 2BA-2-DMHA.

Similarly, non-genetic ablation of immune cell function or numbers can be effected through treatment with a radiomimetic drug such as busulfan or nitrogen mustard. Other immune cell cytoreductive drugs, include, among others, cyclophosphamide, ifosfamide, etoposide, cytosine arabinoside, carboplatin, and other chemotherapeutic agents (Montillo et al. (2004) Leukemia 18:57-62; Dasgupta et al. (1996) J. Infusional Chemother. 6:12; and Wright et al. (2001) Blood 97:2278-2285). Other classes of cytoreductive drugs include, but are not limited to, those selected from among the following groups of compounds: platinum compounds, cytotoxic antibiotics, antimetabolites, anti-mitotic agents, alkylating agents, arsenic compounds, DNA topoisomerase inhibitors, taxanes, nucleoside analogues, plant alkaloids, and toxins; and synthetic derivatives thereof. Exemplary compounds include, but are not limited to, alkylating agents: cisplatin, treosulfan, and trofosfamide; plant alkaloids: vinblastine, paclitaxel, docetaxol; DNA topoisomerase inhibitors: teniposide, crisnatol, and mitomycin; anti-folates: methotrexate, mycophenolic acid, and hydroxyurea; pyrimidine analogs: 5-fluorouracil, doxifluridine, and cytosine arabinoside; purine analogs: mercaptopurine and thioguanine; DNA antimetabolites: 2'-deoxy-5-fluorouridine, aphidicolin glycinate, and pyrazoloimidazole; and antimitotic agents: halichondrin, colchicine, and rhizoxin. Compositions comprising one or more chemotherapeutic agents (*e.g.,* FLAG, CHOP) may also be used. FLAG comprises fludarabine, cytosine arabinoside (Ara-C) and G-CSF. CHOP comprises cyclophosphamide, vincristine, doxorubicin, and prednisone. In another embodiment according to the disclosure, PARP (*e.g.,* PARP-1 and/or PARP-2) inhibitors are used and such inhibitors are well-known in the art (*e.g.*, Olaparib, ABT-888, BSI-201, BGP-15 (N-Gene Research Laboratories, Inc.); INO-1001 (Inotek Pharmaceuticals Inc.); PJ34 (Soriano *et al.,* 2001; Pacher *et al.,* 2002b); 3-aminobenzamide (Trevigen); 4-amino-1,8-naphthalimide; (Trevigen); 6(5H)-phenanthridinone (Trevigen); benzamide (U.S. Pat. Re. 36,397); and NU1025 (Bowman *et al*.). The mechanism of action is generally related to the ability of PARP inhibitors to bind PARP and decrease its activity. PARP catalyzes the conversion of beta-nicotinamide adenine dinucleotide (NAD+) into nicotinamide and poly-ADP-ribose (PAR). Both poly (ADP-ribose) and PARP have been linked to regulation of transcription, cell proliferation, genomic stability, and carcinogenesis (Bouchard V. J. et.al. Experimental Hematology, Volume 31, Number 6, June 2003, pp. 446-454(9); Herceg Z.; Wang Z.-Q. Mutation Research/Fundamental and Molecular Mechanisms of Mutagenesis, Volume 477, Number 1, 2 Jun. 2001, pp. 97-110(14)). Poly(ADP-ribose) polymerase 1 (PARP1) is a key molecule in the repair of DNA single-strand breaks (SSBs) (de Murcia J. et al. 1997. Proc Natl Acad Sci USA 94:7303-7307; Schreiber V, Dantzer F, Ame J C, de Murcia G (2006) Nat Rev Mol Cell Biol 7:517-528; Wang Z Q, et al. (1997) Genes Dev 11:2347-2358). Knockout of SSB repair by inhibition of PARP1 function induces DNA double-strand breaks (DSBs) that can trigger synthetic lethality in cancer cells with defective homology-directed DSB repair (Bryant H E, et al. (2005) Nature 434:913-917; Farmer H, et al. (2005) Nature 434:917-921).

Non-genetic ablation of immune cell function or numbers can be effected through treatment with agents, such as antibodies, to deplete immune system-mediating cell populations, or treatment with agents that preferentially deplete immune system-mediating cell populations (see, for example, Hayakawa et al. (2009) Stem Cells 27:175-182). For example, anti-CD4 and anti-CD8 antibodies can be used to neutralize and/or deplete CD4+ T cells and CD8+ T cells, respectively. Similarly, anti-CD3 antibodies can be used to deplete all T cells, anti-B220 and/or anti-CD19 antibodies can be used to deplete all B cells, anti-CD11b antibodies can be used to deplete macrophages, anti-Ly-6G (Gr-1) antibodies can be used to deplete monocytes and granulocytes, and anti-NK1.1 antibodies can be used to deplete Natural Killer (NK) cells.

Assays for confirming immune-incompetence of one or more immune cell types or functions are also well-known in the art. Determining the differentiation potential of cells, and thus the presence or absence of immune cell populations, is typically conducted by exposing the cells to conditions that permit development into various terminally differentiated cells. These conditions generally comprise a mixture of cytokines and growth factors in a culture medium permissive for development of the myeloid or lymphoid lineage. Colony forming culture assays rely on culturing the cells *in vitro* via limiting dilution and assessing the types of cells that arise from their continued development. A common assay of this type is based on methylcellulose medium supplemented with cytokines (*e.g.,* MethoCult, Stem Cell Technologies, Vancouver, Canada and Kennedy et al. (1997) Nature 386:488-493). Cytokine and growth factor formulations permissive for differentiation in the hematopoietic pathway are described in Manz et al. (2002) Proc. Natl. Acad. Sci. U.S.A. 99:11872-11877; U.S. Pat. No. 6,465,249; and Akashi et al., Nature 404:193-197). Cytokines include SCF, FLT-3 ligand, GM-CSF, IL-3, TPO, and EPO. Another *in vitro* assay is long-term culture initiating cell (LTC-IC) assay, which typically uses stromal cells to support hematopoiesis (see, e.g., Ploemache et al. (1989) Blood 74:2755-2763 and Sutherland et al. (1995) Proc. Natl. Acad. Sci. U.S.A. 87:3745).

Another type of assay suitable for determining the immune-incompetence state of a subject relies upon *in vivo* administration of cells into a host animal and assessment of the repopulation of the hematopoietic system. The recipient is immunocompromised or immunodeficient to limit rejection and permits acceptance of allogeneic or xenogeneic cell transplants. A useful animal system of this kind is the NOD/SCID (Pflumio et al. (1996) Blood 88:3731; Szilvassym et al. (2002) "Hematopoietic Stem Cell Protocol" in Methods in Molecular Medicine, Humana Press; Greiner et al. (1998) Stem Cells 16:166-177; Piacibello et al. (1999) Blood 93:3736-3749) or Rag2 deficient mouse (Shinkai et al. (1992) Cell 68:855-867). Cells originating from the infused cells are assessed by recovering cells from the bone marrow, spleen, or blood of the host animal and determining presence of cells displaying specific cellular markers (*i.e.*, marker phenotyping), typically by FACS analysis. Detection of markers specific to the transplanted cells permits distinguishing between endogenous and transplanted cells. For example, antibodies specific to human forms of the cell markers (*e.g.,* HLA antigens) identify human cells when they are transplanted into suitable immunodeficient mouse.

The methods of the present invention can be used to prevent or reverse T cell exhaustion, treat immune disorders, and/or determine the responsiveness to same of administration of genetically engineered T cells described herein. The functions of activated immune cells can be inhibited by down-regulating immune cell responses, by inducing specific anergy in immune cells, or both.

For example, the methods of the present invention can be used to induce tolerance against specific antigens by co-administering an antigen with the therapeutic compositions of such methods. Tolerance can be induced to specific proteins. In one embodiment according to the disclosure, immune responses to allergens (*e.g.,* food allergens), or to foreign proteins to which an immune response is undesirable, can be inhibited. For example, patients that receive Factor VIII frequently generate antibodies against this clotting factor. Co-administration of recombinant factor VIII (or by physically linked to Factor VIII, *e.g.,* by cross-linking) in the methods of the present invention can result in downmodulation of immune responses. In similar manners, reduced clonal deletion and/or increased exhaustion (*e.g.,* T cell exhaustion) can be induced.

Downregulating immune responses is useful for treating a number of other "immune disorders" according to the present invention including, without limitation, situations of tissue, skin and other solid organ transplantation (*e.g.,* kidney, liver, heart, and vascularized composite allotransplantation transplants), in hematopoietic stem cell transplantation rejection (*e.g.,* graft-versus-host disease (GVHD)), in autoimmune diseases such as systemic lupus erythematosus, multiple sclerosis, allergy, a transplant, hypersensitivity response, in a disorder requiring increased CD4+ T cell production or function, in a disorder requiring improved vaccination efficiency, and in a disorder requiring increased regulatory T cell production or function. For example, blockage of immune cell function results in reduced tissue destruction in tissue transplantation. Typically, in tissue transplants, rejection of the transplant is initiated through its recognition as foreign by immune cells, followed by an immune reaction that destroys the transplant. The administration of an agent described herein prior to or at the time of transplantation can promote the generation of an inhibitory signal. Moreover, inhibition may also be sufficient to anergize the immune cells, thereby inducing tolerance in a subject. Induction of long-term tolerance avoids the necessity of repeated administration of these blocking reagents.

Downmodulation of immune responses are also useful in treating autoimmune disease, such as type 1 diabetes (T1D) and multiple sclerosis. Many autoimmune disorders are the result of inappropriate activation of immune cells that are reactive against self-tissue and which promote the production of cytokines and autoantibodies involved in the pathology of the diseases. Preventing the activation of autoreactive immune cells may reduce or eliminate disease symptoms. Administration of agents described herein are useful for preventing the generating of autoantibodies or cytokines which may be involved in the disease process. Additionally, the methods of the present invention can induce antigen-specific tolerance of autoreactive immune cells, which could lead to long-term relief from the disease. The efficacy of reagents in preventing or alleviating autoimmune disorders can be determined using a number of well-characterized animal models of human autoimmune diseases. Examples include murine experimental autoimmune encephalitis, systemic lupus erythematosus in MRL/*lpr*/*lpr* mice or NZB hybrid mice, murine autoimmune collagen arthritis, diabetes mellitus in NOD mice and BB rats, and murine experimental myasthenia gravis (see, *e.g.,* Paul ed., Fundamental Immunology, Raven Press, New York, Third Edition 1993, chapter 30).

Inhibition of immune cell activation is also useful therapeutically in the treatment of allergy and allergic reactions, *e.g.,* by inhibiting IgE production. Allergic reactions can be systemic or local in nature, depending on the route of entry of the allergen and the pattern of deposition of IgE on mast cells or basophils. Thus, inhibition of immune cell mediated allergic responses (*e.g.,* to food) locally or systemically according to the methods of the present invention. In one embodiment according to the disclosure, the allergy is allergic asthma.

Inhibition of immune cell activation may also be important therapeutically in parasitic and viral infections of immune cells. For example, in the acquired immune deficiency syndrome (AIDS), viral replication is stimulated by immune cell activation. Modulation of these interactions may result in inhibition of viral replication and thereby ameliorate the course of AIDS. Modulation of these interactions may also be useful in promoting the maintenance of pregnancy. Females at risk for spontaneous abortion (*e.g.,* those who have previously had a spontaneous abortion or those who have had difficulty conceiving) because of immunologic rejection of the embryo or fetus can be treated with agents that modulate these interactions.

Downregulation of an immune response according to the methods of the present invention may also be useful in treating an autoimmune attack of autologous tissues. It is therefore within the scope of the invention to modulate conditions exacerbated by autoimmune attack, such as autoimmune disorders, as well as conditions such as heart disease, myocardial infarction, and atherosclerosis.

In a preferred embodiment according to the disclosure, the immune disorder is graft-versus-host-disease *(e.g.,* chronic GVHD). For many patients with hematologic malignancies, allogeneic hematopoietic stem cell transplant (HSCT) offers the only opportunity for cure. Unfortunately, significant obstacles remain, most notably disease recurrence and GVHD. Over 40% of patients undergoing HSCT relapse while more than 50% will develop cGVHD, a debilitating condition with multi-system immune manifestations associated with a considerable morbidity and mortality (Kahl et al. (2007) Blood 110:2744-2748; Perez-Simon et al. (2008) Biol. Blood Marrow Transplant. 14:1163-1171). Although the incidence in the pediatric population is lower, cGVHD remains a leading cause of non-relapse morbidity and mortality following allogeneic HSCT for malignant disease, occurring in 20 to 50% of children surviving greater than 100 days post-HSCT (Baird et al. (2010) Pediatr. Clin. North Am. 57:297-322). Donor cell-mediated immune responses are responsible for GVL and GVHD reactions. Inadequate recognition and destruction of residual tumor cells by a newly engrafted donor immune system permits recurrence of a patient's malignancy, while uncontrolled reactions against host antigens lead to GVHD (Antin (1993) Blood 82:2273-2277; Ferrara et al. (2009) Lancet 373:1550-1561). Chronic GVHD pathogenesis involves inflammatory T- and B-cell responses to allogeneic (donor/recipient polymorphic) and autologous (donor/recipient non-polymorphic) antigens and it remains a common problem and major therapeutic challenge after allogeneic HSCT, and long-term survivors often experience impaired quality of life and increased late mortality (Subramaniam et al. (2007) Leukemia 21:853-859). The increasing use of mobilized peripheral blood progenitor cells rather than bone marrow as a source of stem cells for HCT has resulted in a clear increase in the incidence of cGVHD (Cutler et al. (2001) J. Clin. Oncol. 19:3685-3691; Lee et al. (2007) Blood 110:4576-4583). The incidence of cGVHD in pediatric patients is expected to rise as allogeneic HSCT is increasingly being performed for non-malignant indications such as sickle cell anemia, immunodeficiency and congenital metabolic diseases. In both adults and children, the inflammatory or fibrotic changes associated with cGVHD most commonly involve the skin, eyes, mouth, liver and respiratory tract. PD-1 expression and/or inhibition can be downregulated in advance of any adoptive cell therapy, such as stem cell therapy, organ transplantation, and the like.

By contrast, the present invention also provides methods for increasing immune responses, such as by preventing or reversing T cell exhaustion. Agents that upregulate immune responses can be in the form of enhancing an existing immune response or eliciting an initial immune response. Thus, enhancing an immune response using the subject compositions and methods is useful for treating cancer, but can also be useful for treating an infectious disease (*e.g.,* bacteria, viruses, or parasites), a parasitic infection, and an immunosuppressive disease.

Exemplary infectious disorders include viral skin diseases, such as Herpes or shingles, in which case such an agent can be delivered topically to the skin. In addition, systemic viral diseases, such as influenza, the common cold, and encephalitis might be alleviated by systemic administration of such agents. In one preferred embodiment according to the disclosure, agents that upregulate the immune response described herein are useful for modulating the arginase/iNOS balance during *Trypanosoma cruzi* infection in order to facilitate a protective immune response against the parasite.

Immune responses can also be enhanced in an infected patient through an *ex vivo* approach, for instance, by removing immune cells from the patient, contacting immune cells *in vitro* with an agent described herein and reintroducing the *in vitro* stimulated immune cells into the patient.

### b. Administration of agents

Modulatory methods of the present invention, as described above, involve genetically engineering a T cell to modulate the expression of at least one gene in the T cell by modulating a genomic region, wherein the genomic region is selectively chromatin accessible in the genome of an exhausted CD8+ T cell and the genomie region regulates the at least one gene. The engineered T cells can be analyzed *in vitro* or *ex vivo* (*i.e.,* isolated from a subject and manipulated outside the subject's body with minimal manipulations designed to preserve as much *in vivo* quality as possible, either alone or with administration of the manipulated cells back to the subject). Alternatively, the engineered T cells can be administered to subjects described above in order to modulate T cell exhaustion and/or treat immune disorders.

The engineered T cells will have an immunocompatibility relationship to the subject host and any such relationship is contemplated for use according to the present invention. For example, the Tregs and/or Teffs can be syngeneic. The term "syngeneic" can refer to the state of deriving from, originating in, or being members of the same species that are genetically identical, particularly with respect to antigens or immunological reactions. These include identical twins having matching MHC types. Thus, a "syngeneic transplant" refers to transfer of cells from a donor to a recipient who is genetically identical to the donor or is sufficiently immunologically compatible as to allow for transplantation without an undesired adverse immunogenic response (*e.g.,* such as one that would work against interpretation of immunological screen results described herein).

A syngeneic transplant can be "autologous" if the transferred cells are obtained from and transplanted to the same subject. An "autologous transplant" refers to the harvesting and reinfusion or transplant of a subject's own cells or organs. Exclusive or supplemental use of autologous cells may eliminate or reduce many adverse effects of administration of the cells back to the host, particular graft versus host reaction.

A syngeneic transplant can be "matched allogeneic" if the transferred cells are obtained from and transplanted to different members of the same species yet have sufficiently matched major histocompatibility complex (MHC) antigens to avoid an adverse immunogenic response. Determining the degree of MHC mismatch may be accomplished according to standard tests known and used in the art. For instance, there are at least six major categories of MHC genes in humans, identified as being important in transplant biology. HLA-A, HLA-B, HLA-C encode the HLA class I proteins while HLA-DR, HLA-DQ, and HLA-DP encode the HLA class II proteins. Genes within each of these groups are highly polymorphic, as reflected in the numerous HLA alleles or variants found in the human population, and differences in these groups between individuals is associated with the strength of the immune response against transplanted cells. Standard methods for determining the degree of MHC match examine alleles within HLA-B and HLA-DR, or HLA-A, HLA-B and HLA-DR groups. Thus, tests may be made of at least 4, and even 5 or 6 MHC antigens within the two or three HLA groups, respectively. In serological MHC tests, antibodies directed against each HLA antigen type are reacted with cells from one subject (*e.g.,* donor) to determine the presence or absence of certain MHC antigens that react with the antibodies. This is compared to the reactivity profile of the other subject (*e.g.,* recipient). Reaction of the antibody with an MHC antigen is typically determined by incubating the antibody with cells, and then adding complement to induce cell lysis (*i.e.*, lymphocytotoxicity testing). The reaction is examined and graded according to the amount of cells lysed in the reaction (see, for example, Mickelson and Petersdorf (1999) Hematopoietic Cell Transplantation, Thomas, E. D. et al. eds., pg 28-37, Blackwell Scientific, Malden, Mass.). Other cell-based assays include flow cytometry using labeled antibodies or enzyme linked immunoassays (ELISA). Molecular methods for determining MHC type are well-known and generally employ synthetic probes and/or primers to detect specific gene sequences that encode the HLA protein. Synthetic oligonucleotides may be used as hybridization probes to detect restriction fragment length polymorphisms associated with particular HLA types (Vaughn (2002) Method. Mol. Biol. MHC Protocol. 210:45-60). Alternatively, primers may be used for amplifying the HLA sequences (*e.g.,* by polymerase chain reaction or ligation chain reaction), the products of which may be further examined by direct DNA sequencing, restriction fragment polymorphism analysis (RFLP), or hybridization with a series of sequence specific oligonucleotide primers (SSOP) (Petersdorf et al. (1998) Blood 92:3515-3520; Morishima et al. (2002) Blood 99:4200-4206; and Middleton and Williams (2002) Method. Mol. Biol. MHC Protocol. 210:67-112).

A syngeneic transplant can be "congenic" if the transferred cells and cells of the subject differ in defined loci, such as a single locus, typically by inbreeding. The term "congenic" refers to deriving from, originating in, or being members of the same species, where the members are genetically identical except for a small genetic region, typically a single genetic locus *(i.e.,* a single gene). A "congenic transplant" refers to transfer of cells or organs from a donor to a recipient, where the recipient is genetically identical to the donor except for a single genetic locus. For example, CD45 exists in several allelic forms and congenic mouse lines exist in which the mouse lines differ with respect to whether the CD45.1 or CD45.2 allelic versions are expressed.

By contrast, "mismatched allogeneic" refers to deriving from, originating in, or being members of the same species having non-identical major histocompatibility complex (MHC) antigens *(i.e.,* proteins) as typically determined by standard assays used in the art, such as serological or molecular analysis of a defined number of MHC antigens, sufficient to elicit adverse immunogenic responses. A "partial mismatch" refers to partial match of the MHC antigens tested between members, typically between a donor and recipient. For instance, a "half mismatch" refers to 50% of the MHC antigens tested as showing different MHC antigen type between two members. A "full" or "complete" mismatch refers to all MHC antigens tested as being different between two members.

Similarly, in contrast, "xenogeneic" refers to deriving from, originating in, or being members of different species, *e.g.,* human and rodent, human and swine, human and chimpanzee, etc. A "xenogeneic transplant" refers to transfer of cells or organs from a donor to a recipient where the recipient is a species different from that of the donor.

For cell-based agents, engineered T cells can be administered at 0.1 × 10⁶, 0.2 × 10⁶, 0.3 × 10⁶, 0.4 × 10⁶, 0.5 × 10⁶, 0.6 × 10⁶, 0.7 × 10⁶, 0.8 × 10⁶, 0.9 × 10⁶, 1.0 × 10⁶, 5.0 × 10⁶, 1.0 × 10⁷, 5.0 × 10⁷, 1.0 × 10⁸, 5.0 × 10⁸, or more, or any range in between or any value in between, cells per kilogram of subject body weight. The number of cells transplanted may be adjusted based on the desired level of engraftment in a given amount of time. Generally, 1×10⁵ to about 1×10⁹ cells/kg of body weight, from about 1×10⁶ to about 1×10⁸ cells/kg of body weight, or about 1×10⁷ cells/kg of body weight, or more cells, as necessary, may be transplanted. In some embodiment according to the disclosure, transplantation of at least about 0.1×10⁶, 0.5×10⁶, 1.0×10⁶, 2.0×10⁶, 3.0×10⁶, 4.0×10⁶, or 5.0×10⁶ total cells relative to an average size mouse is effective.

Two or more cell types can be combined and administered, such as genetically engineered T cells and adoptive cell transfer of stem cells, genetically engineered T cells and regular Tregs, genetically engineered T cells and cell-based vaccines, genetically engineered T cells in combination with CAR T cells, and the like. For example, adoptive cell-based immunotherapies can be combined with genetically engineered T cells. Well-known adoptive cell-based immunotherapeutic modalities, including, without limitation, irradiated autologous or allogeneic tumor cells, tumor lysates or apoptotic tumor cells, antigen-presenting cell-based immunotherapy, dendritic cell-based immunotherapy, adoptive T cell transfer, adoptive CAR T cell therapy, autologous immune enhancement therapy (AIET), cancer vaccines, and/or antigen presenting cells. Such cell-based immunotherapies can be further modified to express one or more gene products to further modulate immune responses, such as expressing cytokines like GM-CSF, and/or to express tumor-associated antigen (TAA) antigens, such as Mage-1, gp-100, and the like. The ratio of genetically engineered T cells to other cell types can be 1:1, but can modulated in any amount desired *(e.g.,* 1:1, 1.1:1, 1.2:1, 1.3:1, 1.4:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1, 10:1, or greater).

Engraftment of transplanted cells may be assessed by any of various methods, such as, but not limited to, tumor volume, cytokine levels, time of administration, flow cytometric analysis of cells of interest obtained from the subject at one or more time points following transplantation, and the like. For example, a time-based analysis of waiting 1, 2, 3, 4, 5, 6, 7, 8, 9, 10, 11, 12, 13, 14, 15, 16, 17, 18, 19, 20, 21, 22, 23, 24, 25, 26, 27, 28 days or can signal the time for tumor harvesting. Any such metrics are variables that can be adjusted according to well-known parameters in order to determine the effect of the variable on a response to anti-immune disorder therapy. In addition, the transplanted cells can be co-transplanted with other agents, such as cytokines, extracellular matrices, cell culture supports, and the like.

In addition, immune modulating agents of the present invention can be administered to subjects or otherwise applied outside of a subject body in a biologically compatible form suitable for pharmaceutical administration, to modulate immune cell mediated immune responses. By "biologically compatible form suitable for administration *in vivo"* is meant a form to be administered in which any toxic effects are outweighed by the therapeutic effects. The term "subject" is intended to include living organisms in which an immune response can be elicited, *e.g.,* mammals. Examples of subjects include humans, dogs, cats, mice, rats, and transgenic species thereof. Administration of an agent as described herein can be in any pharmacological form including a therapeutically active amount of an agent alone or in combination with a pharmaceutically acceptable carrier.

Administration of a therapeutically active amount of the therapeutic composition of the present invention is defined as an amount effective, at dosages and for periods of time necessary, to achieve the desired result. For example, a therapeutically active amount of an agent may vary according to factors such as the disease state, age, sex, and weight of the individual, and the ability of peptide to elicit a desired response in the individual. Dosage regimens can be adjusted to provide the optimum therapeutic response. For example, several divided doses can be administered daily or the dose can be proportionally reduced as indicated by the exigencies of the therapeutic situation.

A combination dosage form or simultaneous administration of single agents can result in effective amounts of each desired modulatory agent present in the patient at the same time.

Administration can be accomplished using methods generally known in the art. Agents, including cells, may be introduced to the desired site by direct injection, or by any other means used in the art including, but are not limited to, intravascular, intracerebral, parenteral, intraperitoneal, intravenous, epidural, intraspinal, intrasternal, intra-articular, intra-synovial, intrathecal, intra-arterial, intracardiac, or intramuscular administration. For example, subjects of interest may be engrafted with the transplanted cells by various routes. Such routes include, but are not limited to, intravenous administration, subcutaneous administration, administration to a specific tissue (*e.g.,* focal transplantation), injection into the femur bone marrow cavity, injection into the spleen, administration under the renal capsule of fetal liver, and the like. Cells may be administered in one infusion, or through successive infusions over a defined time period sufficient to generate a desired effect. Exemplary methods for transplantation, engraftment assessment, and marker phenotyping analysis of transplanted cells are well-known in the art (see, for example, Pearson et al. (2008) Curr. Protoc. Immunol. 81:15.21.1-15.21.21; Ito et al. (2002) Blood 100:3175-3182; Traggiai et al. (2004) Science 304:104-107; Ishikawa et al. Blood (2005) 106:1565-1573; Shultz et al. (2005) J. Immunol. 174:6477-6489; and Holyoake et al. (1999) Exp. Hematol. 27:1418-1427).

For example, the therapeutic agents described herein can be administered in a convenient manner such as by injection (subcutaneous, intravenous, etc.), oral administration, inhalation, transdermal application, or rectal administration. Depending on the route of administration, the active compound can be coated in a material to protect the compound from the action of enzymes, acids and other natural conditions which may inactivate the compound. For example, for administration of agents, by other than parenteral administration, it may be desirable to coat the agent with, or co-administer the agent with, a material to prevent its inactivation.

An agent can be administered to an individual in an appropriate carrier, diluent or adjuvant, co-administered with enzyme inhibitors or in an appropriate carrier such as liposomes. Pharmaceutically acceptable diluents include saline and aqueous buffer solutions. Adjuvant is used in its broadest sense and includes any immune stimulating compound such as interferon. Adjuvants contemplated herein include resorcinols, nonionic surfactants such as polyoxyethylene oleyl ether and n-hexadecyl polyethylene ether. Enzyme inhibitors include pancreatic trypsin inhibitor, diisopropylfluorophosphate (DEEP) and trasylol. Liposomes include water-in-oil-in-water emulsions as well as conventional liposomes (Sterna et al. (1984) J. Neuroimmunol. 7:27).

The agent may also be administered parenterally or intraperitoneally. Dispersions can also be prepared in glycerol, liquid polyethylene glycols, and mixtures thereof, and in oils. Under ordinary conditions of storage and use, these preparations may contain a preservative to prevent the growth of microorganisms.

Pharmaceutical compositions of agents suitable for injectable use include sterile aqueous solutions (where water soluble) or dispersions and sterile powders for the extemporaneous preparation of sterile injectable solutions or dispersion. In all cases the composition will preferably be sterile and must be fluid to the extent that easy syringeability exists. It will preferably be stable under the conditions of manufacture and storage and preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it is preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating a genetically engineered T cell and/or other agent of the present invention (*e.g.,* an antibody, peptide, fusion protein or small molecule) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the agent plus any additional desired ingredient from a previously sterile-filtered solution thereof.

When the agent is suitably protected, as described above, the protein can be orally administered, for example, with an inert diluent or an assimilable edible carrier. As used herein "pharmaceutically acceptable carrier" includes any and all solvents, dispersion media, coatings, antibacterial and antifungal agents, isotonic and absorption delaying agents, and the like. The use of such media and agents for pharmaceutically active substances is well-known in the art. Except insofar as any conventional media or agent is incompatible with the active compound, use thereof in the therapeutic compositions is contemplated. Supplementary active compounds can also be incorporated into the compositions.

It is especially advantageous to formulate parenteral compositions in dosage unit form for ease of administration and uniformity of dosage. "Dosage unit form ", as used herein, refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by, and directly dependent on, (a) the unique characteristics of the active compound and the particular therapeutic effect to be achieved, and (b) the limitations inherent in the art of compounding such an active compound for the treatment of sensitivity in individuals.

In one embodiment according to the disclosure, a therapeutic agent of the present invention is an antibody. As defined herein, a therapeutically effective amount of antibody *(i.e.,* an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of an antibody can include a single treatment or, preferably, can include a series of treatments. In a preferred example, a subject is treated with antibody in the range of between about 0.1 to 20 mg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of antibody used for treatment may increase or decrease over the course of a particular treatment. Changes in dosage may result from the results of diagnostic assays. In some embodiments according to the disclosure, efficacy of treatment occurs and can be measured directly or indirectly within 1, 2, 3, 4, 5, 6, 7, 8, 9, 10, 11, 12, 13, 14, or more days after initiation of administration.

In some embodiments according to the disclosure, biomarker nucleic acid molecules are useful and can be inserted into vectors and used as gene therapy vectors. Gene therapy vectors can be delivered to a subject by, for example, intravenous injection, local administration (see U.S. Pat. No. 5,328,470) or by stereotactic injection (see *e.g.,* Chen et al. (1994) Proc. Natl. Acad. Sci. USA 91:3054 3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

Any means for the introduction of a polynucleotide into mammals, human or non-human, or cells thereof may be adapted to the practice of this invention for the delivery of the various constructs of the invention into the intended recipient. In one embodiment of the disclosure, the DNA constructs are delivered to cells by transfection, i.e., by delivery of "naked" DNA or in a complex with a colloidal dispersion system. A colloidal system includes macromolecule complexes, nanocapsules, microspheres, beads, and lipid-based systems including oil-in-water emulsions, micelles, mixed micelles, and liposomes. The preferred colloidal system of this invention is a lipid-complexed or liposome-formulated DNA. In the former approach, prior to formulation of DNA, e.g., with lipid, a plasmid containing a transgene bearing the desired DNA constructs may first be experimentally optimized for expression (*e.g*., inclusion of an intron in the 5' untranslated region and elimination of unnecessary sequences (Felgner, et al., Ann NY Acad Sci 126-139, 1995). Formulation of DNA, *e.g.* with various lipid or liposome materials, may then be effected using known methods and materials and delivered to the recipient mammal. See, *e.g.,* Canonico et al, Am J Respir Cell Mol Biol 10:24-29, 1994; Tsan et al, Am J Physiol 268; Alton et al., Nat Genet. 5: 135-142, 1993 and U.S. patent No. 5,679,647 by Carson et al.

The targeting of liposomes can be classified based on anatomical and mechanistic factors. Anatomical classification is based on the level of selectivity, for example, organ-specific, cell-specific, and organelle-specific. Mechanistic targeting can be distinguished based upon whether it is passive or active. Passive targeting utilizes the natural tendency of liposomes to distribute to cells of the reticulo-endothelial system (RES) in organs, which contain sinusoidal capillaries. Active targeting, on the other hand, involves alteration of the liposome by coupling the liposome to a specific ligand such as a monoclonal antibody, sugar, glycolipid, or protein, or by changing the composition or size of the liposome in order to achieve targeting to organs and cell types other than the naturally occurring sites of localization.

The surface of the targeted delivery system may be modified in a variety of ways. In the case of a liposomal targeted delivery system, lipid groups can be incorporated into the lipid bilayer of the liposome in order to maintain the targeting ligand in stable association with the liposomal bilayer. Various linking groups can be used for joining the lipid chains to the targeting ligand. Naked DNA or DNA associated with a delivery vehicle, e.g., liposomes, can be administered to several sites in a subject (see below).

Nucleic acids can be delivered in any desired vector. These include viral or non-viral vectors, including adenovirus vectors, adeno-associated virus vectors, retrovirus vectors, lentivirus vectors, and plasmid vectors. Exemplary types of viruses include HSV (herpes simplex virus), AAV (adeno associated virus), HIV (human immunodeficiency virus), BIV (bovine immunodeficiency virus), and MLV (murine leukemia virus). Nucleic acids can be administered in any desired format that provides sufficiently efficient delivery levels, including in virus particles, in liposomes, in nanoparticles, and complexed to polymers.

The nucleic acids encoding a protein or nucleic acid of interest may be in a plasmid or viral vector, or other vector as is known in the art. Such vectors are well-known and any can be selected for a particular application. In one embodiment of the disclosure, the gene delivery vehicle comprises a promoter and a demethylase coding sequence. Preferred promoters are tissue-specific promoters and promoters which are activated by cellular proliferation, such as the thymidine kinase and thymidylate synthase promoters. Other preferred promoters include promoters which are activatable by infection with a virus, such as the α- and β-interferon promoters, and promoters which are activatable by a hormone, such as estrogen. Other promoters which can be used include the Moloney virus LTR, the CMV promoter, and the mouse albumin promoter. A promoter may be constitutive or inducible.

In another embodiment according to the disclosure, naked polynucleotide molecules are used as gene delivery vehicles, as described in WO 90/11092 and U.S. Patent 5,580,859. Such gene delivery vehicles can be either growth factor DNA or RNA and, in certain embodiments according to the disclosure, are linked to killed adenovirus. Curiel et al., Hum. Gene. Ther. 3:147-154, 1992. Other vehicles which can optionally be used include DNA-ligand (Wu et al., J. Biol. Chem. 264:16985-16987, 1989), lipid-DNA combinations (Felgner et al., Proc. Natl. Acad. Sci. USA 84:7413 7417, 1989), liposomes (Wang et al., Proc. Natl. Acad. Sci. 84:7851-7855, 1987) and microprojectiles (Williams et al., Proc. Natl. Acad. Sci. 88:2726-2730, 1991).

A gene delivery vehicle can optionally comprise viral sequences such as a viral origin of replication or packaging signal. These viral sequences can be selected from viruses such as astrovirus, coronavirus, orthomyxovirus, papovavirus, paramyxovirus, parvovirus, picornavirus, poxvirus, retrovirus, togavirus or adenovirus. In a preferred embodiment according to the disclosure, the growth factor gene delivery vehicle is a recombinant retroviral vector. Recombinant retroviruses and various uses thereof have been described in numerous references including, for example, Mann et al., Cell 33:153, 1983, Cane and Mulligan, Proc. Nat'l. Acad. Sci. USA 81:6349, 1984, Miller et al., Human Gene Therapy 1:5-14, 1990, U.S. Patent Nos. 4,405,712, 4,861,719, and 4,980,289, and PCT Application Nos. WO 89/02,468, WO 89/05,349, and WO 90/02,806. Numerous retroviral gene delivery vehicles can be utilized in the present invention, including for example those described in EP 0,415,731; WO 90/07936; WO 94/03622; WO 93/25698; WO 93/25234; U.S. Patent No. 5,219,740; WO 9311230; WO 9310218; Vile and Hart, Cancer Res. 53:3860-3864, 1993; Vile and Hart, Cancer Res. 53:962-967, 1993; Ram et al., Cancer Res. 53:83-88, 1993; Takamiya et al., J. Neurosci. Res. 33:493-503, 1992; Baba et al., J. Neurosurg. 79:729-735, 1993 (U.S. Patent No. 4,777,127, GB 2200651, EP 0345242, and WO 91/02805).

Other viral vector systems that can be used to deliver a polynucleotide of the invention have been derived from herpes virus, *e.g.,* Herpes Simplex Virus (U.S. Patent No. 5,631,236 by Woo et al., issued May 20, 1997 and WO 00/08191 by Neurovex), vaccinia virus (Ridgeway (1988) Ridgeway, "Mammalian expression vectors," In: Rodriguez R L, Denhardt D T, ed. Vectors: A survey of molecular cloning vectors and their uses. Stoneham: Butterworth; Baichwal and Sugden (1986) "Vectors for gene transfer derived from animal DNA viruses: Transient and stable expression of transferred genes," In: Kucherlapati R, ed. Gene transfer. New York: Plenum Press; Coupar et al. (1988) Gene, 68:1-10), and several RNA viruses. Preferred viruses include an alphavirus, a poxivirus, an arena virus, a vaccinia virus, a polio virus, and the like. They offer several attractive features for various mammalian cells (Friedmann (1989) Science, 244:1275-1281; Ridgeway, 1988, supra; Baichwal and Sugden, 1986, supra; Coupar *et al.,* 1988; Horwich et al.(1990) J. Virol., 64:642-650).

In other embodiments according to the disclosure, recombinant biomarker polypeptides, and fragments thereof, can be administered to subjects. In some embodiments according to the disclosure, fusion proteins can be constructed and administered which have enhanced biological properties. In addition, the biomarker polypeptides, and fragment thereof, can be modified according to well-known pharmacological methods in the art *(e.g.,* pegylation, glycosylation, oligomerization, *etc.)* in order to further enhance desirable biological activities, such as increased bioavailability and decreased proteolytic degradation.

Stimulation of biomarker activity is desirable in situations in which the biomarker is abnormally downregulated and/or in which increased biomarker activity is likely to have a beneficial effect. Likewise, inhibition of biomarker activity is desirable in situations in which biomarker is abnormally upregulated and/or in which decreased biomarker activity is likely to have a beneficial effect.

In addition, these modulatory agents can also be administered in combination therapy with, *e.g.,* chemotherapeutic agents, hormones, antiangiogens, radiolabelled, compounds, or with surgery, cryotherapy, and/or radiotherapy. The preceding treatment methods can be administered in conjunction with other forms of conventional therapy (*e.g.,* standard-of-care treatments for immune disorders well-known to the skilled artisan), either consecutively with, pre- or post-conventional therapy. For example, these modulatory agents can be administered with a therapeutically effective dose of an immunosuppressive agent or therapy.

In another embodiment according to the disclosure, the immune response can be downregulated by the methods described herein, in order to maintain preexisting tolerance, clonal deletion, and/or exhaustion (*e.g.,* T cell exhaustion). For example, immune responses against antigens to which a subject cannot mount a significant immune response, *e.g.,* to an autologous antigen, can be maintained.

In one embodiment according to the disclosure, immune cells are obtained from a subject and cultured *ex vivo* in the presence of an agent as described herein, to modify Tregs and/or remove or further remove a population of immune cells that enhance immune cell activation. In a further embodiment according to the disclosure the filtered immune cells are then administered to a subject. Since immune cells can be stimulated *in vitro* by, for example, providing to the immune cells a primary activation signal and a costimulatory signal, various agents can also be used to reduce the costimulation and proliferation of effector immune cells and/or promote inhibitory regulatory immune cells. In one embodiment according to the disclosure immune cells are cultured *ex vivo* according to the method described in PCT Application No. WO 94/29436. The costimulatory polypeptide can be soluble, attached to a cell membrane, or attached to a solid surface, such as a bead. Similarly, agents useful for promoting immune cell depletion can further be linked, or operatively attached, to toxins using techniques that are known in the art, *e.g.,* crosslinking or via recombinant DNA techniques. Such agents can result in cellular destruction of desired cells. In one embodiment according to the disclosure, a toxin can be conjugated to an antibody, such as a bispecific antibody. Such antibodies are useful for targeting a specific cell population, *e.g.,* using a marker found only on a certain type of cell. The preparation of immunotoxins is, in general, well-known in the art (see, *e.g.,* U.S. Pat. Nos. 4,340,535, and EP 44167). Numerous types of disulfide-bond containing linkers are known which can successfully be employed to conjugate the toxin moiety with a polypeptide. In one embodiment according to the disclosure, linkers that contain a disulfide bond that is sterically "hindered" are preferred, due to their greater stability *in vivo,* thus preventing release of the toxin moiety prior to binding at the site of action. A wide variety of toxins are known that may be conjugated to polypeptides or antibodies of the disclosure. Examples include: numerous useful plant-, fungus- or even bacteria-derived toxins, which, by way of example, include various A chain toxins, particularly ricin A chain, ribosome inactivating proteins such as saporin or gelonin, α-sarcin, aspergillin, restrictocin, ribonucleases, such as placental ribonuclease, angiogenic, diphtheria toxin, and *Pseudomonas* exotoxin, etc. A preferred toxin moiety for use in connection with the invention is toxin A chain which has been treated to modify or remove carbohydrate residues, deglycosylated A chain. (U.S. Patent 5,776,427). Infusion of one or a combination of such cytotoxic agents, (*e.g.,* ricin fusions) into a patient may result in the death of immune cells.

In yet another embodiment according to the disclosure, the efficacy of the treatment methods described herein can be enhanced by incorporating a step of lymphodepletion prior to, concurrently with, or after the administration of agents described herein. For example, therapeutic benefits of administering the described agents can be synergistically enhanced by performing such administration after or in conjunction with lymphodepletion. Methods for achieving lymphodepletion in various forms and at various levels are well-known in the art (see, for example, U.S. Patent 7,138,144). For example, the term "transient lymphodepletion" refers to destruction of lymphocytes and T cells, usually prior to immunotherapy. This can be accomplished in a number of ways, including "sublethal irradiation," which refers to administration of one or more doses of radiation that is generally non-lethal to all members of a population of subjects to which the administration is applied. Transient lymphodepletion is generally not myeloablative, as would be the case in complete lymphodepletion, such that the subject's hematopoietic or immunological capacity remains sufficiently intact to regenerate the destroyed lymphocyte and T cell populations. By contrast, "lethal irradiation" occurs when the administration is generally lethal to some but not all members of the population of subjects and "supralethal irradiation" occurs when the administration is generally lethal to all members of the population of subjects.

Depending on the application and purpose, transient lymphodepletion or complete lymphodepletion may be effected, for example, by any combination of irradiation, treatment with a myeloablative agent, and/or treatment with an immunosuppressive agent, according to standard protocols. For example, biological methods include administration of immunity-suppressing cells or by administration of biological molecules capable of inhibiting immunoreactivity, such as, for example, Fas-ligand and CTLA4-Ig. Examples of myeloablative agents include busulfan, dimethyl mileran, melphalan and thiotepa. Examples of immunosuppressive agents include prednisone, methyl prednisolone, azathioprine, cyclosporine A, cyclophosphamide, fludarabin, CTLA4-Ig, anti-T cell antibodies, etc.

Regarding irradiation, a sublethal dose of irradiation is generally within the range of 1 to 7.5 Gy whole body irradiation, a lethal dose is generally within the range of 7.5 to 9.5 Gy whole body irradiation, and a supralethal dose is within the range of 9.5 to 16.5 Gy whole body irradiation.

Depending on the purpose and application, the dose of irradiation may be administered as a single dose or as a fractionated dose. Similarly, administering one or more doses of irradiation can be accomplished essentially exclusively to the body part or to a portion thereof, so as to induce myeloreduction or myeloablation essentially exclusively in the body part or the portion thereof. As is widely recognized in the art, a subject can tolerate as sublethal conditioning ultra-high levels of selective irradiation to a body part such as a limb, which levels constitute lethal or supralethal conditioning when used for whole body irradiation (see, for example, Breitz (2002) Cancer Biother Radiopharm. 17:119; Limit (1997) J. Nucl. Med. 38:1374; and Dritschilo and Sherman (1981) Environ. Health Perspect. 39:59). Such selective irradiation of the body part, or portion thereof, can be advantageously used to target particular blood compartments, such as specific tissues or immune cell populations, in treating immune disorders.

### c. Additional agents and therapies useful for modulating T cell exhaustion and/or immune disorders

The genetically engineered T cells described herein can be used alone or in combination with one or more additional therapeutic agents. The modulatory agents described herein (*e.g.,* antibodies, small molecules, peptides, fusion proteins, or small nucleic acids) can be incorporated into pharmaceutical compositions and administered to a subject *in vivo.* The compositions may contain a single such molecule or agent or any combination of agents described herein. "Single active agents" described herein can be combined with other pharmacologically active compounds ("second active agents") known in the art according to the methods and compositions provided herein. It is believed that certain combinations work synergistically in the treatment of conditions that would benefit from the modulation of immune responses. Second active agents can be large molecules (*e.g.,* proteins) or small molecules (*e.g.,* synthetic inorganic, organometallic, or organic molecules). For example, anti-PD-L1 and anti-TIM-3 antibodies can be further combined with anti-LAG-3, anti-PD-L2, anti-CTLA4, etc. antibodies or combinations thereof.

In certain instances, it may be desirable to further administer other agents that upregulate immune responses, for example, forms of other B7 family members that transduce signals via costimulatory receptors, in order to further augment the immune response. Such additional agents and therapies are described further below.

Agents that upregulate an immune response can be used prophylactically in vaccines against various polypeptides (*e.g.,* polypeptides derived from pathogens). Immunity against a pathogen (*e.g.,* a virus) can be induced by vaccinating with a viral protein along with an agent that upregulates an immune response, in an appropriate adjuvant.

In another embodiment according to the disclosure, upregulation or enhancement of an immune response function, as described herein, is useful in the induction of tumor immunity.

In another embodiment according to the disclosure, the immune response can be stimulated by the methods described herein, such that preexisting tolerance, clonal deletion, and/or exhaustion (*e.g.,* T cell exhaustion) is overcome. For example, immune responses against antigens to which a subject cannot mount a significant immune response, *e.g.,* to an autologous antigen, such as a tumor specific antigens can be induced by administering appropriate agents described herein that upregulate the immune response. In one embodiment, an autologous antigen, such as a tumor-specific antigen, can be coadministered. In another embodiment according to the disclosure, the subject agents can be used as adjuvants to boost responses to foreign antigens in the process of active immunization.

In one embodiment according to the disclosure, immune cells are obtained from a subject and cultured *ex vivo* in the presence of an agent as described herein, to expand the population of immune cells and/or to enhance immune cell activation. In a further embodiment according to the disclosure the immune cells are then administered to a subject. Immune cells can be stimulated *in vitro* by, for example, providing to the immune cells a primary activation signal and a costimulatory signal, as is known in the art. Various agents can also be used to costimulate proliferation of immune cells. In one embodiment according to the disclosure immune cells are cultured *ex vivo* according to the method described in PCT Application No. WO 94/29436. The costimulatory polypeptide can be soluble, attached to a cell membrane, or attached to a solid surface, such as a bead.

In still another embodiment according to the disclosure, agents described herein useful for upregulating immune responses can further be linked, or operatively attached, to toxins using techniques that are known in the art, *e.g.,* crosslinking or via recombinant DNA techniques. Such agents can result in cellular destruction of desired cells. In one embodiment according to the disclosure, a toxin can be conjugated to an antibody, such as a bispecific antibody. Such antibodies are useful for targeting a specific cell population, *e.g.,* using a marker found only on a certain type of cell. The preparation of immunotoxins is, in general, well known in the art (see, *e.g.,* U.S. Pat. Nos. 4,340,535, and EP 44167). Numerous types of disulfide-bond containing linkers are known which can successfully be employed to conjugate the toxin moiety with a polypeptide. In one embodiment according to the disclosure, linkers that contain a disulfide bond that is sterically "hindered" are preferred, due to their greater stability *in vivo,* thus preventing release of the toxin moiety prior to binding at the site of action. A wide variety of toxins are known that may be conjugated to polypeptides or antibodies of the invention. Examples include: numerous useful plant-, fungus- or even bacteria-derived toxins, which, by way of example, include various A chain toxins, particularly ricin A chain, ribosome inactivating proteins such as saporin or gelonin, α-sarcin, aspergillin, restrictocin, ribonucleases, such as placental ribonuclease, angiogenic, diphtheria toxin, and *Pseudomonas* exotoxin, etc. A preferred toxin moiety for use in connection with the invention is toxin A chain which has been treated to modify or remove carbohydrate residues, deglycosylated A chain (U.S. Patent 5,776,427). Infusion of one or a combination of such cytotoxic agents, (*e.g.,* ricin fusions) into a patient may result in the death of immune cells.

In another embodiment according to the disclosure, certain combinations work synergistically in the treatment of conditions that would benefit from the modulation of immune responses. Second active agents can be large molecules (*e.g.,* proteins) or small molecules (*e.g.,* synthetic inorganic, organometallic, or organic molecules). For example, immune checkpoint inhibitors can be further combined with other agents or therapies useful in treating a condition of interest.

Moreover, certain immunotherapies can be used to promote immune responses. Immunotherapy can involve passive immunity for short-term protection of a host, achieved by the administration of pre-formed antibody directed against a cancer antigen or disease antigen (*e.g.,* administration of a monoclonal antibody, optionally linked to a chemotherapeutic agent or toxin, to a tumor antigen). Immunotherapy can also focus on using the cytotoxic lymphocyte-recognized epitopes of cancer cell lines. Alternatively, antisense polynucleotides, ribozymes, RNA interference molecules, triple helix polynucleotides and the like, can be used to selectively modulate biomolecules that are linked to the initiation, progression, and/or pathology of a tumor or cancer.

In one embodiment according to the disclosure, immunotherapy comprises adoptive cell-based immunotherapies. Well known adoptive cell-based immunotherapeutic modalities, including, without limitation, irradiated autologous or allogeneic tumor cells, tumor lysates or apoptotic tumor cells, antigen-presenting cell-based immunotherapy, dendritic cell-based immunotherapy, adoptive T cell transfer, adoptive CAR T cell therapy, autologous immune enhancement therapy (AIET), cancer vaccines, and/or antigen presenting cells. Such cell-based immunotherapies can be further modified to express one or more gene products to further modulate immune responses, such as expressing cytokines like GM-CSF, and/or to express tumor-associated antigen (TAA) antigens, such as Mage-1, gp-100, patient-specific neoantigen vaccines, and the like.

In another embodiment according to the disclosure, immunotherapy comprises non-cell-based immunotherapies. Examples of large molecule active agents include, but are not limited to, hematopoietic growth factors, cytokines, and monoclonal and polyclonal antibodies. Typical large molecule active agents are biological molecules, such as naturally occurring or artificially made proteins. Proteins that are particularly useful in this invention include proteins that stimulate the survival and/or proliferation of hematopoietic precursor cells and immunologically active poietic cells *in vitro* or *in vivo.* Others stimulate the division and differentiation of committed erythroid progenitors in cells *in vitro* or *in vivo.* Particular proteins include, but are not limited to: interleukins, such as IL-2 (including recombinant IL-2 ("rIL2") and canarypox IL-2), IL-10, IL-12, IL-15, and IL-18; interferons, such as interferon alfa-2a, interferon alfa-2b, interferon alpha-n1, interferon alpha-n3, interferon beta-Ia, and interferon gamma-Ib; GM-CF and GM-CSF; and EPO.

Particular proteins that can be used in the methods and compositions provided herein include, but are not limited to: filgrastim, which is sold in the United States under the trade name Neupogen^{®} (Amgen, Thousand Oaks, Calif.); sargramostim, which is sold in the United States under the trade name Leukine^{®} (Immunex, Seattle, Wash.); and recombinant EPO, which is sold in the United States under the trade name Epogen^{®} (Amgen, Thousand Oaks, Calif.). Recombinant and mutated forms of GM-CSF can be prepared as described in U.S. Pat. Nos. 5,391,485; 5,393,870; and 5,229,496. Recombinant and mutated forms of G-CSF can be prepared as described in U.S. Pat. Nos. 4,810,643; 4,999,291; 5,528,823; and 5,580,755.

In one embodiment according to the disclosure, compositions comprising antigens with or without vaccine-enhancing adjuvants are used. Such compositions exist in many well-known forms, such as peptide compositions, oncolytic viruses, recombinant antigen comprising fusion proteins, and the like. In still another embodiment according to the disclosure, immunomodulatory interleukins, such as IL-2, IL-6, IL-7, IL-12, IL-17, IL-23, and the like, as well as modulators thereof (*e.g.,* blocking antibodies or more potent or longer lasting forms) are used. In another embodiment, immunomodulatory chemokines, such as CCL3, CCL26, and CXCL7, and the like, as well as modulators thereof (*e.g.,* blocking antibodies or more potent or longer lasting forms) are used. In another embodiment according to the disclosure, immunomodulatory molecules targeting immunosuppression, such as STAT3 signaling modulators, NFkappaB signaling modulators, and immune checkpoint modulators, are used. The terms "immune checkpoint" and "anti-immune checkpoint therapy" are described above.

In still another embodiment according to the disclosure, immunomodulatory drugs, such as immunocytostatic drugs, glucocorticoids, cytostatics, immunophilins and modulators thereof (*e.g.,* rapamycin, a calcineurin inhibitor, tacrolimus, ciclosporin (cyclosporin), pimecrolimus, abetimus, gusperimus, ridaforolimus, everolimus, temsirolimus, zotarolimus, etc.), hydrocortisone (cortisol), cortisone acetate, prednisone, prednisolone, methylprednisolone, dexamethasone, betamethasone, triamcinolone, beclometasone, fludrocortisone acetate, deoxycorticosterone acetate (doca) aldosterone, a non-glucocorticoid steroid, a pyrimidine synthesis inhibitor, leflunomide, teriflunomide, a folic acid analog, methotrexate, anti-thymocyte globulin, anti-lymphocyte globulin, thalidomide, lenalidomide, pentoxifylline, bupropion, curcumin, catechin, an opioid, an IMPDH inhibitor, mycophenolic acid, myriocin, fingolimod, an NF-xB inhibitor, raloxifene, drotrecogin alfa, denosumab, an NF-xB signaling cascade inhibitor, disulfiram, olmesartan, dithiocarbamate, a proteasome inhibitor, bortezomib, MG132, Prol, NPI-0052, curcumin, genistein, resveratrol, parthenolide, thalidomide, lenalidomide, flavopiridol, non-steroidal anti-inflammatory drugs (NSAIDs), arsenic trioxide, dehydroxymethylepoxyquinomycin (DHMEQ), 13C(indole-3-carbinol)/DIM(di-indolmethane) (13C/DIM), Bay 11-7082, luteolin, cell permeable peptide SN-50, IKBa.-super repressor overexpression, NFKB decoy oligodeoxynucleotide (ODN), or a derivative or analog of any thereo, are used. In yet another embodiment according to the disclosure, immunomodulatory antibodies or protein are used. For example, antibodies that bind to CD40, Toll-like receptor (TLR), OX40, GITR, CD27, or to 4-1BB, T-cell bispecific antibodies, an anti-IL-2 receptor antibody, an anti-CD3 antibody, OKT3 (muromonab), otelixizumab, teplizumab, visilizumab, an anti-CD4 antibody, clenoliximab, keliximab, zanolimumab, an anti-CD11 a antibody, efalizumab, an anti-CD18 antibody, erlizumab, rovelizumab, an anti-CD20 antibody, afutuzumab, ocrelizumab, ofatumumab, pascolizumab, rituximab, an anti-CD23 antibody, lumiliximab, an anti-CD40 antibody, teneliximab, toralizumab, an anti-CD40L antibody, ruplizumab, an anti-CD62L antibody, aselizumab, an anti-CD80 antibody, galiximab, an anti-CD147 antibody, gavilimomab, a B-Lymphocyte stimulator (BLyS) inhibiting antibody, belimumab, an CTLA4-Ig fusion protein, abatacept, belatacept, an anti-CTLA4 antibody, ipilimumab, tremelimumab, an anti-eotaxin 1 antibody, bertilimumab, an anti-a4-integrin antibody, natalizumab, an anti-IL-6R antibody, tocilizumab, an anti-LFA-1 antibody, odulimomab, an anti-CD25 antibody, basiliximab, daclizumab, inolimomab, an anti-CD5 antibody, zolimomab, an anti-CD2 antibody, siplizumab, nerelimomab, faralimomab, atlizumab, atorolimumab, cedelizumab, dorlimomab aritox, dorlixizumab, fontolizumab, gantenerumab, gomiliximab, lebrilizumab, maslimomab, morolimumab, pexelizumab, reslizumab, rovelizumab, talizumab, telimomab aritox, vapaliximab, vepalimomab, aflibercept, alefacept, rilonacept, an IL-1 receptor antagonist, anakinra, an anti-IL-5 antibody, mepolizumab, an IgE inhibitor, omalizumab, talizumab, an IL12 inhibitor, an IL23 inhibitor, ustekinumab, and the like.

Similarly, chemotherapeutic agents are well-known in the art. For example, chemotherapeutic agents include alkylating agents such as thiotepa and cyclophosphamide (Cytoxan^{™}); alkyl sulfonates such as busulfan, improsulfan and piposulfan; aziridines such as benzodopa, carboquone, meturedopa, and uredopa; emylerumines and memylamelamines including alfretamine, triemylenemelamine, triethylenephosphoramide, triethylenethiophosphoramide, and trimemylolomelamine; acetogenins (especially bullatacin and bullatacinone); a camptothecin (including synthetic analogue topotecan); bryostatin; callystatin; CC-1065 (including its adozelesin, carzelesin and bizelesin synthetic analogues); cryptophycins (articularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, foremustine, lomustine, nimustine, ranimustine; antibiotics such as the enediyne antibiotics (*e.g.,* calicheamicin, especially calicheamicin gammall and calicheamicin phili); dynemicin, including dynemicin A; bisphosphonates, such as clodronate; an esperamicin; as well as neocarzinostatin chromophore and related chromoprotein enediyne antibiotic chromomophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (Adramycin^{™}) (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; antimetabolites such as methotrexate and 5-fluorouracil (5-FU); folic acid analogues such as demopterin, methotrexate, pteropterin, trimetrexate; purine analogs such as fludarabine, 6-mercaptopurine, thiamiprine, thioguanine; pyrimidine analogues such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replinisher such as frolinic acid; aceglatone; aldophosphamide glycoside; aminolevulinic acid; eniluracil; amsacrine; hestrabucil; bisantrene; edatraxate; defofamine; demecolcine; diaziquone; elformthine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK^{™}; razoxane; rhizoxin; sizofiran; spirogermanium; tenuazonic acid; triaziquone; 2,2',2"-tricUorotriemylamine; trichothecenes (especially T-2 toxin, verracurin A, roridin A and anguidine); urethane; vindesine; dacarbazine; mannomustine; mitobronitol; mitolactol; pipobroman; gacytosine; arabinoside ("Ara-C"); cyclophosphamide; thiopeta; taxoids, e.g., paclitaxel (Taxol^{™}, Bristol Meyers Squibb Oncology, Princeton, N.J.) and docetaxel (Taxoteret^{™}, Rhone-Poulenc Rorer, Antony, France); chlorambucil; gemcitabine (Gemzar^{™}); 6-thioguanine; mercaptopurine; methotrexate; platinum analogs such as cisplatin and carboplatin; vinblastine; platinum; etoposide (VP-16); ifosfamide; mitroxantrone; vancristine; vinorelbine (Navelbine^{™}); novantrone; teniposide; edatrexate; daunomycin; aminopterin; xeoloda; ibandronate; CPT-11; topoisomerase inhibitor RFS 2000; difluoromethylornithine (DMFO); retinoids such as retinoic acid; capecitabine; and pharmaceutically acceptable salts, acids or derivatives of any of the above. Also included in the definition of "chemotherapeutic agent" are anti-hormonal agents that act to regulate or inhibit hormone action on tumors such as anti-estrogens and selective estrogen receptor modulators (SERMs), including, for example, tamoxifen (including Nolvadex^{™}), raloxifene, droloxifene, 4-hydroxytamoxifen, trioxifene, keoxifene, LY117018, onapristone, and toremifene (Fareston^{™}); inhibitors of the enzyme aromatase, which regulates estrogen production in the adrenal glands, such as, for example, 4(5)-imidazoles, aminoglutethimide, megestrol acetate (Megace^{™}), exemestane, formestane, fadrozole, vorozole (Rivisor^{™}), letrozole (Femara^{™}), and anastrozole (Arimidex^{™}); and antiandrogens such as flutamide, nilutamide, bicalutamide, leuprohde, and goserelin; and pharmaceutically acceptable salts, acids or derivatives of any of the above. In some embodiments according to the disclosure, the inhibitor downregulates Rac1 output. Additional examples of chemotherapeutic and other anti-cancer agents are described in US Pat. Publs. 2013/0239239 and 2009/0053224.

Nutritional supplements that enhance immune responses, such as vitamin A, vitamin E, vitamin C, and the like, are well known in the art (see, for example, U.S. Pat. Nos. 4,981,844 and 5,230,902 and PCT Publ. No. WO 2004/004483) can be used in the methods described herein.

Similarly, agents and therapies other than immunotherapy or in combination thereof can be used to stimulate an immune response to thereby treat a condition that would benefit therefrom. For example, chemotherapy, radiation, epigenetic modifiers (*e.g.,* histone deacetylase (HDAC) modifiers, methylation modifiers, phosphorylation modifiers, and the like), targeted therapy, and the like are well known in the art.

In still another embodiment according to the disclosure, the term "targeted therapy" refers to administration of agents that selectively interact with a chosen biomolecule to thereby treat cancer. For example, bevacizumab (Avastin^{®}) is a humanized monoclonal antibody that targets vascular endothelial growth factor (see, for example, U.S. Pat. Publ. 2013/0121999, WO 2013/083499, and Presta et al. (1997) Cancer Res. 57:4593-4599) to inhibit angiogenesis accompanying tumor growth. In some cases, targeted therapy can be a form of immunotherapy depending on whether the target regulates immunomodulatory function.

The term "untargeted therapy" refers to administration of agents that do not selectively interact with a chosen biomolecule yet treat cancer. Representative examples of untargeted therapies include, without limitation, chemotherapy, gene therapy, and radiation therapy.

In another embodiment according to the disclosure, hormone therapy is used. Hormonal therapeutic treatments can comprise, for example, hormonal agonists, hormonal antagonists (*e.g.,* flutamide, bicalutamide, tamoxifen, raloxifene, leuprolide acetate (LUPRON), LH-RH antagonists), inhibitors of hormone biosynthesis and processing, and steroids (*e.g.,* dexamethasone, retinoids, deltoids, betamethasone, cortisol, cortisone, prednisone, dehydrotestosterone, glucocorticoids, mineralocorticoids, estrogen, testosterone, progestins), vitamin A derivatives (*e.g.,* all-trans retinoic acid (ATRA)); vitamin D3 analogs; antigestagens (*e.g.,* mifepristone, onapristone), or antiandrogens (*e.g.,* cyproterone acetate).

In still another embodiment according to the disclosure, treatment methods may further use agents that block an activity of costimulatory pathways, such as that of other B lymphocyte antigen like B7-1, B7-2, or B7-3) to further downmodulate immune responses. Two separate agents that downmodulate immune responses can be combined as a single composition or administered separately (simultaneously or sequentially) to more effectively downregulate immune cell mediated immune responses in a subject. Furthermore, a therapeutically active amount of one or more of the subject agents, can be used in conjunction with other downmodulating reagents to influence immune responses. Examples of other immunomodulating reagents include, without limitation, antibodies that block a costimulatory signal, (*e.g.,* against CD28 or ICOS), antibodies that act as agonists of CTLA4, and/or antibodies against other immune cell markers (*e.g.,* against CD40, against CD40 ligand, or against cytokines), fusion proteins (*e.g.*, CTLA4-Fc), and immunosuppressive drugs, (*e.g.,* rapamycin, cyclosporine A or FK506).

Moreover, agents that promote the activity of immune checkpoint proteins are useful.
The term "immune checkpoint protein" refers to a group of molecules on the cell surface of CD4+ and/or CD8+ T cells that fine-tune immune responses by down-modulating or inhibiting an anti-tumor immune response. Immune checkpoint proteins are well-known in the art and include, without limitation, CTLA-4 as described above, as well as PD-1, VISTA, B7-H2, B7-H3, PD-L1, B7-H4, B7-H6, ICOS, HVEM, PD-L2, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, and A2aR (see, for example, WO 2012/177624). Agents useful for promoting immune checkpoint protein levels and activity are well-known in the art.

In yet another embodiment according to the disclosure, any first- or second-line immune disorder treatment can be combined with the methods of the present invention. Representative examples include, but are not limited to, steroidal, mycophenolate mofetil (MMF), and pentostatin (see, for example, Busca et al. (2000) Bone Marrow Transplant 25:1067-1071; Berger et al. (2007) J. Pediatr. Hematol. Oncol. 29:678-687; Jacobsohn et al. (2009) Blood 114:4354-4360).

Also provided herein are compositions comprising one or more nucleic acids comprising or capable of expressing at least 1, 2, 3, 4, 5, 10, 20 or more small nucleic acids or antisense oligonucleotides or derivatives thereof, wherein said small nucleic acids or antisense oligonucleotides or derivatives thereof in a cell specifically hybridize (*e.g.,* bind) under cellular conditions, with cellular nucleic acids (*e.g.,* small non-coding RNAS such as miRNAs, pre-miRNAs, pri-miRNAs, miRNA*, anti-miRNA, a miRNA binding site, a variant and/or functional variant thereof, cellular mRNAs or a fragments thereof). In one embodiment according to the disclosure, expression of the small nucleic acids or antisense oligonucleotides or derivatives thereof in a cell can inhibit expression or biological activity of cellular nucleic acids and/or proteins, *e.g.,* by inhibiting transcription, translation and/or small nucleic acid processing of, for example, one or more biomarkers of the invention, including one or more biomarkers listed in Table 1, or fragment(s) thereof (Zeng et al. (2002) Mol. Cell 9:1327-1333; Tuschl (2002) Nat. Biotechnol. 20:446-448; Brummelkamp et al. (2002) Science 296:550-553; Miyagishi et al. (2002) Nat. Biotechnol. 20:497-500; Paddison et al. (2002) Genes Dev. 16:948-958; Lee et al. (2002) Nat. Biotechnol. 20:500-505; and Paul et al. (2002) Nat. Biotechnol. 20:505-508; U.S. Patents 5,176,996; 5,264,564; and 5,256,775; van der Krol et al. (1988) BioTechniq. 6:958-976; Stein et al. (1988) Cancer Res. 48:2659-2668; Wagner (1994) Nature 372:333; Letsinger et al. (1989) Proc. Natl. Acad. Sci. U.S.A. 86:6553-6556; Lemaitre et al. (1987) Proc. Natl. Acad. Sci. U.S.A. 84:648-652; PCT Publication No. W0 88/09810 and W0 89/10134; Zon (1988) Pharm. Res. 5:539-549)

Small nucleic acids and/or antisense oligonucleotides can also contain a neutral peptide-like backbone. Such molecules are termed peptide nucleic acid (PNA)-oligomers and are described, *e.g.,* in Perry-O'Keefe et al. (1996) Proc. Natl. Acad. Sci. U.S.A. 93:14670; Eglom et al. (1993) Nature 365:566; Gautier et al. (1987) Nucl. Acids Res. 15:6625-6641; Inoue et al. (1987) Nucl. Acids Res. 15:6131-6148; Inoue et al. (1987) FEBS Lett. 215:327-330; Sarin et al. (1988) Proc. Natl. Acad. Sci. U.S.A. 85:7448-7451; Elbashir et al. (2001) Nature 411:494-498; McManus et al. (2002) RNA 8:842; Xia et al. (2002) Nat. Biotechnol. 20:1006; and Brummelkamp et al. (2002) Science 296:550).

Ribozyme molecules designed to catalytically cleave cellular mRNA transcripts can also be used to prevent translation of cellular mRNAs and expression of cellular polypeptides, or both (PCT Publ. WO90/11364; Sarver et al. (1990) Science 247:1222-1225; and U.S. Patent No. 5,093,246; Haseloff and Gerlach (1988) Nature 334:585-591; Zaug et al. (1984) Science 224:574-578; Zaug et al. (1986) Science 231:470-475; Zaug et al. (1986) Nature 324:429-433; WO 88/04300; and Been et al. (1986) Cell 47:207-216).

Nucleic acid molecules to be used in triple helix formation for the inhibition of transcription of cellular genes are preferably single stranded and composed of deoxyribonucleotides. The base composition of these oligonucleotides should promote triple helix formation via Hoogsteen base pairing rules, which generally require sizable stretches of either purines or pyrimidines to be present on one strand of a duplex. Nucleotide sequences may be pyrimidine-based, which will result in TAT and CGC triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, containing a stretch of G residues. These molecules will form a triple helix with a DNA duplex that is rich in GC pairs, in which the majority of the purine residues are located on a single strand of the targeted duplex, resulting in CGC triplets across the three strands in the triplex.

Alternatively, the potential sequences that can be targeted for triple helix formation may be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizable stretch of either purines or pyrimidines to be present on one strand of a duplex.

Small nucleic acids (*e.g.,* miRNAs, pre-miRNAs, pri-miRNAs, miRNA*, anti-miRNA, or a miRNA binding site, or a variant thereof), antisense oligonucleotides, ribozymes, and triple helix molecules of the methods and compositions presented herein may be prepared by any method known in the art for the synthesis of DNA and RNA molecules. These include techniques for chemically synthesizing oligodeoxyribonucleotides and oligoribonucleotides well-known in the art such as for example solid phase phosphoramidite chemical synthesis. Alternatively, RNA molecules may be generated by in vitro and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Moreover, various well-known modifications to nucleic acid molecules may be introduced as a means of increasing intracellular stability and half-life. Possible modifications include but are not limited to the addition of flanking sequences of ribonucleotides or deoxyribonucleotides to the 5' and/or 3' ends of the molecule or the use of phosphorothioate or 2' O-methyl rather than phosphodiesterase linkages within the oligodeoxyribonucleotide backbone. One of skill in the art will readily understand that polypeptides, small nucleic acids, and antisense oligonucleotides can be further linked to another peptide or polypeptide *(e.g.,* a heterologous peptide), *e.g.,* that serves as a means of protein detection. Non-limiting examples of label peptide or polypeptide moieties useful for detection in the invention include, without limitation, suitable enzymes such as horseradish peroxidase, alkaline phosphatase, beta-galactosidase, or acetylcholinesterase; epitope tags, such as FLAG, MYC, HA, or HIS tags; fluorophores such as green fluorescent protein; dyes; radioisotopes; digoxygenin; biotin; antibodies; polymers; as well as others known in the art, for example, in Principles of Fluorescence Spectroscopy, Joseph R. Lakowicz (Editor), Plenum Pub Corp, 2nd edition (July 1999).

The agents can be incorporated into pharmaceutical compositions suitable for administration to a subject. Such compositions typically comprise the antibody, peptide, fusion protein, small molecule, or the like, and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary active compounds can also be incorporated into the compositions.

A pharmaceutical composition is formulated to be compatible with its intended route of administration. Examples of routes of administration include parenteral, *e.g.,* intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the composition should be sterile and should be fluid to the extent that easy syringeability exists. It must be stable under the conditions of manufacture and storage and should be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it is preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressured container or dispenser which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g.,* with conventional suppository bases such as cocoa butter and other glycerides) or retention enemas for rectal delivery.

In one embodiment according to the disclosure, modulatory agents are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations should be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by, and directly dependent on, the unique characteristics of the active compound, the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects can be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC50 (*i.e.,* the concentration of the test compound which achieves a half-maximal inhibition of symptoms) as determined in cell culture. Such information can be used to more accurately determine useful doses in humans. Levels in plasma can be measured, for example, by high performance liquid chromatography.

The above described modulating agents may be administered in the form of expressible nucleic acids which encode said agents. Such nucleic acids and compositions in which they are contained, are also encompassed by the present invention. For instance, the nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see *e.g.,* Chen et al. (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.,* retroviral vectors, the pharmaceutical preparation can include one or more cells which produce the gene delivery system.

The pharmaceutical compositions can be included in a container, pack, or dispenser together with instructions for administration.

### d. Clinical efficacy

Clinical efficacy can be measured by any method known in the art. For example, the response to a therapy, such as administration of engineered T cells with or without additional therapeutic agents, relates to any modulated response of T cell exhaustion and/or the immune disorder, *e.g.,* a tumor, to the therapy, preferably to a change in tumor mass and/or volume after initiation of neoadjuvant or adjuvant chemotherapy.

For example, clinical efficacy for treating immune disorders caused by infectious agents can involve determining reductions in the infectious microbial (e.g., viral, bacterial, fungal, mycoplasm, or parasitic) load in the subject relative to such load in an untreated control. As compared with an equivalent untreated control, such reduction or degree of prevention is at least 5%, 10%, 20%, 40%, 50%, 60%, 80%, 90%, 95%, or 100% as measured by any standard technique. The infectious agent can be completely cleared as detected by any standard method known in the art. Diagnosis and monitoring may involve, for example, detecting the level of microbial load in a biological sample (*e.g.,* tissue biopsy, blood test, or urine test), detecting the level of a biomarker surrogate marker of the infectious agent in a biological sample, detecting symptoms associated with the chronic immune disorder, or detecting immune cells involved in the immune response typical of the chronic immune disorder (*e.g.,* detection of antigen-specific, exhausted CD8+ T cells).

Clinical efficacy against cancers can also be assessed. Tumor response may be assessed in a neoadjuvant or adjuvant situation where the size of a tumor after systemic intervention can be compared to the initial size and dimensions as measured by CT, PET, mammogram, ultrasound or palpation and the cellularity of a tumor can be estimated histologically and compared to the cellularity of a tumor biopsy taken before initiation of treatment. Response may also be assessed by caliper measurement or pathological examination of the tumor after biopsy or surgical resection. Response may be recorded in a quantitative fashion like percentage change in tumor volume or cellularity or using a semiquantitative scoring system such as residual cancer burden (Symmans et al., J. Clin. Oncol. (2007) 25:4414-4422) or Miller-Payne score (Ogston et al., (2003) Breast (Edinburgh, Scotland) 12:320-327) in a qualitative fashion like "pathological complete response" (pCR), "clinical complete remission" (cCR), "clinical partial remission" (cPR), "clinical stable disease" (cSD), "clinical progressive disease" (cPD) or other qualitative criteria. Assessment of tumor response may be performed early after the onset of neoadjuvant or adjuvant therapy, *e.g.,* after a few hours, days, weeks or preferably after a few months. A typical endpoint for response assessment is upon termination of neoadjuvant chemotherapy or upon surgical removal of residual tumor cells and/or the tumor bed.

In some embodiments according to the disclosure, clinical efficacy of the therapeutic treatments described herein may be determined by measuring the clinical benefit rate (CBR). The clinical benefit rate is measured by determining the sum of the percentage of patients who are in complete remission (CR), the number of patients who are in partial remission (PR) and the number of patients having stable disease (SD) at a time point at least 6 months out from the end of therapy. The shorthand for this formula is CBR=CR+PR+SD over 6 months. In some embodiments according to the disclosure, the CBR for a particular anti-immune checkpoint inhibitor therapeutic regimen is at least 25%, 30%, 35%, 40%, 45%, 50%, 55%, 60%, 65%, 70%, 75%, 80%, 85%, or more.

Additional criteria for evaluating the response to therapy are related to "survival," which includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (*e.g.,* time of diagnosis or start of treatment) and end point *(e.g.,* death, recurrence or metastasis). In addition, criteria for efficacy of treatment can be expanded to include response to chemotherapy, probability of survival, probability of metastasis within a given time period, and probability of tumor recurrence.

For example, in order to determine appropriate threshold values, a therapeutic regimen can be administered to a population of subjects and the outcome can be correlated to biomarker measurements that were determined prior to administration of the therapy or any therapy. The outcome measurement may be pathologic response to therapy given in the neoadjuvant setting. Alternatively, outcome measures, such as overall survival and disease-free survival can be monitored over a period of time for subjects following anti-immune checkpoint inhibitor therapy for whom biomarker measurement values are known. In certain embodiments according to the disclosure, the same doses of therapeutic agents are administered to each subject. In related embodiments according to the disclosure, the doses administered are standard doses known in the art for such agents. The period of time for which subjects are monitored can vary. For example, subjects may be monitored for at least 2, 4, 6, 8, 10, 12, 14, 16, 18, 20, 25, 30, 35, 40, 45, 50, 55, or 60 months. Biomarker measurement threshold values that correlate to outcome of a therapy can be determined using methods such as those described in the Examples section.

### VI. Additional methods

As described above regarding therapeutic methods described, the present disclosure also provides prophylactic methods for preventing T cell exhaustion and/or an immune disorder in a subject by administereing engineered T cells either alone or in combination with additional therapeutic agents. Subjects at risk for unwanted T cell exhaustion and/or an unwanted immune disorder can be identified, for example, by any or a combination of diagnostic or prognostic assays known in the art. Administration of a prophylactic agent(s) can occur prior to the manifestation of symptoms associated with an undesired immune response. The appropriate agent(s) used for treatment (e.g. antibodies, peptides, fusion proteins, or small molecules) can be determined based on clinical indications and can be identified using diagnostic assays well-known in the art, as well as those described herein.

The present disclosure also provides diagnostic methods described further below.

In any method described herein, such as a diagnostic method, prognostic method, therapeutic method, or combination thereof, all steps of the method can be performed by a single actor or, alternatively, by more than one actor. For example, diagnosis can be performed directly by the actor providing therapeutic treatment. Alternatively, a person providing a therapeutic agent can request that a diagnostic assay be performed. The diagnostician and/or the therapeutic interventionist can interpret the diagnostic assay results to determine a therapeutic strategy. Similarly, such alternative processes can apply to other assays, such as prognostic assays.

The present disclosure is not restricted to *in vivo* treatment in a subject. For example, T cells can be isolated from a subject for *ex vivo* procedures (*e.g.,* immune cell processing out side of the body and re-administration of the cells back into the body). Similarly, T cells and engineered forms thereof can be cultured *in vitro.* Expression and/or activity of biomarkers of the present invention, such as PD-1 enhancers, can be selectively modulated in T cells *in vitro, ex vivo,* or *in vivo* and the resulting effects on cellular processes (*e.g.,* cell proliferation, differentiation, death, etc.) or immune responses (*e.g.,* cytotoxicity, cytokine production, etc.) can be analyzed *in vitro, ex vivo,* or *in vivo.* In this manner, engineered T cells can be used *in vitro* to assay T cell exhaustion biology either alone or in combination with other cell types, such as regulatory T cells, and/or manipulations, such as treatment with test compounds. Moreover, such manipulations can be performed in combination. For example, T cells can be isolated from a subject, subjected to recombinant genetic manipulation, re-administered to a subject, and then provided with systemic administration of a therapeutic *in vivo.*

In one aspect, the present disclosure pertains to the field of predictive medicine in which diagnostic assays, prognostic assays, and monitoring clinical trials are used for prognostic (predictive) purposes to thereby treat an individual prophylactically. Accordingly, one aspect of the present invention relates to diagnostic assays for determining the amount and/or activity level of a genomic region selectively present within the genome of an exhausted CD8+ T cell, either alone or in connection with expression of at least one gene whose expression the genomic region regulates, in the context of a biological sample (e.g., blood, serum, cells, or tissue) to thereby identify the presence of exhausted CD8+ T cells and/or the status of an individual afflicted with an immune disorder. Such assays can be used for prognostic or predictive purpose to thereby prophylactically treat an individual prior to the onset or after recurrence of a disorder characterized by or associated with biomarker polypeptide, nucleic acid expression or activity. The skilled artisan will appreciate that any method can use one or more (e.g., combinations) of biomarkers listed in Table 1.

Another aspect of the present disclosure pertains to monitoring the influence of agents (*e.g.,* drugs, compounds, and small nucleic acid-based molecules) on the expression or activity of a biomarker listed in Table 1. For example, in one aspect, a method for identifying an agent which modulates T cell exhaustion and/or an immune response entails determining the ability of the candidate agent to promote or inhibit T cell function of the engineered T cells is provided. In one embodiment according to the disclosure, the assay assesses the physical and/or functional interaction of the engineered T cells with other cell types, such Tregs, Teffs, cell-based vaccines, and the like. In another embodiment according to the disclosure, the assay assesses the amount of gene expression modulation resulting from the genetic modification. The assay can also combine analyses of 1, 2, 3, 4, 5, 6, 7, 8, 9, 10, or more genes whose expression is modulated by the genetic modulation.

The assays are cell-based assays and may comprise, for example, contacting T cells modified as described herein with a test agent and determining the ability of the test agent to modulate (e.g. stimulate or inhibit) the physical and/or functional interaction between immune-related cells of interest. Determining the ability of the polypeptides to bind to, or interact with, each other may be accomplished, *e.g.,* by measuring direct binding or by measuring a parameter of immune cell response.

For example, in a direct binding assay, polypeptides may be coupled with a radioisotope or enzymatic label such that binding of immune-related biomolecules may be determined by detecting the labeled protein in a complex. For example, the polypeptides may be labeled with ¹²⁵I, ³⁵S, ¹⁴C, or ³H, either directly or indirectly, and the radioisotope detected by direct counting of radioemission or by scintillation counting. Alternatively, the polypeptides may be enzymatically labeled with, for example, horseradish peroxidase, alkaline phosphatase, or luciferase, and the enzymatic label detected by determination of conversion of an appropriate substrate to product.

It is also disclosed herein to determine the ability of a compound to modulate the interaction between immune-related biomolecules or cells of interest without the labeling of any of the interactants. For example, a microphysiometer may be used to detect the interaction of immune-related biomolecule polypeptides without the labeling of either polypeptide (McConnell et al. (1992) Science 257:1906-1912). As used herein, a "microphysiometer" (*e.g.,* Cytosensor) is an analytical instrument that measures the rate at which a cell acidifies its environment using a light-addressable potentiometric sensor (LAPS). Changes in this acidification rate may be used as an indicator of the interaction between compound and receptor.

Preferably, determining the ability of the test agents (*e.g.* nucleic acids, polypeptides, antibodies, fusion proteins, peptides, or small molecules) to antagonize or agonize the physical and/or functional interaction between a given set of immune-related biomolecules or cells may be accomplished by determining the activity of one or more members of a set of immune-related biomolecule polypeptides. For example, the activity of polypeptides may be determined by detecting induction of a cellular second messenger (*e.g.,* PD-1 downstream signaling activity), detecting catalytic/enzymatic activity of an appropriate substrate, detecting the induction of a reporter gene (comprising a target-responsive regulatory element operatively linked to a nucleic acid encoding a detectable marker, *e.g.,* chloramphenicol acetyl transferase), or detecting a cellular response regulated by the polypeptides, such as various autoimmune, allergic (*e.g.,* asthma, atopic dermatitis, allergic conjunctivitis, pollen allergy, food allergy, etc.), vaccination, immunotolerance, cancer immunotherapy, immune exhaustion, immunological memory, or immunological epitope responses. Determining the ability of the test agent to bind to or interact with said polypeptide may be accomplished, for example, by measuring the ability of a compound to modulate immune cell costimulation or inhibition in a proliferation assay, or by interfering with the ability of said polypeptide to bind to antibodies that recognize a portion thereof.

Test agents that modulate immune responses may be identified by their ability to modulate immune cell proliferation, and/or effector function, or to modulate anergy, clonal deletion, and/or exhaustion when added to an assay. For example, cells may be cultured in the presence of an agent that stimulates signal transduction via an activating receptor. A number of recognized readouts of cell activation may be employed to measure cell proliferation or effector function (*e.g.,* antibody production, cytokine production, phagocytosis) in the presence of the activating agent. The ability of a test agent to block this activation may be readily determined by measuring the ability of the agent to effect a decrease in proliferation or effector function being measured, using techniques known in the art.

For example, agents disclosed herein may be tested for the ability to inhibit or enhance co-stimulation and/or co-inhibition in a T cell assay, such as described in Freeman et al. (2000) J. Exp. Med. 192:1027 and Latchman et al. (2001) Nat. Immunol. 2:261. Immune cells of interest may be activated, such as with anti-CD3 antibody, and presented with modified T cells as described herein. Proliferation of T cells may be measured by ³H thymidine incorporation. An assay may be performed with or without CD28 costimulation in the assay.

Alternatively, agents disclosed herein may be tested for the ability to modulate cellular production of cytokines which are produced by or whose production is enhanced or inhibited in immune cells in response to immune response modulation. For example, immune cells of interest may be suboptimally stimulated *in vitro* with a primary activation signal. For example, engineered T cells may be stimulated with phorbol ester, anti-CD3 antibody or preferably antigen in association with an MHC class II molecule, and given a costimulatory signal, *e.g.,* by a stimulatory form of B7 family antigen, for instance by a cell transfected with nucleic acid encoding a B7 polypeptide and expressing the peptide on its surface or by a soluble, stimulatory form of the peptide. Known cytokines released into the media may be identified by ELISA or by the ability of an antibody which blocks the cytokine to inhibit immune cell proliferation or proliferation of other cell types that is induced by the cytokine. For example, an IL-4 ELISA kit is available from Genzyme (Cambridge MA), as is an IL-7 blocking antibody. The effects of Tregs modified as described herein and added to the assay can be assessed. The effect of stimulating or blocking the interaction of immune-related biomolecules on the cytokine profile may then be determined.

In one or more embodiments of the above described assay methods according to the disclosure, it may be desirable to immobilize either polypeptides to facilitate separation of complexed from uncomplexed forms of one or both of the proteins, as well as to accommodate automation of the assay. Binding of a test compound to a polypeptide, may be accomplished in any vessel suitable for containing the reactants. Examples of such vessels include microtiter plates, test tubes, and micro-centrifuge tubes. In one embodiment according to the disclosure, a fusion protein may be provided which adds a domain that allows one or both of the proteins to be bound to a matrix. For example, glutathione-S-transferase/immune-related polypeptide fusion proteins, or glutathione-S-transferase/target fusion proteins, may be adsorbed onto glutathione sepharose beads (Sigma Chemical, St. Louis, MO) or glutathione derivatized microtiter plates, which are then combined with the test compound, and the mixture incubated under conditions conducive to complex formation (*e.g.,* at physiological conditions for salt and pH). Following incubation, the beads or microtiter plate wells are washed to remove any unbound components, the matrix immobilized in the case of beads, complex determined either directly or indirectly, for example, as described above. Alternatively, the complexes may be dissociated from the matrix, and the level of polypeptide binding or activity determined using standard techniques.

In an alternative embodiment according to the disclosure, determining the ability of the test compound to modulate the activity of an immune-related polypeptide of interest may be accomplished as described above for cell-based assays, such as by determining the ability of the test compound to modulate the activity of a polypeptide that functions downstream of the polypeptide. For example, levels of second messengers may be determined, the activity of the interactor polypeptide on an appropriate target may be determined, or the binding of the interactor to an appropriate target may be determined as previously described.

In some embodiments according to the disclosure, determination as to modulation of an immune-related indication of interest may be made in comparison to a control, which term is described above, and determination of an overexpression, overactivity, underexpression, underactivity, etc, which terms are also described above.

In another aspect according to the disclosure, monitoring the influence of agents on the expression or activity of one or more biomarkers of the present invention, including one or more biomarkers listed in Table 1, the Figures, and the Examples, or a fragment thereof (*e.g.,* the modulation of T cell exhaustion and/or an immune disorder) can be applied not only in basic drug screening, but also in clinical trials. For example, the effectiveness of an agent determined by screening assay as described herein to modulate gene expression, including one or more biomarkers listed in Table 1, the Figures, and the Examples, or a fragment thereof, can be monitored in clinical trials of subjects exhibiting modulated expression of a gene of interest and/or functional consequences therefrom (e.g., the modulation of T cell exhaustion and/or an immune disorder). In such clinical trials, the direct expression and/or activity of the biomarker or the indirect result of modulated T cell exhaustion and/or immune disorder condition can be used as a "read out" or marker of the phenotype of a particular cell.

In some embodiments, the present disclosure provides a method for monitoring the effectiveness of treatment of a subject with an agent (*e.g.,* engineered T cell, an agonist, antagonist, peptidomimetic, polypeptide, peptide, nucleic acid, small molecule, or other drug candidate identified by the screening assays described herein) including the steps of (i) obtaining a pre-administration sample from a subject prior to administration of the agent; (ii) detecting the level of expression and/or activity of one or more biomarkers of the present invention, including one or more biomarkers listed in Table 1, the Figures, and the Examples, or fragments thereof in the preadministration sample; (iii) obtaining one or more post-administration samples from the subject; (iv) detecting the level of expression or activity of the biomarker in the post-administration samples; (v) comparing the level of expression or activity of the biomarker or fragments thereof in the pre-administration sample with the that of the biomarker in the post administration sample or samples; and (vi) altering the administration of the agent to the subject accordingly. For example, increased administration of the agent may be desirable to increase the expression or activity of one or more biomarkers to higher levels than detected (*e.g.,* to increase the effectiveness of the agent). Alternatively, decreased administration of the agent may be desirable to decrease expression or activity of the biomarker to lower levels than detected (*e.g.,* to decrease the effectiveness of the agent). According to such an embodiment according to the disclosure, biomarker expression or activity may be used as an indicator of the effectiveness of an agent, even in the absence of an observable phenotypic response.

The skilled artisan will also appreciate that, in certain embodiments according to the disclosure, the methods of the present invention implement a computer program and computer system. For example, a computer program can be used to perform the algorithms described herein. A computer system can also store and manipulate data generated by the methods of the present invention which comprises a plurality of biomarker signal changes/profiles which can be used by a computer system in implementing the methods of this invention. In certain embodiments according to the disclosure, a computer system receives biomarker expression data; (ii) stores the data; and (iii) compares the data in any number of ways described herein (*e.g.,* analysis relative to appropriate controls) to determine the state of informative biomarkers from cancerous or pre-cancerous tissue. In other embodiments according to the disclosure, a computer system (i) compares the determined expression biomarker level to a threshold value; and (ii) outputs an indication of whether said biomarker level is significantly modulated (*e.g.,* above or below) the threshold value, or a phenotype based on said indication.

In certain embodiments according to the disclosure, such computer systems are also considered part of the present invention. Numerous types of computer systems can be used to implement the analytic methods of this invention according to knowledge possessed by a skilled artisan in the bioinformatics and/or computer arts. Several software components can be loaded into memory during operation of such a computer system. The software components can comprise both software components that are standard in the art and components that are special to the present invention (*e.g*., dCHIP software described in Lin et al. (2004) Bioinformatics 20, 1233-1240; radial basis machine learning algorithms (RBM) known in the art).

The methods of the present disclosure can also be programmed or modeled in mathematical software packages that allow symbolic entry of equations and high-level specification of processing, including specific algorithms to be used, thereby freeing a user of the need to procedurally program individual equations and algorithms. Such packages include, *e.g.,* Matlab from Mathworks (Natick, Mass.), Mathematica from Wolfram Research (Champaign, Ill.) or S-Plus from MathSoft (Seattle, Wash.).

In certain embodiments according to the disclosure, the computer comprises a database for storage of biomarker data. Such stored profiles can be accessed and used to perform comparisons of interest at a later point in time. For example, biomarker expression profiles of a sample derived from the non-cancerous tissue of a subject and/or profiles generated from population-based distributions of informative loci of interest in relevant populations of the same species can be stored and later compared to that of a sample derived from the cancerous tissue of the subject or tissue suspected of being cancerous of the subject.

In addition to the exemplary program structures and computer systems described herein, other, alternative program structures and computer systems will be readily apparent to the skilled artisan. Such alternative systems, which do not depart from the above described computer system and programs structures either in spirit or in scope, are therefore intended to be comprehended within the accompanying claims.

### VII. Kits

Also disclosed herein are kits. For example, the kit can comprise engineered T cells either alone or in combination with other immunomodulatory agents, packaged in a suitable container and can further comprise instructions for using such reagents. The kit may also contain other components, such as administration tools packaged in a separate container.

Other embodiments of the present disclosure are described in the following Examples. The present invention is further illustrated by the following examples which should not be construed as further limiting.

### EXAMPLES

### Example 1: Materials and Methods for Examples 2-6

### a. Mice

Wild type C57/BL/6N (CD45.2+) and congenic B6.SJL-Ptprca Pepcb/BoyJ (CD45.1+) mice were obtained from the US National Cancer Institute. C57BL/6N P14 mice (LCMV-specific T cell receptor transgenic) were bred in-house. Male mice were used at between 6 and 10 weeks of age. All animal experiments were done in accordance with the Animal Care and Use Guidelines for the University of Pennsylvania.

### b. Infection and isolation of lymphocytes

LCMV strains were produced and titered as described in Lara-Astiaso et al. (2014) Science 345:943-949; Gupta et al. (2015) PLoS Pathogens 11:e1005177; Kurachi et al. (2014) Nat. Immunol. 15:373-383; and Odorizzi et al. (2015) J. Exp. Med. 212:1125-1137. Two cohorts of mice were infected by intraperitoneal injection of LCMV Armstrong (2 × 10⁵ plaque-forming units) or intravenous injection of LCMV Clone 13 (4 × 10⁶ plaque-forming units). All donor P14 cells were prepared from naïve spleen using CD8 negative selection (Miltenyi Biotec). To generate effector, memory, and exhausted CD8⁺ T cells, approximately 1-2 × 10³ P14 cells were transferred into recipient mice followed by LCMV infection one day later. Target effector/memory/exhausted P14 cells were harvested only from recipient spleens at d8 or d27 post-infection (p.i.). After enrichment using CD45.2 FITC Ab and anti-FITC microbes (Miltenyi), cells were stained and sorted by Aria^{™} II (BD). Naive and effector/memory/exhausted CD8⁺ T (P14) cells were sorted by gating on CD8⁺ TCRVa2⁺ CD62L⁺ CD44^{lo} and CD8⁺ TCRVa2⁺ CD45.2⁺ CD45.1⁻ population, respectively. The following fluorochrome-conjugated antibodies from BioLegend were used for flow-cytometry: anti-TCR Vα2 (B20.1), anti-CD8α (53-6.7), anti-CD44 (IM7), anti-CD45.1 (A20), anti-CD45.2 (104), and anti-CD62L (MEL14).

### c. ATAC-seq

Approximately 40-50,000 cells per biological replicate (Naive, Acute d8, Acute d27, Chronic d8, Chronic d27 and EL4) were washed once in cold PBS and lysed in 50 µL cold lysis buffer (10 mM Tris-HCl, pH 7.4, 10 mM NaCl, 3 mM MgCl₂, 0.1% IGEPAL^{®} CA-630). Lysed nuclei were incubated in Tn5 transposition reaction mix as described in Buenrostro et al. (2013) Nature Methods 10:1213-1218 and Long et al. (2015) Nat. Med. 21:581-590 and purified using MinElute^{®} Reaction Cleanup kit (Qiagen). ATAC-seq fragments from one set of replicates for Acute d8, Acute d27, Chronic d8 and Chronic d27, as well as both biological replicates for Naive cells and EL4 cells, were size-selected for fragments between 115 and 600 base pairs (bp) using Pippin Prep^{™} 2% Agarose Gel Cassettes and the Pippin Prep^{™} DNA Size Selection System (Sage Science). Post size-selection, ATAC libraries were amplified and Nextera^{™} sequencing primers ligated using the polymerase chain reaction (PCR). Finally, PCR primers were removed using Agencourt^{®} AMPure^{®} XP bead cleanup (Beckman Coulter/Agencourt) and library quality was verified using a TapeStation machine. High quality 'multiplexed' DNA libraries were sequenced using the Illumina HiSeq^{™} 2000 sequencing system.

### d. Quality control of ATAC-Seq data and alignment to the genome

The FASTQC pipeline (Babraham Bioinformatics) was used on the reads, which were aligned to the reference genome (mm9) with bowtie software version 1.1.1 (Langmead et al. (2009) Genome Biol. 10:R25). Only reads that mapped to a unique position in the genome were retained ["-m 1"]. Duplicate reads were marked in the bam files using PICARD software and were checked for contamination of primer sequences.

### e. Identification of peaks in ATAC-Seq data

After alignment of the reads to the reference genome, reads aligned to the positive strand were moved +4 base pairs (bp), and reads aligning the negative strand were moved - 5 bp (Buenrostro et al. (2013) Nature Methods 10:1213-1218; Long et al. (2015) Nat. Med. 21:581-590). For each of the five replicate pairs, the two replicates were merged by using samtools software, and the peaks were called using macs2 software version 2.1.0, wherein the FDR was set to 0.001 and the "nomodel" and default mouse genome size options were used. In order to generate the universe of peaks, the union of all of these peaks while merging overlapping regions was taken.

### f. Identification of differential activity in peaks

The shifted ATAC-Seq cut sites were extracted from the data and the number that fell into each peak region was counted. DESeq2 software was then used to identify the differential abundance of cut sites between the peaks. All pairwise comparisons of the five conditions were performed and those with a FDR below 0.05 were considered to be differential.

### g. Enrichment of genomic features in ATAC-Seq peaks

For each sample, the set of peaks calculated by MACS2 software for enrichment of genomic features relative to their coverage in the mouse genome was investigated. For each genomic feature, the fold enrichment was calculated as [Overlap of Genomic Feature with ATAC-Seq Peak Regions / Total Size of Peak Regions] / [Overlap of Genomic Feature with Mouse Genome / Effective Size of Mouse Genome], with all sizes expressed in base pairs.

The effective size of the mouse genome was set to be 2,716,965,481 bp. P-values were calculated using a binomial test, where n= Number of Peaks, x = Number of Peaks Overlapped by Annotation, p = (Overlap with Mouse Genome / Effective Size of the Mouse Genome).

The genomic features used included: (i) regulatory features annotations from the Ensemble database (Flicek et al. (2011) Nucl. Acids Res. 39:6), (ii) regulatory features found by the ORegAnno database (Griffith et al. (2008) Nucl. Acids Res. 36:13), (iii) conserved regions annotated by the multiz30way algorithm, here considering regions with multiz30way score > 0.7, (iv) repeat regions annotated by RepeatMasker (available on the World Wide Web at repeatmasker.org), (v) putative promoter regions - taking 10 kb upstream and 1 kb downstream of transcripts annotated in RefSeq (Pruitt et al. (2007) Nucl. Acids Res. 35:5), (vi) gene body annotations in RefSeq; (vii) 3' proximal regions (taking 1kb upstream and 5kb upstream to 3' end); (viii) regions enriched in binding of BATF, IRF4, c-Jun, JunD, and JunB, along with histone marks, H3K4me1, H3K4me3, H3K27ac, H3K36me3, and H3K27me3 (polycomb-repression), and additional annotations using Poised Enhancer, Active Enhancer, Bivalent Promoter, Developmental Enhancer, and Init software. Promoters in CD8+ T cells was used (Kurachi et al. (2014) Nat. Immunol. 15:373-383 and Lara-Astiaso et al. (2014) Science 345:943-949). Also, the peaks obtained in this study for two replicates of EL4 cells were added.

### h. Cluster and gene ontology analysis

K-means clustering was applied using GENE-E (Broad Institute) software to differential ChAR signal intensity across all five cell states. The optimum number of clusters was determined using the gap statistic in MATLAB. Association of ChARs to neighboring genes and enrichment of Gene Ontology terms within modules was determined using GREAT software with default settings (available on the World Wide Web at bejerano.stanford.edu/great/public/html/). All significant GO terms are enumerated in Table 1, while selected GO terms are presented as a heat map in Figure 3D.

### i. CRISPR design

sgRNA-specifying oligo sequences were chosen to maximize on-target efficiency based on publicly available tools available on the World Wide Web at broadinstitute.org/rnai/public/analysis-tools/sgma-design-v1. Single-stranded sgRNA oligos (Integrated DNA Technologies) were annealed and ligated into PXPR003 as previously described in Shalem et al. (2014) Science 343:84-87 and Gjoneska et al. (2015) Nature 518:365-369. sgRNA plasmids were introduced into cell lines using lentiviral packaging or nucleofection. For lentivirus production, 293T cells were seeded in DMEM with 10% (vol/vol) FBS. Cells were transfected with sgRNA plasmids and the packaging plasmids Δ8.9 and VSV-g using TurboFect^{™} (ThermoFisher). Viral supernatants were collected 72 hours later. For nucleofection, EL4 cells were mixed with 100 µl of Nucleofector^{™} solution and 2 µg of sgRNA plasmid. Cells were then transfected using Nucleofector^{™} Kit L (Lonza) and the C-09 program on the Amaxa Nucleofector^{™} I machine.

### j. Cell culture

EL4 cells (ATCC) were cultured in RPMI with 10% (vol/vol) FBS, HEPES, penicillin/streptomycin and β-mercaptoethanol (R10 media). Cas9-expressing cell lines were produced using PLX304 plasmid lentivirally packaged as described above. Cas9-expressing cells were then transduced or transfected with sgRNA plasmids, selected for plasmid presence using puromycin, and used for all subsequent experiments 3-5 days post-selection. Single cell clones of bulk EL4s were made using limiting dilution.

### k. Reporter assays

DNA sequences corresponding to the 781 bp core of the -23.8kb PD-1 enhancer (chrl:113 95971119 - 95971900, mm9), and the CMV promoter were cloned upstream of a TATA box minimal promoter driving GFP expression within a lentiviral construct. Plasmids were lentivirally packaged as described above. Viral supernatant was concentrated 100x by ultracentrifugation for 2 hours at 20,000 × g.

CD8⁺ T cells were isolated from naive spleens using the CD8 negative selection MACS^{®} kit (Miltenyi Biotec) and cultured in R10 medium. Naive cells were stimulated for 24 hrs with plate-bound anti-CD3 (4 µg/ml; 2C11; BD Pharmingen) and anti-CD28 (4 µg/ml; 37.51; BD Pharmingen) in the presence of recombinant human IL-2 (100 U/ml, R&D Systems) for *in vitro* activation prior to transduction. *In vitro* activated T cells and EL4 cells were transduced with concentrated virus by incubation with polybrene (5 ug/ml) and spin infection (2,000 RPM for 45 min at 37°C). GFP expression was measured by flow cytometry 48-72 hours post-transduction.

### 1. Enhancer deletion

The region -23.8kb from the *Pdcd1* TSS was deleted using a pair of sgRNA targeting sequences flanking the region. EL4 cells transfected with -23.8kb-targeting sgRNAs or negative control sgRNAs (Figures 8A and 9A) were sorted on the basis of differing levels of PD-1 expression and genomic PCR was used to detect deletion in sorted EL4s. Genomic DNA was isolated from bulk and clonal cell lines using 50 ul QuickExtract^{™} DNA solution and incubated according to manufacturer instructions. PCR was performed on genomic DNA from each sample using the deletion screening primer pairs (Figures 8B and 9B), and run on a 1% agarose gel with EtBr. For clonal lines, biallelic deletion clones were defined as having PCR amplification of only the deletion band, while non-deleter clones amplified only the WT band. Deletion was verified by submitting the PCR amplification products for Sanger sequencing.

### m. RT-qPCR

Clonal cells lines, as well as control sgRNA receiving bulk populations, were lysed in RLT and RNA was extracted using the RNeasy^{®} Plus Mini kit (Qiagen). cDNA was generated using ImProm-II^{™} Reverse Transcriptase (Promega) and analyzed on a ViiA^{™} 7 Quantitative PCR instrument using TaqMan^{®} probes.

### n. In situ saturation mutagenesis

All possible tiling sgRNAs as constrained by Cas9-NGG protospacer adjacent motif presence within all nine PD-1 enhancers and all PD-1 exons were designed using publicly available online tools (available on the World Wide Web at portals.broadinstitute.org/gpp/public/analysis-tools/sgma-design). Non-targeting sgRNAs were designed by the Genomics Perturbation Platform at the Broad Institute. All sgRNA oligos were synthesized and cloned into the lentiGuide-Puro vector backbone as previously described in Lara-Astiaso et al. (2014) Science 345:943-949 and Raimondi et al. (2006) J. Immunol. 176:2808-2816. Plasmid libraries were lentivirally packaged as described above, and Cas9-expressing EL4s were transduced by incubation with polybrene (5ug/ml) and spin infection (2,000 RPM for 45 min at 37°C). Control transductions were done to ensure 1,000x library coverage with a multiplicity of infection of approximately 0.3. Transduced cells were selected with puromycin for 72 hours, and a sample was taken post-selection as the starting d0 representation of the library. Cells were cultured throughout the experiment to ensure 1,000x coverage of the library at all times.

Library-transduced cells were stained with anti-PD-1 fluorescent antibodies and sorted into PD-1 high and PD-low populations (Figure 7C). Replicate sorts were done between d20-d50 post-selection, and genomic DNA was isolated from sorted populations using the DNeasy^{®} Blood and Tissue Kit (Qiagen). Bulk cells from d0 post-selection, input plasmid pools, as well as sorted populations were sequenced as previously described in Lara-Astiaso et al. (2014) Science 345:943-949 and Raimondi et al. (2006) J. Immunol. 176:2808-2816. SgRNA abundance was quantified within each sample by normalizing reads to library sequencing depth. SgRNA enrichment in PD-1 high vs. PD-1 low populations were calculated as follows: 1) sgRNA abundance in each sample was log-transformed; 2) sgRNA abundance at d0 was subtracted from its abundance in each sample to account for initial variation in sgRNA representation; 3) any sgRNA that was not detected in at least one sorted sample was excluded from further analysis as a cellular dropout; 4) for each sorted sample (high and low analyzed separately), the abundance of enhancer- and exon-targeting sgRNAs were normalized calculating z-scores relative to the distribution of the control guides; 5) the normalized score for each sgRNA in the PD-1 low compartment was subtracted from the score in the PD-high compartment, to generate a normalized enrichment score for each sgRNA for each replicate; and 6) the enrichment score for each sgRNA was averaged across replicates. Enhancer- and exon-targeting sgRNA cleavage sites were mapped to the mouse genome (mm9), while control non-targeting sgRNAs were pseudomapped with 5 bp spacing.

### o. Validation of individual sgRNAs

Thirty-two (32) sgRNAs that were enriched in the PD-1 low fraction within the - 23.8 kb enhancer were chosen for individual validation. Oligos representing each sgRNA (IDT) were cloned individually into Cas9 and sgRNA dual delivery construct. Plasmids were then nucleofected into EL4 cells as previously described to produce 32 isogenic cell lines. Each cell line was selected 18-36 hours post-nucleofection in puromycin, and cultured in R10 medium. Cells were stained for PD-1 expression or the corresponding isotype control. Geometric mean fluorescence intensity (MFI) of PD-1 expression in each cell line was normalized to the MFI of staining with isotype control.

### p. Identifying motifs in genome

The PWM databases from Jolma 2013 (Jolma et al. (2013) Cell 152:327-339), Chen 2008 (Chen et al. (2008) Cell 133:1106-1117), UniPROBE (mouse) (Hume et al. (2015) Nucl. Acids Res. 43:22), and JASPAR 2014 (core vertebrates) (Mathelier et al. (2014) Nucl. Acids Res. 42:7) available in the list of databases on the World Wide Web at meme-suite.org/db/motifs, were used. Motifs were then called in version 9 of the mouse genome using FIMO (Grant et al. (2011) Bioinformatics 27:1017-1018) and a threshold of 1×10⁻⁵. A total of 1,446 motifs was initially started out with and any that had zero hits, or more than 1.5 million, were removed. Next, evolutionary conservation and distance to TSS were added for each motif. The mean evolutionary conservation found in each motif was taken and bedtools software (Quinlan (2010) Bioinformatics 26:841-842) "closest" function was used to find the nearest TSS for each motif. A file of TSS was created from the UCSC genome browser.

### q. Inferring transcription factor (TF) footprints using centipede software

The program, Centipede, was used to infer the posterior probability of transcription factor (TF) binding at each motif instance. The evolutionary conservation score, distance to TSS, and PWM score for each motif, along with the number of ATAC-Seq cuts found in every position in a 220 bp window surrounding the motif, were passed on both strands separately. In order to evaluate the accuracy of the models, a separate instance of Centipede was run without conservation, posterior probability on evolutionary conservation, distance to TSS, and PWM score was regressed, and those that did not have a strong, positive association with evolutionary conservation in at least one sample (*P* < 0.05 for one-sided Z value test of coefficient) were removed.

### r. Identifying association of TF with condition

In order to test whether a given TF was associated with a condition, hypergeometric tests on all pairwise combinations of conditions were conducted. A TF was marked as differentially bound in a given condition if the TF was found more often than expected by chance in peaks that were active in that condition versus the other condition.

### s. Ranking of TFs

The normalized mRNA expression of each TF was obtained from previously published datasets (Doering et al. (2012) Immunity 37:1130-1144; Buenrostro et al. (2013) Nat. Methods 10:1213-1218), and the absolute value of log fold change between Acute and Chronic infection was calculated at day 7 (d7) and day 30 (d30). TFs were then associated with a hypergeometric *P*-value quantifying differential binding at d8 and d27 (as above). Each TF was then ranked from most to least differential for these four annotations, representing changes in gene expression and TF binding at d7-8 and d27-30. The final ranking for each TF was determined as the average of the four individual scores.

### t. Analysis of TF binding to regions

Binding of a transcription factor to a region was inferred from Centipede using a posterior probability cutoff of 0.90 or greater. The binding genome-wide was inferred for each of 106 transcription factor motifs, which were determined by their ranking (top 100, as above; Table 1) and known relevance in CD8⁺ T cell biology (JUND, RUNX3, JUNB, STAT5A-STAT5B, FOXP1, and TCF7). Those 106 TFs were further filtered to 50 based on their differential binding and known role in CD8⁺ T cell transcriptional regulation. The binding at each TF motif instance of the 50 TFs in the acute and chronic state was then analyzed. Each binding event was classified as being present in acute only, chronic only, or both. This comparison between motif binding in acute or chronic infection was done at both d8 and d27. Thus, at d8 and d27, calculation of the percentage of binding events per TF motif that was unique to chronic infection, acute infection or shared, was determined. This partiality of TF binding in either acute or chronic infection only, as opposed to shared binding, was quantified as the percentage of all unique binding events seen in chronic infection at each time-point. Furthermore, each region containing at least one bound motif was associated with a target gene using GREAT software. All target genes could then be defined as being upregulated, downregulated or unchanged by mRNA expression between acute and chronic infection at d7 and d30 (Doering et al. (2012) Immunity 37:1130-1144). Thus, each binding event between a TF motif and a near target gene (defined using a more stringent probability cutoff of > 0.95) was classified into those that targeted a gene upregulated in acute infection, upregulated in chronic infection, or unchanged (Table 1).

### u. Subjects

Four HIV-infected participants in the Seattle Natural Progression cohort were selected for this study (Sunshine et al. (2014) J. Virol. 88: 1354-1365). Progressors were defined as having median viral loads >10,000 RNA copies/ml in the last year. All subjects were studied in chronic infection and were antiretroviral therapy naïve. Approximately 40-60 million PBMCs were obtained from each subject. The relevant institutional review boards approved all human subject protocols, and all subjects provided written informed consent before enrollment. A baseline sample was obtained for a single HCV-infected patient enrolled on a trial to evaluate the effect of successful antiviral therapy on innate and adaptive immune responses for genotype 1a hepatitis C virus infection (NCT02476617). The HCV subject was chronically infected with an HCV viral load of 828,000 IU/ml. Based on the expressed HLA class I alleles, virus-specific CD8 T cell responses were screened using HLA class I multimers. Responses targeting HCV A*02:01 C63B CINGVCWTV, HCV A*24:01 174D VIAPAVQTNW, and influenza A*02:01 MP GILGFVFTL, were identified. Multimer-binding CD8⁺ T cells were isolated by FACS from PBMCs obtained through leukapheresis. The patient had consented to this institutional review board-approved study.

PBMCs were obtained via density centrifugation from a normal volunteer and screened for a CMV tetramer⁺ T cell response. The relevant institutional review board approved the human subject protocol, and all subjects provided written informed consent before enrollment.

### v. Isolation of human lymphocyte populations for ATAC-seq

PBMCs were thawed rapidly in warm RPMI supplemented in 10% FBS. CD8⁺ T cells were enriched using MACS^{®} CD8 negative selection kit (Miltenyi Biotec). After enrichment, cells were stained and sorted on a FACSAria^{™} cell sorter (BD Biosciences) (see Figure 11A for the cell sorting strategy). The following fluorochrome-conjugated antibodies from Biolegend were used for flow cytometry: anti-CD45RA (HI100), anti-CCR7 (G043H7), anti-2B4 (C1.7), anti-CD3 (OKT3), anti-275 CD39 (A1), anti-PD-1 (EH12.2H7), and anti-CD8a (SK1). The following fluorochrome conjugated multimers were used for flow-cytometry: HIV A*03:01 RLRPGGKKK, HIV A*02:01 SLYNTVATL, HIV B*08:01 GEIYKRWII, CMV A*02:01 pp65-NLV NLVPMVATV, HCV A*02:01 C63B CINGVCWTV, HCV A*24:01 174D VIAPAVQTNW, and influenza A*02:01 MP GILGFVFTL. A maximum of 70,000 cells were sorted PBS supplemented with 10% FBS and ATAC-seq libraries were generated as previously described. Due to limiting starting material, ATAC libraries were not size-selected prior to multiplexed sequencing on the Illumina HiSeq2000 sequencer. Post-sequencing quality control, alignment of human ATAC reads to the human genome (hg19), MACS2 peak calling, and DESeq2 peak normalization was done as previously described.

### w. Mapping peaks to human genome and SNP analysis

Orthologous mouse ChARs (mm10) were mapped to the human genome (hg19) as described in Gjoneska et al. (2015) Nature 518:365-369 and Ernst et al. (2011) Nature 473:43-49. Since the mapping algorithm required input regions in mm10, the UCSC liftover tool was applied to ChARs to transfer them onto mm10 from mm9. GWAS SNPs in the NHGRI catalog (available on the World Wide Web at ebi.ac.uk/gwas/) annotated as "immune system disease" were defined as being immune-related. Hypergeometric tests were performed quantifying the overlap of mapped enhancers with all GWAS SNPS, immune-related SNPS as well as PICS SNPs.

### x. Comparative analysis of mouse and human data

All mouse peaks identified across the 5 cell states (Naïve, Acute d8, Acute d27, Chronic d8, Chronic d27) were partitioned into 3 categories based on MACS2-called peaks: peaks identified uniquely in Naive cells relative to Acute and Chronic d27 (mouse Naive only), peaks identified uniquely in Acute d27 cells relative to Naive and Chronic d27 (mouse Memory only), and peaks identified uniquely in Chronic d27 cells relative to Naive and Acute d27 (mouse Exhaustion only). All mouse peaks not classified into one of those 3 categories were excluded from further analysis. Orthologous mouse peaks within the three categories (Naive, Memory, Exhaustion) were mapped to the human genome and filtered for overlap with at least one MACS2 human peak called independently across all human samples. Then, for each human sample, average chromatin accessibility was calculated separately for the 3 categories of mouse orthologous peaks (Naive, Memory, Exhaustion). Average chromatin accessibility within each category was normalized to account for inherent differences in chromatin accessibility across the 3 classes of peaks.

### y. Amplification and deep sequencing of HCV epitopes

For HCV epitopes 174D and C63B, amplicons surrounding these epitopes were generated using the following conditions. The reaction consisted of 2x First Strand Buffer, sense (A2F: AAC GTT GCG ATC TGG AAG AC or A3F: GCT CTC ATG ACC GGC TTT AC) and antisense primers (A2R: GGA AGC GTG GTT GTC TCA AT or A3R: AGA GAT CTC CCG CTC ATC CT) at 0.4 µM, and a Superscript III RT/Platinum *Taq* Mix (Invitrogen), with the following conditions: cDNA synthesis for 30 minutes at 50°C, followed by heat denaturation at 94°C for 2 minutes, the PCR amplification conditions were 40 × (94°C, 15 s; 55°C, 30 s; 68°C 180 s), with a final extension at 68°C for 5 minutes. PCR amplicons were fragmented and barcoded using NexteraXT DNA Library Prep Kit per the manufacturer's protocol. Samples were pooled and sequenced on an Illumina MiSeq platform using a 2 × 250 bp V2 reagent kit. Paired-end reads obtained from Illumina MiSeq sequencing were assembled into a HCV consensus sequence using the VICUNA *de novo* assembler software (Yang et al. (2012) BMC Genomics 13:475) and finished with V-FAT v1.0. Reads were mapped back to this consensus using Mosaik v2.1.73 software and intra-host variants called using V-Phaser v2.0 software (Yang et al. (2013) BMC Genomics 14:674; Henn et al. (2012) PLoSPathogens 8:e1002529). All reads have been deposited to the NCBI Sequence Read Archive under accession number SRR3951347.

### Example 2: Chromatin accessibility of naive CD8⁺ T cells undergo large-scale remodeling during differentiation

In order to identify regulatory regions, such as enhancers and promoters, in exhausted CD8⁺ T cells, ATAC-seq (Buenrostro et al. (2013) Nat. Methods 10:1213-1218) was used to demarcate areas of accessible chromatin in antigen-specific CD8⁺ T cells differentiating in response to lymphocytic choriomeningitis virus (LCMV) infection (Figure 1A). Acute LCMV infection elicits highly functional effector CD8⁺ T cells at day 8 (d8) post-infection (p.i.), which differentiate into protective memory CD8⁺ T cells at d27 p.i. In contrast, chronic LCMV infection gives rise to exhausted CD8⁺ T cells (Wherry et al. (2007) Immunity 27:670-684; Zajac et al. (1998) J. Exp. Med. 188:2205-2213; Barber et al. (2006) Nature 439:682-687; Doering et al. (2012) Immunity 37:1130-1144; Paley et al. (2012) Science 338:1220-1225). Initial analysis of naive CD8⁺ T cells and those at d8 and d27 p.i. identified between 22,646 and 31,969 chromatin accessible regions (ChARs) in each state (*FDR* < 0.001; Figure 2A and Table 1). A high correlation between biological replicates and expected fragment size distribution confirmed good data quality (Buenrostro et al. (2013) Nat. Methods 10:1213-1218) (Figures 2B-2D). The distribution of ChARs across transcriptional start sites (TSS), exons, introns and intergenic regions was consistent with previous reports (Thurman et al. (2012) Nature 489:75-82) (Figures 2A and 2E). As expected, ChARs from each state were strongly enriched for regions of high evolutionary conservation, regulatory annotations, activating promoter and enhancer histone marks, but were depleted of Polycomb repressed regions (Thurman et al. (2012) Nature 489:75-82; Ernst et al. (2011) Nature 473:43-49) (Figures 2F and 2G).

It was found that the chromatin accessibility of naive CD8⁺ T cells underwent large-scale remodeling (Figures 1B-1C) during differentiation (DE-seq2, *FDR* < 0.05). The majority (71%, Figure 1C) of ChARs either emerged (*e.g.,* those at the *Ifng* locus) (Figure 1B) or disappeared (*e.g., Ccr7*) (Figure 1B) as naive CD8⁺ T cells underwent differentiation. The gain and loss of ChARs were not balanced; a much larger fraction of regions emerged at d8 p.i. and persisted (d8 and d27; acute 31.6%, chronic 38.8%) (Figure 1D), or emerged only at d27 (d27 only; acute 14.3%, chronic 16.9%) (Figure 1D), than were either transiently detected at d8 p.i. (d8 only; acute 14.9%, chronic 19.6%) (Figure 1D) or lost from naive cells (naive only; acute 11.3% of total regions; chronic 12.6%) (Figure 1D). Thus, differentiation from a naive CD8⁺ T cell state is associated with a net increase, rather than decrease, in chromatin accessibility (Figure 2H).

### Example 3: State-specific genomic regulatory regions in exhausted CD8+ T cells relative to functional effector or memory CD8+ T cells

ChARs from exhausted CD8⁺ T cells were compared to those found in functional effector or memory CD8⁺ T cells and marked, global differences in the pattern of regulatory regions were found. A total of 7,368 ChARs were identified that were present at d8 and/or d27 p.i. in CD8⁺ T cells responding to chronic infection, but that were absent in acute infection (Figures 1E and 2I). In addition, 4,395 regions were identified that were present at d8 and/or d27 p.i. in acute infection, but lacking in exhausted CD8⁺ T cells. Differential regulatory regions between acute and chronic infection showed features of enhancers: they tended to be depleted of TSSs (5% vs. 14% in non-differential regions), enriched for intergenic and intronic areas (37% vs. 29% and 45% vs. 31%) (Figure 1F), and were distal to gene promoters (Figure 1G). The magnitude of difference in the profile of regulatory regions between exhausted and functional CD8⁺ T cells was greater than that seen in gene expression. It was found that 44.48% of all ChARs were differentially present between functional and exhausted cells at each time point, compared to only 9.75% of differentially expressed genes (both values estimated at *FDR* < 0.05). Consistent with this finding, the rank correlation between each T cell state by gene expression was much higher than at the level of regulatory regions (Figure 1H). These results indicate that state change during CD8⁺ T cell differentiation is accompanied by a larger reorganization of accessible chromatin than is apparent by examination of gene expression.

Unsupervised clustering identified "modules" of differential ChARs with similar patterns of activity (Figures 4 and 5A). Seven modules of regulatory regions representing highly state-specific patterns of activity were identified. Modules a-e corresponded to ChARs primarily active in CD8⁺ T cells from naïve, acute d8 p.i, acute d27 p.i., chronic d8 p.i. and chronic d27 p.i., respectively. Module f identified a shared set of ChARs between naive T cells and acute d27 p.i., while module g contained shared ChARs between naive and chronic d8 p.i. (Figure 3B). A highly significant positive correlation between the average peak-intensity of ChARs within each module and the average gene expression of the adjacent genes (*p* < 0.001) was found (Figures 3B-3C). These results indicate that, on average, the ChARs contained in each module tended to be associated with the activation, rather than repression, of corresponding genes.

Genes in each state-specific module included many with known functions in the corresponding T cell state. For example, module a, most active in naive T cells, contained ChARs adjacent to *Ccr7* and *Lef1* (Figure 3D). Module d, active in chronic d8 and d27 p.i., contained ChARs adjacent to the inhibitory receptors *Pdcd1* and *Havcr2* (which encodes Tim3) and the transcription factor *Batf,* all genes that are upregulated in exhausted CD8⁺ T cells (Wherry et al. (2007) Immunity 27:670-684; Doering et al. (2012) Immunity 37:1130-1144). It was hypothesized that the modular structure of active regulatory regions would be associated with distinct regulatory programs, and surveyed the function of genes adjacent to ChARs within each module. Indeed, the functional classes of genes in each module were distinct, as measured by Gene Ontology term enrichment (Figures 3D and Table 1). For example, naive-associated module a was associated with genes involved in histone lysine methylation, regulation of IL-2 production, and stem cell division. In contrast, module b, which was primarily active in cells from acute d8 p.i., was associated with genes involved in cell chemotaxis and regulation of CD8⁺ α-β T cell differentiation. Module e, which was associated with exhaustion, was enriched for genes involved in negative regulation of cytokine production, negative regulation of LPS-mediated signaling, negative regulation of T cell activation, and regulation of IL-10 production. Thus, ChARs that distinguish naive, effector, memory and exhausted CD8⁺ T cells are organized into state-specific modules that positively regulate functionally distinct programs of genes.

### Example 4: State-specific genomic regulatory regions in exhausted CD8+ T cells regulate genes differentially expressed in exhausted CD8+ T cells

It was next sought to test whether regulatory regions specific to exhausted cells could regulate genes differentially expressed in exhausted CD8⁺ T cells. Persistent expression of PD-1 is a cardinal feature of exhausted CD8⁺ T cells, but PD-1 is also transiently expressed by effector CD8⁺ T cells during acute LCMV infection (Barber et al. (2006) Nature 439:682-687; Doering et al. (2012) Immunity 37:1130-1144). Although persistent demethylation at known regulatory regions has been observed in exhausted CD8⁺ T cells in humans (Youngblood et al. (2013) J. Immunol. 191:540-544) and mouse models (Youngblood et al. (2011) Immunity 35:400-412), it remains unclear whether PD-1 expression in exhausted CD8⁺ T cells is regulated by exhaustion-specific regulatory regions not present in functional CD8⁺ cells. Nine ChARs were identified within 45 kb of the *Pdcd1* gene locus (Figure 6A), and several were found that correspond to previously-described regions with enhancer activity (-1.5kb and -3.7kb) (Figure 6A) (Austin et al. (2014) J. Immunol. 192:4876-4886); these were present in both acute and chronic infection. An additional region approximately -23.8kb upstream of the *Pdcd1* TSS that only showed appreciable chromatin accessibility in exhausted CD8⁺ T cells at d8 and at d27 p.i. from chronic infection and not in naive, effector, or memory CD8⁺ T cells from acute infection was also identified (Figure 6A).

It was hypothesized that this ChAR might function as an enhancer of PD-1 that is required for persistent, high levels of expression in exhausted CD8⁺ T cells. Consistent with this idea, the region includes footprints for two of the top ranking exhaustion-associated TFs: Runx2 (Best et al. (2013) Nat. Immunol. 14:404-412) and retinoic acid receptor alpha (*Rara*) (Allie et al. (2013) J. Immunol. 190:2178-2187; Guo et al. (2014) J. Immunol. 192:3336-3344). This prompted a survey of chromatin accessibility at this region in the murine EL4 T cell line, and in previously published DHS (Vierstra et al. (2014) Science 346:1007-1012) or ATAC-seq data (Lara-Astiaso et al. (2014) Science 345:943-949). It was found that chromatin accessibility was not detected at any significant level throughout hematopoietic cell differentiation from Lin⁻ Sca⁺Kit⁺ cells, including terminally differentiated cell types such as granulocytes, monocytes, B cells, and NK cells (Figure 6A). However, a ChAR was identified at the same region in EL4 cells and regulatory T cells, both of which can constitutively express high levels of PD-1 (Austin et al. (2014) J. Immunol. 192:4876-4886; Raimondi et al. (2006) J. Immunol. 176:2808-2816). To test the function of the -23.8kb ChAR as a T cell enhancer, a 781 bp fragment corresponding to this region (i.e., chr1:113 95,971,118-95,971,899 of the mm9 build) was cloned into a reporter construct and had the following sequence (which is to be fully incorporated into Table 1 by this reference):

TCTCCCCAAGGGAGCCATTTCTTTCACCCTTTGTCTCTGCTCTTTCTCCTCACACT
TTTGTTTTTGTTATGGTCTGACACCCCCCCCCCCCAATCTCTCACCCCCAGTAG
CTGTGGCCGTTTTGTCCATGCCAGCCAGCTATTTCATATCGTTGCTGTAACGTGC
TTGCCAGTCACTGACACAGAGAAGTGACTTGGCTCAAGGATGTACTGACCACA
TAACCTGTTTTGTTCTGTACGCTCTGAATGTTCTGTATGAGGTTTGCTAATCTTA
AGAAATTCCACAAAGCTTTACGCAGGACCCATCAAATCAAACGTCAATATGAA
CTGTTATGTCTAAAACGGCTTGGGTCAGACCACCGGGCAACCTTTCCTGTGCCT
ACGTCTGAGCTCACTGTGGTTTTTGTGGTTGACACTGAAAAATGCTACTAATAA
GCCAAAAAATATGATTATAATATGCATGTTCTGTTATCTCAATGTTCTGAAATT
CTTCTGTTCACCACCCAACCACCACCACTCTTACCTTACTCAGAACCAATCAGC
TTAAAGGTTAACTGATAATATTTCACCCAGTAAACCAACTTCTCCCCTCTTTACC
CTTTAAACTTTTGAGCTTTTCTCATATAAAATGCCTACACTGAGAGCAGACTAG
TACCATCGCCAGGCTCCTGCAGTCCCTTCTGTGGTCCTAGATGCCCAGCACTAT
GGTGTGCATTCAATAAAGTATTCTTGCTTAACTGAGATCAGGGTTTGTTTGGTTT
GAGGGGTGACTCCCTGAACC

This fragment was sufficient to induce a 10-12-fold increase in reporter gene expression in EL4 cells and *in vitro* activated CD8⁺ T cells (Figure 6B) relative to a vector encoding a minimal promoter only. This suggests that the ChAR at -23.8kb can function as an enhancer.

It was then tested whether the -23.8kb enhancer was necessary for high-level PD-1 expression. The CRISPR/Cas9 nuclease was used to delete a 1.2 kb fragment at that position in EL4 cells, which have both sustained high-level PD-1 expression and open chromatin at that enhancer site (Shalem et al. (2014) Science 343 :84-87; Canver et al. (2015) Nature 527:192-197) (Figures 5A-5E). It was then tested whether loss of the - 23.8kb region affected PD-1 expression using several approaches. First, it was determined that the regions were required for high-level PD-1 expression. Cas9-expressing EL4 cells were transfected with a pair of sgRNAs flanking the enhancer or with control guides, the cells were sorted into sub-populations that differed in PD-1 expression, and the cells were tested for the relative abundance of enhancer-deleted or wild-type alleles by genomic PCR (Figure 6C) in each sub-population. In EL4 cells transfected with the -23.8kb targeting pair of sgRNAs, the cells with the lowest PD-1 expression had the highest abundance of the enhancer-deleted band, and lowest abundance of the wild-type band.

Second, a large number of single-cell clones from PD-1^{lo} EL4 cells transfected with the -23.8kb sgRNA pair were generated. Nine clones out of 45 screened (20%) (Figures 5F-5G) were identified as having the deletion of the -23.8kb region and it was further found that the geometric mean fluorescence intensity (MFI) of the deleted clones was significantly lower than the non-deleted clones (Mann-Whitney test *P* < 0.002) (Figure 5G). The deletion of the -23.8kb ChAR was confirmed using Sanger sequencing (Figure 5H) and all nine clones were found to have a PCR deletion band that showed a biallelic deletion of the target ChAR. The sequences shown in Figure 5H are as follows:

GTTGCCTCAGAAAGCTAACATCTGGTGCCAAAACCCAGGAGGACATCTGGAGTCCACAGACGGCTTTGAGCTTCTGC
TGGATCTCCCCT GAAGGACACATCCTTAGCCTGTCTCGGGCTAATCTTAAAGAGTTCT WT

GTTGCCTCAGAAAGCTAACATCTGGTGCCAAAACCCAGGAGGACATCTGGAGTCCAC---------------------
------------------------------------------------------------------------------
----------------CCTGTCTCGGGCTAATCTTAAAGAGTTC 21

GTTGCCTCAGAAAGCTAACATCTGGTGCCAAAACCCAGGAGGACATCTGGAGTCCAC--------------------
-----------------------------------------------------------------------------
--------------CCTGTCTCGGGCTAATCTTAAAGAGTTCT                    22

GTTGCCTCAGAAAGCTAACATCTGGTGCCAAAACCCAGGAGGACATCTGGAGTCCAC---------------------
------------------------------------------------------------------------------
--------------CCTGTCTCGGGCTAATCTTAAAGAGTTCT 34

GTTGCCTCAGAA---------------------------------------------------------------
---------------------------------------------------------------------------
-----------------GAAGGACACATCCTTAGCCTGTCTCGGGCTAATCTTAAAGAGTTCT 40

GTTGCCTCAGAAAGCTAACATCTGGTGC-------------------------------------------------
-----------------------------------------------------------------------------
-------------------------------------------------------------------
GCTAATCTTAAAGAGTTCT                                                        41

GTTGCCTCAGAAAGCTAACATCTGGTGC------------------------------------------------
----------------------------------------------------------------------------
------------------------------------------------------------------
GCTAATCTTAAAGAGTTCT                                                    42

GTTGCCTCAGAAAGCTAACATCTGGTGC------------------------------------------------
----------------------------------------------------------------------------
------------------------------------------------------------------
GCTAATCTTAAAGAGTTCT                                                       43

GTTGCCTCAGAAAGCTAACATCTGGTGCCAAAACCCAGGAGGACATCTGGAGTCCAC----

--------------------------------------------------------------------------
-----CCTGTCTCGGGCTAATCTTAAAGAGTTCT 44

GTTGCCTCAGAAAGCTAACATCTGGTGCCAAAACCCAGGAGGACATCTGGAGTCCAC----------------------
-------------------------------------------------------------------------------
--------------CCTGTCTCGGCTAATCTTAAAGAGTTCT 45

Deletion of this region resulted in a 63.2% decrease in PD-1 expression, but not total loss of expression, suggesting additional regulatory regions in EL4 cells were also involved in regulating PD-1 expression (Figure 6D).

Third, it was assessed whether deletion of the -23.8kb ChAR specifically decreased PD-1 expression by quantifying the expression of all genes with detectable expression that are within 1.5Mb of the *Pdcd1* locus. Only PD-1 mRNA expression was significantly decreased by deletion of the -23.8kb ChAR (Figure 5I). This result indicates that the - 23.8kb ChAR present in exhausted, but not functional CD8⁺ T cells, serves as an enhancer that is required to maintain high-level PD-1 expression.

It was next sought to identify the functional contribution of specific sequences within the -23.8kb enhancer to the regulation of PD-1 expression. Cas9-mediated *in situ* saturation mutagenesis was used. All possible sgRNAs within the enhancer, as constrained by the protospacer adjacent motif (PAM) (Canver et al. (2015) Nature 527:192-197; Vierstra et al. (2015) Nat. Methods 12:927-930), were designed. In order to compare the effect of mutagenizing the -23.8kb enhancer with other regulatory elements adjacent to the *Pdcd1* locus, a similar tiled set of sgRNAs to target the other eight PD-1 regulatory sequences defined by the ATAC-seq analysis was also designed (Figure 6A). Cas9-expressing EL4 cells were transduced with a pool of 1,754 enhancer-targeting sgRNAs, 117 sgRNAs targeting the *Pdcd1* exons as positive controls, and 200 non-targeting sgRNAs as negative controls (Figure 7A). The median distance between adjacent sgRNAs was 5 bp, with 90% of sgRNAs falling within 18bp of each other, allowing for the identification of genomic cleavage sites that disrupt PD-1 expression with high resolution (Figure 7B).

Transduced EL4s were transduced into populations based on high vs. low PD-1 expression, and the abundance of individual sgRNAs within the two fractions were quantified using sequencing of extracted genomic DNA (Figure 7C). In comparison to non-targeting sgRNAs, which were equivalently distributed between PD-1 high and low fractions, sgRNAs targeting *Pdcd1* exons were highly enriched in the PD-1 low fraction as expected (Figures 6E and 7D-7E). It was found that sgRNAs targeting eight of the nine regulatory regions were also significantly enriched in the PD-1 low fraction to varying degrees (*p* < 0.00001 to *p* < 0.01), indicating a dense representation of critical sequences affecting PD-1 expression in each of the eight regulatory regions. However, sgRNAs in the -35.6kb ChAR had no significant effect on PD-1 expression, consistent with prior observations that this region falls outside the CTCF-mediated boundaries of the *Pdcd1* locus (Austin et al. (2014) J. Immunol. 192:4876-4886).

sgRNAs within the -23.8kb enhancer were focused upon by generating 32 isogenic lines of Cas9-expressing EL4 cells, each transfected with one of the sgRNAs within the enhancer that was enriched in the PD-1 low fraction. A strong correlation between the predicted activity in a pooled setting of the sgRNAs most strongly enriched in the PD-1 negative fraction (PD-1 high:low ratio >1SD below mean) and their effect on PD-1 MFI in individual cell lines (*p* = 0.0041) (Figure 6F) was found. This result indicates that the pooled screen identified sgRNAs disrupting critical sequences of the enhancer with high specificity. Inspection of the predicted cleavage site locations revealed three critical regions of the enhancer in which cleavage markedly affected PD-1 expression (Figure 6G, grey shading).

It was next asked whether these critical regions in the -23.8kb enhancer were associated with distinct patterns of transcription factor (TF) binding in exhausted CD8⁺ T cells *in vivo.* As TF ChIP experiments are not feasible in rare populations of exhausted CD8⁺ T cells, ATAC-seq tracks from exhausted d27 CD8⁺ T cells were analyzed to infer TF binding based on an attenuation of transposase cut sites over known TF motifs (*i.e.,* TF footprinting) (Pique-Regi et al. (2011) Genome Res. 21:447-455) (Figures 8A-8B and Table 1). In order to validate TF footprinting in the data, it was first confirmed that the approach could recapitulate ChIP-seq data of TF binding. TF footprinting was compared with existing TF ChIP data for BATF and IRF4 in *in vitro* activated CD8⁺ T cells and high levels of sensitivity and specificity (AUC = 0.77 and 0.84) were found (Figures 8C-8D). Differential TF footprints (posterior probability >0.90 to call binding sites) (Figures 8A-8D) in naive and acute d8 p.i. cells were also identified. This analysis recovered motifs associated with known regulators of effector function including the AP-1 family TFs Batf, Jun, and Fos and the naïve T cell regulator Lef1 (Kaech and Cui (2012) Nat. Rev. Immunol. 12:749-761; Kurachi et al. (2014) Nat. Immunol. 15:373-383) (Figure 10A).

Having validated TF footprinting analysis of the ATAC-seq data, TF footprints were identified in chronic d27 exhausted T cells within the -23.8kb PD-1 enhancer. It was found that cleavage sites of sgRNAs that reduced PD-1 expression in EL4 cells were significantly enriched in TF-bound motifs found in exhausted CD8⁺ cells *in vivo* (*P* = 8.63×10⁴, hypergeometric test). The three TF footprints with greatest sensitivity to disruption corresponded to motifs for Sox3, T-bet (encoded by *Tbx21*) and retinoic acid receptor (RAR) in exhausted CD8⁺ T cells *in vivo* (Figures 6G and 10B). Based on these data, it is believed that TF binding to these motifs in the -23.8kb enhancer plays a critical role in regulating PD-1 in exhausted CD8⁺ T cells.

In order to determine whether increased TF binding at these motifs is a more general feature of T cell exhaustion, genome-wide TF footprinting was compared between chronic and acute infection at d27 and 135 TF motifs that showed significant differential inferred binding were identified (Figure 6H and Table 1). Several features indicated that binding at these TF motifs is likely to play a role in regulating T cell exhaustion, including the fact that top ranking TFs were found to show distinct patterns of preferential binding across the modules of active regulatory regions (Figure 9A). First, bound motifs were observed more frequently at genes differentially expressed between CD8⁺ T cells in chronic vs. acute infection at d27 p.i. than in genes that were not differentially expressed (hypergeometric *P* = 9.85×10⁻³, differential gene expression *FDR* < 0.05) (Figure 10A). Second, many bound motifs recovered by the analyses corresponded to TFs with known roles in exhaustion (Figures 6H and 10C), including T-bet (Tbx21; Paley et al. (2012) Science 338:1220-1225), Eomes (Paley et al. (2012) Science 338:1220-1225), Batf (Quigley et al. (2010) Nat. Med. 16:1147-1151), Blimp1 (Shin et al. (2009) Immunity 31:309-320), Nfatc1 (Martinez et al. (2015) Immunity 42:265-278), Nr4a2 (Giordano et al. (2015) EMBO J. 34:2042-2058), and Mafb (Giordano et al. (2015) EMBO J. 34:2042-2058). Third, other differential TF footprints suggest plausible, novel candidate regulators of T cell exhaustion, such as: Pou2f2 (also known as Oct-2) which interacts with AP-1 family TFs (de Grazia et al. (1994) J. Exp. Med. 180:1485-1497), Foxp1, which regulates quiescence in naive CD8⁺ T cells (Feng et al. (2011) Nature Immunol. 12:544-550), and retinoic acid receptor alpha (*Rara*), which negatively regulates CD8⁺ T cell memory development (Allie et al. (2013) J. Immunol. 190:2178-2187), while promoting CD8⁺ T cell function in the tumor microenvironment (Guo et al. (2014) J. Immunol. 192:3336-3344). Notably, it was found that TF footprints at retinoic acid receptor alpha motifs (*Rara*) were significantly enriched in exhausted CD8⁺ T cells (*FDR* = 3.14×10⁻¹³), indicating that the role of this family of TFs in exhausted CD8⁺ T cells extends beyond PD-1 to the regulation of a larger transcriptional program.

Extensive changes in TF binding over time in chronic infection, which was primarily characterized by the acquisition of new TF binding events in exhausted CD8⁺ T cells, were also found. As described above, for example, *Tbx21* and *Eomes* bound more regions in acute than in chronic infection at d8 p.i, but by d27 p.i, both TFs had acquired large numbers of new binding sites in exhausted CD8⁺ T cells, consistent with their known role in terminal exhaustion (Paley et al. (2012) Science 338:1220-1225) (Figures 10C and 9B). More generally, a pronounced shift in the binding patterns of TFs from d8 to d27 p.i. towards preferential binding at new sites in chronic infection by d27 p.i. was observed (Figure 10C). This led to a corresponding increase in number of differential TF binding events in exhaustion relative to memory (Figure 9B). T cell exhaustion thus develops with extensive reprogramming of TF binding over time.

### Example 5: State-specific genomic regulatory regions in exhausted CD8+ T cells are relevant to human diseases

Analysis of mouse models of LCMV infection suggests that T cell exhaustion is associated with a distinct epigenetic state. To test if the exhaustion-specific enhancer profile in mouse models was also a feature of human exhausted CD8⁺ T cells, global patterns of chromatin accessibility were analyzed in tetramer⁺ CD8⁺ T cells specific for HIV from four subjects with chronic progressive HIV who were not on therapy (Figures 11A and Table 1). These patterns were compared to those found in cytomegalovirus (CMV)-specific CD8⁺ T cells from a healthy donor. Bulk populations of naive (CCR7⁺CD45RA⁺) and effector memory (CCR7⁻CD45RA⁻) from each donor served as controls. It was found that ChARs from each donor were highly enriched for regions associated with activating chromatin marks, and depleted of regions containing repressive marks, confirming good data quality (Figure 11B).

ChARs identified in the mouse model were mapped to their human orthologous regions and successfully aligned 80-85% of all mouse ChARs (Gjoneska et al. (2015) Nature 518:365-369; Villar et al. (2015) Cell 160:554-566) (Figures 12A-12B). Regions mapped from all five mouse CD8⁺ T cell states were significantly enriched for disease-associated SNPs (PICS SNPs, *P* < 2.77 ×10⁻⁸; hypergeometric test) (Farh et al. (2015) Nature 518:337-343) (Figure 12C), and in particular immune-related NHGRI GWAS SNPs (*P* < 3.70×10⁻³0 (Figures 11C-11E), suggesting mapped regions corresponded to important regulatory regions within the immune system. However, regions at the *PDCD1* locus were not among those mapped from the mouse model, as previously observed (Austin et al. (2014) J. Immunol. 192:4876-4886), limiting the ability to detect an ortholog to the -23.8kb enhancer observed in the mouse model.

Non-overlapping sets of ChARs unique to naive, memory (acute d27) or exhausted cells (chronic d27) were identified in the mouse and tested for chromatin accessibility within orthologous regions in each human sample (Figure 12D). Human naïve CD8⁺ T cells from the majority of donors showed significantly greater chromatin accessibility in naive-specific regions defined in the mouse than in memory- or exhaustion-specific regions. In the healthy donor, CMV-specific tetramer⁺ CD8⁺ T cells, and effector memory cells were enriched for memory-specific regions (MW test, *P* = 0.01 to *P* < 0.0001) (Figure 12E). In contrast, in HIV-specific tetramer⁺ cells from 3 out of the 4 subjects showed significantly greater chromatin accessibility in exhaustion-specific regions (MW test, *p* = 0.05 to *p* < 0.001) than in memory-specific regions. Interestingly, effector memory CD8⁺ T cells in HIV-infected subjects also showed greater chromatin accessibility in exhaustion-specific regions, possibly as a result of unmeasured exhausted HIV-specific T cells within that compartment or from bystander T cells with aberrant differentiation (Stelekati et al. (2014) Immunity 40:801-813).

These findings were confirmed in a subject with chronic HCV infection by surveying exhaustion-specific epigenetic patterns in multiple tetramer⁺ populations from the same donor. Tetramer⁺ CD8⁺ T cells specific for two different epitopes from HCV and one specific for influenza (Flu), as well as bulk naive and effector memory cells, were isolated. Flow cytometry demonstrated that naive CD8⁺ T cells were uniformly negative for the exhaustion markers PD-1 and CD39, as expected. The majority of tetramer⁺ CD8⁺ T cells specific for the HCV C63B epitope expressed high levels of PD-1 (Rutebemberwa et al. (2008) J. Immunol. 181:8215-8225; Kasprowicz et al. (2008) J. Virol. 82:3154-3160) and CD39 (Gupta et al. (2015) PLoSPathogens 11:e1005177), as previously described for HCV-specific CD8⁺ T cells in chronic infection (Figure 12F). However, HCV tetramer⁺ cells specific for the 174D epitope were PD-1-and CD39-low, closely resembling the staining pattern of functional Flu tetramer⁺ cells. Sequencing of the HCV genome in this subject revealed that unlike the C63B epitope, the 174D epitope had undergone extensive viral escape and no wild-type viral sequence could be detected. This suggests that the 174D tetramer⁺ T cells were no longer encountering cognate antigen (Figure 12G). Consistent with this observation, it was determined that the C63B Tet⁺ cells showed significantly greater chromatin accessibility at exhaustion-specific regions (MW test, *p* = 0.01) than memory regions. In contrast, 174D tetramer⁺ cells showed greater chromatin accessibility in memory-specific regions, as did influenza-specific CD8⁺ T cells (MW test, *p* = 0.04) (Figure 12H). Thus, the state-specific pattern of chromatin accessibility found in mouse exhausted CD8⁺ T cells is conserved in human exhausted CD8⁺ T cells.

### Example 6: State-specific genomic regulatory regions in exhausted CD8+ T cells are relevant to human diseases

Further to the discovery of the ChAR region at -23kb upstream of PD-1 that is present in exhausted but not functional CD8+ T cells and serves as an enhancer required to maintain high-level PD-1 expression *in vitro,* the role of this ChAR *in vivo* was characterized. Mice were generated with germline deletion of this region (Figure 13A). Briefly, C57BL/6J eggs were injected with Cas9 mRNA, as well as *in* vitro-validated sgRNAs before implantation into pseudo-pregnant females (Figure 13B). Out of 7 viable progeny, 2 mice had heterozygous deletion of the ChAR (Figure 13C), and one was able to pass on the allele to its progeny. These mice were bred to the P14 TCR transgenic line to generate homozygous enhancer deleted animals, as well as WT control littermates, on CD45.1 single positive and CD45.1/CD45.2 double positive backgrounds, respectively. The effect of this deletion on CD8+ T cells was then assessed during acute and chronic LCMV infection using an adoptive cell transfer strategy (Figure 14A). Two mixes were created: one containing enhancer-deleted cells marked with CD45.1 only (SP) versus wild-type (WT) cells with CD45.1/CD45.2 (DP), and the other containing WT cells on a SP versus DP background. Littermate donor mice were used for each mix and recipient mice were on a CD45.2 single positive C57BL/6J background. Data from Armstrong- and Clone 13-infected animal cohorts were combined in further analyses for statistical power.

The data indicate that congenically marked WT vs. WT cell populations are equivalently recovered *in vivo* and have similar expression of the transgenic TCR and the inhibitory receptor PD-1 (Figure 14B). In contrast, enhancer-deleted cell populations outcompete the WT cells in competitive mixes and are thus recovered at a much higher rate (Figure 14B). They also have lower expression of PD-1 (*p* = 0.02 for enhancer-deleted, paired t-test) compared to WT populations (*p* = 0.36, paired t-test) (Figures 14B-14C). Compared to WT vs. WT mixes, the log2 fold enrichment of enhancer deleted populations over WT cells are significantly higher at d8 (*p* < 0.001, t-test) and at d15 (*p* = 0.0044, t-test) (Figsures 14D-14E). Taken together, these data indicate that genetic deletion of the PD-1 enhancer -23kb upstream of the promoter leads to lower expression of PD-1 in both acute and chronic viral infection *in vivo* and increases T cell proliferation and/or survival.

Based on the foregoing, it was found that exhausted CD8⁺ T cells in mice and humans show a global pattern of regulatory regions that is distinct from their functional counterparts. These data have three important implications for our understanding of T cell exhaustion.

First, the data show that exhaustion occurs with a combination of remodeled enhancer accessibility and new TF binding, and supports a model in which exhausted CD8⁺ T cells are not simply "persistent effectors" but rather a differentiation state distinct from their functional counterparts. Identifying the plasticity of this state and whether or how it could be reverted becomes a critical question. The absence of an exhaustion-specific epigenetic profile in HCV-specific CD8⁺ T cells that no longer recognize an escaped epitope indicates that reducing antigen burden may be an important factor in rescuing exhaustion but awaits confirmation in longitudinal studies. Checkpoint blockade in the absence of removing chronic antigen-exposure for reverting the epigenetic profile of T cell exhaustion has important implications for cancer immunotherapy.

Second, it has been demonstrated herein that *in situ* mutagenesis of enhancers *in vitro* combined with TF footprinting *in vivo* is a useful approach for fine mapping of enhancer function. The feasibility of TF ChIP studies is limited in situations where few relevant cells can be isolated. As an alternative, TF footprinting was used *in vivo* to identify motifs with inferred TF binding at state-specific enhancers in exhausted CD8⁺ T cells and probed the function of these TF footprints using Cas9-mediated saturation mutagenesis *in vitro.* While this approach is limited by the degeneracy of TF motifs (*e.g.,* a T-bet motif may in fact be bound by other T-box factors such as Eomesodermin), it highlights the ability to uncover a role for novel potential regulators of T cell exhaustion such as the RAR family of TFs.

Third, mapping state-specific enhancers in exhausted T cells is believed to provide an opportunity to improve the therapeutic efficacy of adoptive T cell therapy. Infusion of T cells engineered to express chimeric antigen receptors directed at cancer have had less success in solid tumors in part because of the development of T cell exhaustion in the engineered T cell product (Long et al. (2015) Nat. Med. 21:581-590; Stromnes et al. (2015) Cancer Cell 28:638-652). Genome editing of therapeutic T cells to make them resistant to exhaustion is an appealing concept and has led to recent studies investigating the deletion of the PD-1 gene locus (Hendel et al. (2015) Nat. Biotechnol. 33 :985-989; Schumann et al. (2015) Proc. Natl. Acad. Sci. U.S.A. 112:10437-10442). However, irreversible deletion of PD-1 may not be desirable, given its critical regulatory role in preventing excessive activation of T cells in chronic infection (Odorizzi et al. (2015) J. Exp. Med. 212:1125-1137). In contrast, editing exhaustion-specific enhancers (Canver et al. (2015) Nature 527:192-197) that regulate genes which impair the function of exhausted CD8⁺ T cells is believed to provide an additional approach to modulate T cell function that is more "tunable" and state-specific than targeting exons. For example, it provides an approach to prevent overexpression that contributes to T cell inhibition but preserve the physiological regulation of PD-1. Creating detailed functional maps of enhancers specific to exhausted CD8⁺ T cells in humans is believed to provide a crucial step towards the rational engineering of T cells for therapeutic use.

**Table 1**

| Summary and Legend | | | | | | |
|---|---|---|---|---|---|---|
| Table 1A shows differential regions between acute and chronic conditions at day 8 or day 27. | | | | | | |
| Table 1B shows differential regions between acute and chronic conditions at day 27. | | | | | | |
| Table 1C shows differential regions between acute and chronic conditions at day 8 & day 27. | | | | | | |
| Table 1D shows regions that appear and are active at day 27. | | | | | | |
| Table 1E shows regions that appear and are active at day 8 and day 27. | | | | | | |
| Table 1F shows regions that are only differential between acute and chronic conditions at day 8 and day 27. | | | | | | |
| Table 1G shows regions that are only differential between acute and chronic conditions at day 27. | | | | | | |
| Table 1H shows all human chromatin accessible regions that were identified in bulk naive and effector memory CD8⁺ T cells, as well as in antigen-specific CD8⁺ T cells responding to CMV, Influenza, HIV or HCV. | | | | | | |
| Table 1I shows only human chromatin accessible regions from Table 1H that could be associated with a mouse orthologous region from mouse naive, memory and exhausted CD8⁺ T cells. | | | | | | |
| Table 1J shows only human chromatin accessible regions from Table 1I that is associated with a mouse orthologous region specific to mouse exhausted CD8⁺ T cells. | | | | | | |
| Table 1K shows sgRNA sequences and genomic location annotations of all analyzed high-resolution *Pdcd1* enhancer sequences. | | | | | | |

| | # of regions | # of enhancers | # of suppressors | # mixed | # no change | # mouse regions mapped to human genome |
|---|---|---|---|---|---|---|
| Table 1A | 7,537 | 60 | 64 | 1 | 7,412 | 6,143 |
| Table 1B | 4,858 | 44 | 36 | 1 | 4,777 | 3,963 |
| Table 1C | 2,154 | 24 | 19 | 0 | 2,111 | 1,745 |
| Table 1D | 1,870 | 26 | 17 | 1 | 1,826 | 1,297 |
| Table 1E | 1,759 | 17 | 12 | 0 | 1,730 | 1,399 |
| Table 1F | 1,043 | 9 | 6 | 0 | 1,028 | 846 |
| Table 1G | 532 | 7 | 5 | 1 | 519 | 426 |
| All regions | 43,171 | | | | | |

The columns labeled in Tables 1A-1K with abbreviations correspond to the following:

| | |
|---|---|
| A | Naive |
| B | Acute d8 (day 8) |
| C | Acute d27 (day 27) |
| D | Chronic d8 |
| E | Chronic d27 |
| a | Acute d8 versus Naive |
| b | Chronic d8 versus Naive |
| c | Acute d27 versus Naive |
| d | Chronic d27 versus Naive |
| e | Chronic d8 versus Acute d8 |
| f | Chronic d27 versus Acute d27 |
| g | Acute d27 versus Acute d8 |
| h | Chronic d27 versus Chronic d8 |
| i | Chronic d8 versus Acute d27 |
| j | Chronic d27 versus Acute d8 |
| F | Naive peak |
| G | Acute d8 peak |
| H | Acute d27 peak |
| I | Chronic d8 peak |
| J | Chronic d27 peak |

The gene expression terms below shown in Tables 1A-1K correspond to the following:

| | |
|---|---|
| Enhancer | Positive association with gene expression |
| Suppressor | Negative association with gene expression |
| Mixed | Positive and negative association with gene expression |
| No change | No or weak association with gene expression |

In addition, the regions shown in Tables 1A-1K map to NCBI Mouse Assembly MGSCv37 (September 23, 2010) (accessible under GenBank assembly accession GCA_000000055.16 and RefSeq assembly accession GCF_000001635.18) and known as "build mm9" or Genome Reference Consortium Human Assembly GRCh37 (March 3, 2009) (accessible under GenBank assembly accession GCA _000001405.1 and RefSeq assembly accession GCF_000001405.13) and known as "build hg19."
* Also included in Table 1 are genomic nucleic acid sequences comprising the listed regions, or an ortholog thereof within the genome of a mammal other than mice, including humans. In addition, the listed regions, or ortholog thereof, further comprising about 1 bp, 2 bp, 3 bp, 4 bp, 5 bp, 10 bp, 15 bp, 20 bp, 25 bp, 30 bp, 35 bp, 40 bp, 45 bp, 50 bp, 55 bp, 60 bp, 65 bp, 70 bp, 75 bp, 80 bp, 85 bp, 90 bp, 95 bp, 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, 2000 bp, 2100 bp, 2200 bp, 2300 bp, 2400 bp, 2500 bp, 2600 bp, 2700 bp, 2800 bp, 2900 bp, or 3000 bp, or any range in between, inclusive (e.g., 1 bp to 222 bp), on the 5' end, on the 3' end, or on both the 5' and the 3' ends. In addition, any portion or fragment thereof is contemplated. Further contemplated is any genomic nucleic acid sequence comprising a nucleic acid sequence having at least 80%, 81%, 82%, 83%, 84%, 85%, 86%, 87%, 88%, 89%, 90%, 91%, 92%, 93%, 94%, 95%, 96%, 97%, 98%, 99%, 99.5%, or more identity across their full length with such a region, or portion/fragment thereof. The described nucleic acid molecules can have a function of the nucleic acids of the listed regions.

## Claims

1. An engineered mammalian T cell comprising a genomic region that 1) regulates the gene expression of *pdcd1* and 2) is selectively chromatin accessible within the genome of an exhausted CD8+ T cell from the mammal, wherein the genomic region is genetically modified to delete the region corresponding to the nucleic acid sequence of SEQ ID NO: 12, which is tctccccaag ggagccattt ctttcaccct ttgtctctgc tctttctcct cacacttttg tttttgttat ggtctgacac cccccccccc ccaatctctc acccccagta gctgtggccg ttttgtccat gccagccagc tatttcatat cgttgctgta acgtgcttgc cagtcactga cacagagaag tgacttggct caaggatgta ctgaccacat aacctgtttt gttctgtacg ctctgaatgt tctgtatgag gtttgctaat cttaagaaat tccacaaagc tttacgcagg acccatcaaa tcaaacgtca atatgaactg ttatgtctaa aacggcttgg gtcagaccac cgggcaacct ttcctgtgcc tacgtctgag ctcactgtgg tttttgtggt tgacactgaa aaatgctact aataagccaa aaaatatgat tataatatgc atgttctgtt atctcaatgt
tctgaaattc ttctgttcac cacccaacca ccaccactct taccttactc agaaccaatc agcttaaagg ttaactgata atatttcacc cagtaaacca acttctcccc tctttaccct ttaaactttt gagcttttct catataaaat gcctacactg agagcagact agtaccatcg ccaggctcct gcagtccctt ctgtggtcct agatgcccag cactatggtg tgcattcaat
aaagtattct tgcttaactg agatcagggt ttgtttggtt tgaggggtga ctccctgaac c, that is -23.8kb upstream of the *pdcd1* transcription start site and the genetic modification decreases the expression of *pdcd1.*

2. An *in vitro* or *ex vivo* method of engineering a mammalian T cell to downregulate expression of *pdcd1* in the mammalian T cell, the method comprising deleting the region corresponding to the nucleic acid sequence of SEQ ID NO: 12, which is tctccccaag ggagccattt ctttcaccct ttgtctctgc tctttctcct cacacttttg tttttgttat ggtctgacac cccccccccc ccaatctctc acccccagta gctgtggccg ttttgtccat gccagccagc tatttcatat cgttgctgta acgtgcttgc cagtcactga cacagagaag tgacttggct caaggatgta ctgaccacat aacctgtttt gttctgtacg
ctctgaatgt tctgtatgag gtttgctaat cttaagaaat tccacaaagc tttacgcagg acccatcaaa tcaaacgtca atatgaactg ttatgtctaa aacggcttgg gtcagaccac cgggcaacct ttcctgtgcc tacgtctgag ctcactgtgg tttttgtggt tgacactgaa aaatgctact aataagccaa aaaatatgat tataatatgc atgttctgtt atctcaatgt
tctgaaattc ttctgttcac cacccaacca ccaccactct taccttactc agaaccaatc agcttaaagg ttaactgata atatttcacc cagtaaacca acttctcccc tctttaccct ttaaactttt gagcttttct catataaaat gcctacactg agagcagact agtaccatcg ccaggctcct gcagtccctt ctgtggtcct agatgcccag cactatggtg tgcattcaat
aaagtattct tgcttaactg agatcagggt ttgtttggtt tgaggggtga ctccctgaac c, that is -23.8kb upstream of the *pdcd1* transcription start site by genetic modification and wherein the genetic modification downregulates the expression of *pdcd1.*

3. The engineered T cell of claim 1 or the method of claim 2,
wherein the engineered genomic gene expression regulatory region results in at least a 5% downregulation of expression of *pdcd1* in the mammalian T cell as compared to the expression of *pdcd1* in the same T cell type from the mammal without the engineered genomic gene expression regulatory region or/and wherein the expression of *pdcd1* is transcription or translation of *pdcd1* or/and wherein the genomic gene expression regulatory region is deleted by genome editing, optionally wherein the genome editing is expressed constitutively or inducibly, preferably
wherein the genome editing is selected from the group consisting of CRISPR-Cas9 RNA-guided engineered nucleases (RGENs), zinc finger nucleases (ZFNs), transcription activator-like effectors (TALEs), homing meganucleases, and homologous recombination.

4. The engineered T cell or the method of any one of claims 1-3, wherein the mammal is an animal model of an immune disorder, optionally wherein the immune disorder is a chronic immune disorder, preferably
wherein the animal model is a mouse model or/and
wherein the mammal is a mouse or a human, preferably
wherein the mammal is a human, preferably
wherein the human is afflicted with an immune disorder, optionally wherein the immune disorder is a chronic immune disorder.

5. The engineered T cell or the method of claim 4, wherein the chronic immune disorder is a chronic infection, preferably
wherein the infection is caused by an agent selected from the group consisting of human immunodeficiency virus (HIV), hepatitis C virus (HCV), hepatitis B virus (HBV), adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyoma virus BK, polyoma virus IC, measles virus, rubella virus, human T cell leukemia virus I, human T cell leukemia virus II, *Leishmania, Toxoplasma, Trypanosoma, Plasmodium, Schistosoma,* and *Encephalitozoon,* preferably
wherein the chronic infection is not a latent infection.

6. The engineered T cell or the method of claim 4, wherein the chronic immune disorder is a cancer, preferably
wherein the cancer is a hematological cancer or a solid cancer, preferably
wherein the solid cancer is selected from the group consisting of lung cancer, non-small cell lung cancer (NSCLC), skin cancer, melanoma, cervical cancer, uterine cancer, ovarian cancer, breast cancer, pancreatic cancer, stomach cancer, esophageal cancer, colorectal cancer, liver cancer, prostate cancer, kidney cancer, bladder cancer, head and neck cancer, sarcoma, lymphoma, and brain cancer.

7. The engineered T cell or the method of any one of claims 1-6, wherein the exhausted CD8+ T cell expresses a T cell exhaustion biomarker selected from the group consisting of a checkpoint inhibitor, PD-1 (Pdcd1), TIM-3 (Havcr2), LAG-3 (Lag3), CTLA-4 (Ctla4), 2B4 (CD244), CD39 (Entpdl), CD160, eomesodermin (Eomes), T-BET (Tbx21), BATF, BLIMP-1 (Prdml), NFATC1, NR4A2, MAFB, OCT-2 (Pou2f2), Foxpl, retinoic acid receptor alpha (Rara), and combinations thereof, preferably
wherein the non-exhausted CD8+ T cell is a naive, functional effector, or memory cell.

8. The engineered T cell or the method of any one of claims 1-7, wherein the T cell is a primary T cell isolatable from the mammal, engineerable, and returnable to the mammal, or
wherein the T cell is administrable to the mammal or is cultured *in vitro.*

9. The method of any one of claims 2-8 wherein exhaustion is prevented in a non-exhausted CD8+ T cell, preferably
wherein the non-exhausted CD8+ T cell is a naive, functional effector, or memory cell, or/and
further comprising formulating the engineered non-exhausted CD8+ T cell into a form administrable to a subject.

10. The method of any one of claims 2-8 wherein CD8+ T cell exhaustion is reversed in an exhausted CD8+ T cell, preferably
wherein the exhausted CD8+ T cell expresses a T cell exhaustion biomarker selected from the group consisting of a checkpoint inhibitor, PD-1 (Pdcd1), TIM-3 (Havcr2), LAG-3 (Lag3), CTLA-4 (Ctla4), 2B4 (CD244), CD39 (Entpdl), CD160, eomesodermin (Eomes), T-BET (Tbx21), BATF, BLIMP-1 (Prdml), NFATC1, NR4A2, MAFB, OCT-2 (Pou2f2), Foxpl, retinoic acid receptor alpha (Rara), and combinations thereof, preferably
further comprising formulating the engineered exhausted CD8+ T cell into a form administrable to a subject.

11. The engineered T cells of any one of claims 1-8 for use in treating an immune disorder in the subject, preferably
wherein the engineered T cells are administered focally or systemically.

12. The engineered T cells for use according to claim 11, wherein the systemic administration is intravenous, intramuscular, intraperitoneal, or intra-articular, or
wherein the engineered T cells administered to the subject are autologous, syngeneic, allogeneic, or xenogeneic to the subject or/and
wherein the engineered T cells administered to the subject are administered in a pharmaceutically acceptable formulation or/and
wherein the engineered T cells maintain the at least 5% modulated expression of *pdcd1* after administration to the subject or land
further comprising administering to the subject a therapeutically effective amount of a pharmaceutical composition comprising one or more anti-immune disorder agents or/and
further comprising contacting the CD8+ T cells with one or more agents that prevent or reverse CD8+ T cell exhaustion, preferably
wherein the one or more agents is an immune checkpoint inhibitor, preferably wherein the immune checkpoint is selected from the group consisting of PD-1, PD-L1, PD-L2, LAG-3, TIM-1, CTLA-4, VISTA, B7-H2, B7-H3, B7-H4, B7-H6, 2B4, ICOS, HVEM, CD160, gp49B, PIR-B, KIR family receptors, TIM-1, TIM-4, BTLA, SIRPalpha (CD47), CD48, 2B4 (CD244), B7.1, B7.2, ILT-2, ILT-4, TIGIT, and A2aR.

13. The engineered T cells for use according to claim 11 or 12, wherein the subject is an animal model of an immune disorder, optionally wherein the immune disorder is a chronic immune disorder, preferably
wherein the animal model is a mouse model, or/and
wherein the subject is a mouse or a human, or
wherein the subject is a human, preferably
wherein the human is afflicted with an immune disorder, optionally wherein the immune disorder is a chronic immune disorder.

14. The engineered T cells for use according to any one of claims 11-13,
wherein the chronic immune disorder is a chronic infection, preferably
wherein the infection is caused by an agent selected from the group consisting of human immunodeficiency virus (HIV), hepatitis C virus (HCV), hepatitis B virus (HBV), adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyoma virus BK, polyoma virus JC, measles virus, rubella virus, human T cell leukemia virus I, human T cell leukemia virus II, *Leishmania, Toxoplasma, Trypanosoma, Plasmodium, Schistosoma,* and *Encephalitozoon* or
wherein the chronic infection is not a latent infection.

15. The engineered T cells for use according to any one of claims 11-13,
wherein the chronic immune disorder is cancer, preferably wherein the cancer is a hematological cancer or a solid cancer, preferably
wherein the solid cancer is selected from the group consisting of lung cancer, non-small cell lung cancer (NSCLC), skin cancer, melanoma, cervical cancer, uterine cancer, ovarian cancer, breast cancer, pancreatic cancer, stomach cancer, esophageal cancer, colorectal cancer, liver cancer, prostate cancer, kidney cancer, bladder cancer, head and neck cancer, sarcoma, lymphoma, and brain cancer.

## Patentansprüche

1. Gentechnisch veränderte Säugetier-T-Zelle, umfassend eine genomische Region, die 1) die Genexpression von *pdcd1* reguliert und 2) innerhalb des Genoms einer erschöpften CD8+-T-Zelle aus dem Säugetier selektiv chromatinzugänglich ist, wobei die genomische Region genetisch modifiziert ist, um die Region zu deletieren, die der Nukleinsäuresequenz von SEQ ID NO: 12 entspricht, bei der es sich um tctccccaag ggagccattt ctttcaccct ttgtctctgc tctttctcct cacacttttg tttttgttat ggtctgacac cccccccccc ccaatctctc acccccagta gctgtggccg ttttgtccat gccagccagc tatttcatat cgttgctgta acgtgcttgc cagtcactga cacagagaag tgacttggct caaggatgta ctgaccacat aacctgtttt gttctgtacg
ctctgaatgt tctgtatgag gtttgctaat cttaagaaat tccacaaagc tttacgcagg acccatcaaa tcaaacgtca atatgaactg ttatgtctaa aacggcttgg gtcagaccac cgggcaacct ttcctgtgcc tacgtctgag ctcactgtgg tttttgtggt tgacactgaa aaatgctact aataagccaa aaaatatgat tataatatgc atgttctgtt atctcaatgt
tctgaaattc ttctgttcac cacccaacca ccaccactct taccttactc agaaccaatc agcttaaagg ttaactgata atatttcacc cagtaaacca acttctcccc tctttaccct ttaaactttt gagcttttct catataaaat gcctacactg agagcagact agtaccatcg ccaggctcct gcagtccctt ctgtggtcct agatgcccag cactatggtg tgcattcaat
aaagtattct tgcttaactg agatcagggt ttgtttggtt tgaggggtga ctccctgaac c, handelt, die -23,8kb stromaufwärts von der Transkriptionsstartstelle von *pdcd1* liegt, und die genetische Modifikation die Expression von *pdcd1* verringert.

2. *In vitro-* oder *ex vivo*-Verfahren zum gentechnischen Verändern einer Säugetier-T-Zelle, um die Expression von *pdcd1* in der Säugetier-T-Zelle herunterzuregulieren, wobei das Verfahren das Deletieren der Region umfasst, die der Nukleinsäuresequenz von SEQ ID NO: 12 entspricht, bei der es sich um tctccccaag ggagccattt ctttcaccct ttgtctctgc tctttctcct cacacttttg tttttgttat ggtctgacac cccccccccc ccaatctctc acccccagta gctgtggccg ttttgtccat gccagccagc tatttcatat cgttgctgta acgtgcttgc cagtcactga cacagagaag tgacttggct caaggatgta ctgaccacat aacctgtttt gttctgtacg
ctctgaatgt tctgtatgag gtttgctaat cttaagaaat tccacaaagc tttacgcagg acccatcaaa tcaaacgtca atatgaactg ttatgtctaa aacggcttgg gtcagaccac cgggcaacct ttcctgtgcc tacgtctgag ctcactgtgg tttttgtggt tgacactgaa aaatgctact aataagccaa aaaatatgat tataatatgc atgttctgtt atctcaatgt
tctgaaattc ttctgttcac cacccaacca ccaccactct taccttactc agaaccaatc agcttaaagg ttaactgata atatttcacc cagtaaacca acttctcccc tctttaccct ttaaactttt gagcttttct catataaaat gcctacactg agagcagact agtaccatcg ccaggctcct gcagtccctt ctgtggtcct agatgcccag cactatggtg tgcattcaat
aaagtattct tgcttaactg agatcagggt ttgtttggtt tgaggggtga ctccctgaac c, handelt, die - 23,8kb stromaufwärts von der Transkriptionsstartstelle von *pdcd1* liegt, durch genetische Modifikation, und wobei die genetische Veränderung die Expression von *pdcd1* herab reguliert.

3. Gentechnisch veränderte T-Zelle nach Anspruch 1 oder Verfahren nach Anspruch 2, wobei die gentechnisch veränderte genomische Genexpressions-Regulationsregion zu einer mindestens 5%igen Herabregulierung der Expression von *pdcd1* in der Säugetier-T-Zelle führt, verglichen mit der Expression von *pdcd1* in demselben T-Zelltyp aus dem Säugetier ohne die gentechnisch veränderte genomische Genexpressions-Regulationsregion, oder/und wobei die Expression von *pdcd1* die Transkription oder Translation von *pdcd1* ist, oder/und wobei die genomische Genexpressions-Regulationsregion durch Genom-Editierung deletiert ist, wobei die Genom-Editierung gegebenenfalls konstitutiv oder induzierbar exprimiert wird, vorzugsweise
wobei die Genom-Editierung ausgewählt ist aus der Gruppe bestehend aus CRISPR-Cas9 RNA-gesteuerten konstruierten Nukleasen (RGENs), Zinkfingernukleasen (ZFNs), Transkriptionsaktivator-ähnlichen Effektoren (TALEs), Homing-Meganukleasen und homologer Rekombination.

4. Gentechnisch veränderte T-Zelle oder Verfahren nach einem der Ansprüche 1 bis 3, wobei das Säugetier ein Tiermodell für eine Immunstörung ist, gegebenenfalls wobei die Immunstörung eine chronische Immunstörung ist, vorzugsweise wobei das Tiermodell ein Mausmodell ist oder/und
wobei das Säugetier eine Maus oder ein Mensch ist, vorzugsweise
wobei das Säugetier ein Mensch ist, vorzugsweise
wobei der Mensch an einer Immunstörung leidet, gegebenenfalls wobei die Immunstörung eine chronische Immunstörung ist.

5. Gentechnisch veränderte T-Zelle oder Verfahren nach Anspruch 4, wobei die chronische Immunstörung eine chronische Infektion ist, vorzugsweise
wobei die Infektion durch einen Erreger verursacht wird, der ausgewählt ist aus der Gruppe bestehend aus dem menschlichen Immunschwächevirus (HIV), Hepatitis-C-Virus (HCV), Hepatitis-B-Virus (HBV), Adenovirus, Cytomegalovirus, Epstein-Barr-Virus, Herpes-Simplex-Virus 1, Herpes-Simplex-Virus 2, dem Humanen Herpesvirus 6, Varizella-Zoster-Virus, Hepatitis-B-Virus, Hepatitis-D-Virus, Papillomavirus, Parvovirus B19, Polyomavirus BK, Polyomavirus JC, Masernvirus, Rötelnvirus, dem Humanen T-Zell-Leukämievirus I, dem Humanen T-Zell-Leukämievirus II, *Leishmania, Toxoplasma, Trypanosoma, Plasmodium, Schistosoma* und *Encephalitozoon,* vorzugsweise
wobei es sich bei der chronischen Infektion nicht um eine latente Infektion handelt.

6. Gentechnisch veränderte T-Zelle oder Verfahren nach Anspruch 4, wobei die chronische Immunstörung ein Krebs ist, vorzugsweise
wobei der Krebs ein hämatologischer Krebs oder ein solider Krebs ist, vorzugsweise wobei der solide Krebs ausgewählt ist aus der Gruppe bestehend aus Lungenkrebs, nicht-kleinzelligem Lungenkrebs (NSCLC), Hautkrebs, Melanom, Gebärmutterhalskrebs, Gebärmutterkrebs, Eierstockkrebs, Brustkrebs, Bauchspeicheldrüsenkrebs, Magenkrebs, Speiseröhrenkrebs, kolorektalem Krebs, Leberkrebs, Prostatakrebs, Nierenkrebs, Blasenkrebs, Kopf- und Halskrebs, Sarkom, Lymphom und Gehirnkrebs.

7. Gentechnisch veränderte T-Zelle oder Verfahren nach einem der Ansprüche 1-6, wobei die erschöpfte CD8+ T-Zelle einen T-Zell-Erschöpfungs-Biomarker exprimiert, der ausgewählt ist aus der Gruppe bestehend aus einem Checkpoint-Inhibitor, PD-1 (Pdcd1), TIM-3 (Havcr2), LAG-3 (Lag3), CTLA-4 (Ctla4), 2B4 (CD244), CD39 (Entpdl), CD160, Eomesodermin (Eomes), T-BET (Tbx21), BATF, BLIMP-1 (Prdml), NFATC1, NR4A2, MAFB, OCT-2 (Pou2f2), Foxpl, Retinsäurerezeptor alpha (Rara), und Kombinationen davon, vorzugsweise wobei die nicht erschöpfte CD8+ T-Zelle eine naive, funktionelle Effektor- oder Gedächtniszelle ist.

8. Gentechnisch veränderte T-Zelle oder Verfahren nach einem der Ansprüche 1 bis 7, wobei die T-Zelle eine primäre T-Zelle ist, die aus dem Säugetier isoliert werden kann, gentechnisch verändert werden kann und dem Säugetier zurückgegeben werden kann, oder
wobei die T-Zelle dem Säugetier verabreicht werden kann oder *in vitro* gezüchtet wird.

9. Verfahren nach einem der Ansprüche 2 bis 8, wobei die Erschöpfung in einer nicht erschöpften CD8+ T-Zelle verhindert wird, vorzugsweise
wobei die nicht erschöpfte CD8+ T-Zelle eine naive, funktionelle Effektor- oder Gedächtniszelle ist, oder/und
ferner umfassend die Formulierung der gentechnisch veränderten, nicht erschöpften CD8+ T-Zelle in einer Form, die einem Individuum verabreicht werden kann.

10. Verfahren nach einem der Ansprüche 2 bis 8, wobei die Erschöpfung der CD8+ T-Zellen in einer erschöpften CD8+ T-Zelle umgekehrt wird, vorzugsweise
wobei die erschöpfte CD8+ T-Zelle einen T-Zell-Erschöpfungs-Biomarker exprimiert, der ausgewählt ist aus der Gruppe bestehend aus einem Checkpoint-Inhibitor, PD-1 (Pdcd1), TIM-3 (Havcr2), LAG-3 (Lag3), CTLA-4 (Ctla4), 2B4 (CD244), CD39 (Entpdl), CD160, Eomesodermin (Eomes), T-BET (Tbx21), BATF, BLIMP-1 (Prdml), NFATC1, NR4A2, MAFB, OCT-2 (Pou2f2), Foxpl, Retinsäure-Rezeptor alpha (Rara) und Kombinationen davon, vorzugsweise
ferner umfassend die Formulierung der gentechnisch veränderten, erschöpften CD8+ T-Zelle in einer Form, die einem Individuum verabreicht werden kann.

11. Gentechnisch veränderte T-Zellen nach einem der Ansprüche 1 bis 8 zur Verwendung bei der Behandlung einer Immunstörung bei dem Individuum, vorzugsweise
wobei die gentechnisch veränderten T-Zellen fokal oder systemisch verabreicht werden.

12. Gentechnisch veränderte T-Zellen zur Verwendung gemäß Anspruch 11, wobei die systemische Verabreichung intravenös, intramuskulär, intraperitoneal oder intraartikulär erfolgt oder wobei die dem Individuum verabreichten gentechnisch veränderten T-Zellen für das Individuum autolog, syngen, allogen oder xenogen sind oder/und
wobei die dem Individuum verabreichten gentechnisch veränderten T-Zellen in einer pharmazeutisch akzeptablen Formulierung verabreicht werden oder/und wobei die gentechnisch veränderten T-Zellen die mindestens 5%ige modulierte Expression von *pdcd1* nach der Verabreichung an das Individuum beibehalten oder/und
ferner umfassend die Verabreichung einer therapeutisch wirksamen Menge einer pharmazeutischen Zusammensetzung an das Individuum, die ein oder mehrere Mittel gegen eine Immunstörung umfasst, oder/und
ferner umfassend das Inkontaktbringen der CD8+-T-Zellen mit einem oder mehreren Mitteln, die die Erschöpfung der CD8+-T-Zellen verhindern oder umkehren, vorzugsweise
wobei es sich bei dem einen oder den mehreren Mitteln um einen Immun-Checkpoint-Inhibitor handelt, vorzugsweise wobei der Immun-Checkpoint ausgewählt ist aus der Gruppe bestehend aus PD-1, PD-L1, PD-L2, LAG-3, TIM-1, CTLA-4, VISTA, B7-H2, B7-H3, B7-H4, B7-H6, 2B4, ICOS, HVEM, CD160, gp49B, PIR-B, KIR-Rezeptoren, TIM-1, TIM-4, BTLA, SIRPalpha (CD47), CD48, 2B4 (CD244), B7.1, B7.2, ILT-2, ILT-4, TIGIT und A2aR.

13. Gentechnisch veränderte T-Zellen zur Verwendung nach Anspruch 11 oder 12, wobei das Individuum ein Tiermodell einer Immunstörung ist, optional wobei die Immunstörung eine chronische Immunstörung ist, vorzugsweise
wobei das Tiermodell ein Mausmodell ist, oder/und
wobei das Individuum eine Maus oder ein Mensch ist, oder
wobei das Individuum ein Mensch ist, vorzugsweise
wobei der Mensch an einer Immunstörung leidet, gegebenenfalls wobei die Immunstörung eine chronische Immunstörung ist.

14. Gentechnisch veränderte T-Zellen zur Verwendung nach einem der Ansprüche 11 bis 13, wobei die chronische Immunstörung eine chronische Infektion ist, vorzugsweise wobei die Infektion durch einen Erreger verursacht wird, der ausgewählt ist aus der Gruppe bestehend aus dem menschlichen Immunschwächevirus (HIV), Hepatitis-C-Virus (HCV), Hepatitis-B-Virus (HBV), Adenovirus, Cytomegalovirus, Epstein-Barr-Virus, Herpes-Simplex-Virus 1, Herpes-Simplex-Virus 2, dem Humanen Herpesvirus 6, Varizella-Zoster-Virus, Hepatitis-B-Virus, Hepatitis-D-Virus, Papillomavirus, Parvovirus B19, Polyomavirus BK, Polyomavirus JC, Masernvirus, Rötelnvirus, dem Humanen T-Zell-Leukämievirus I, dem Humanen T-Zell-Leukämievirus II, *Leishmania, Toxoplasma, Trypanosoma, Plasmodium, Schistosoma* und *Encephalitozoon,* oder
wobei es sich bei der chronischen Infektion nicht um eine latente Infektion handelt.

15. Gentechnisch veränderte T-Zellen zur Verwendung nach einem der Ansprüche 11 bis 13, wobei die chronische Immunstörung ein Krebs ist, vorzugsweise wobei der Krebs ein hämatologischer Krebs oder ein solider Krebs ist, vorzugsweise
wobei der solide Krebs ausgewählt ist aus der Gruppe bestehend aus Lungenkrebs, nicht-kleinzelligem Lungenkrebs (NSCLC), Hautkrebs, Melanom, Gebärmutterhalskrebs, Gebärmutterkrebs, Eierstockkrebs, Brustkrebs, Bauchspeicheldrüsenkrebs, Magenkrebs, Speiseröhrenkrebs, kolorektalem Krebs, Leberkrebs, Prostatakrebs, Nierenkrebs, Blasenkrebs, Kopf- und Halskrebs, Sarkom, Lymphom und Gehirnkrebs.

## Revendications

1. Lymphocyte T de mammifère modifiée comprenant une région génomique qui 1) régule l'expression génique de *pdcd1* et 2) est sélectivement accessible à la chromatine dans le génome d'un lymphocyte T CD8+ épuisé du mammifère, où la région génomique est génétiquement modifiée pour supprimer la région correspondant à la séquence d'acide nucléique de SEQ ID NO : 12, qui est tctccccaag ggagccattt ctttcaccct ttgtctctgc tctttctcct cacacttttg tttttgttat ggtctgacac cccccccccc ccaatctctc acccccagta gctgtggccg ttttgtccat gccagccagc tatttcatat cgttgctgta acgtgcttgc cagtcactga cacagagaag tgacttggct caaggatgta ctgaccacat aacctgtttt gttctgtacg
ctctgaatgt tctgtatgag gtttgctaat cttaagaaat tccacaaagc tttacgcagg acccatcaaa tcaaacgtca atatgaactg ttatgtctaa aacggcttgg gtcagaccac cgggcaacct ttcctgtgcc tacgtctgag ctcactgtgg tttttgtggt tgacactgaa aaatgctact aataagccaa aaaatatgat tataatatgc atgttctgtt atctcaatgt
tctgaaattc ttctgttcac cacccaacca ccaccactct taccttactc agaaccaatc agcttaaagg ttaactgata atatttcacc cagtaaacca acttctcccc tctttaccct ttaaactttt gagcttttct catataaaat gcctacactg agagcagact agtaccatcg ccaggctcct gcagtccctt ctgtggtcct agatgcccag cactatggtg tgcattcaat
aaagtattct tgcttaactg agatcagggt ttgtttggtt tgaggggtga ctccctgaac c, qui est -23,8 kb en amont du site d'initiation de la transcription de *pdcd1* et la modification génétique diminue l'expression de *pdcd1.*

2. Méthode *in vitro* ou *ex vivo* d'ingénierie d'un lymphocyte T de mammifère pour réguler à la baisse l'expression de *pdcd1* dans le lymphocyte T de mammifère, la méthode comprenant la suppression de la région correspondant à la séquence d'acide nucléique de SEQ ID NO : 12, qui est tctccccaag ggagccattt ctttcaccct ttgtctctgc tctttctcct cacacttttg tttttgttat ggtctgacac cccccccccc ccaatctctc acccccagta gctgtggccg ttttgtccat gccagccagc tatttcatat cgttgctgta acgtgcttgc cagtcactga cacagagaag tgacttggct caaggatgta ctgaccacat aacctgtttt gttctgtacg
ctctgaatgt tctgtatgag gtttgctaat cttaagaaat tccacaaagc tttacgcagg acccatcaaa tcaaacgtca atatgaactg ttatgtctaa aacggcttgg gtcagaccac cgggcaacct ttcctgtgcc tacgtctgag ctcactgtgg tttttgtggt tgacactgaa aaatgctact aataagccaa aaaatatgat tataatatgc atgttctgtt atctcaatgt
tctgaaattc ttctgttcac cacccaacca ccaccactct taccttactc agaaccaatc agcttaaagg ttaactgata atatttcacc cagtaaacca acttctcccc tctttaccct ttaaactttt gagcttttct catataaaat gcctacactg agagcagact agtaccatcg ccaggctcct gcagtccctt ctgtggtcct agatgcccag cactatggtg tgcattcaat
aaagtattct tgcttaactg agatcagggt ttgtttggtt tgaggggtga ctccctgaac c, qui est -23,8 kb en amont du site d'initiation de la transcription de *pdcd1* par modification génétique et dans laquelle la modification génétique régule à la baisse l'expression de *pdcd1.*

3. Lymphocyte T modifié selon la revendication 1 ou méthode selon la revendication 2, où la région génomique régulatrice de l'expression génique modifiée entraîne une diminution d'au moins 5 % de l'expression de *pdcd1* dans le lymphocyte T de mammifère par rapport à l'expression de *pdcd1* dans le même type de lymphocyte T du mammifère sans la région génomique régulatrice de l'expression génique modifiée ou/et
où l'expression de *pdcd1* est la transcription ou la traduction de *pdcd1* ou/et
où la région génomique régulatrice de l'expression génique est supprimée par édition du génome, éventuellement où l'édition du génome est exprimée de manière constitutive ou inductible, de préférence
où l'édition du génome est choisie dans le groupe constitué des nucléases ingénierées guidées par l'ARN CRISPR-Cas9 (RGEN), des nucléases à doigt de zinc (ZFN), des effecteurs de type activateur de transcription (TALE), des méganucléases de homing et de la recombinaison homologue.

4. Lymphocyte T modifié ou méthode selon l'une quelconque des revendications 1 à 3, où le mammifère est un modèle animal d'un trouble immunitaire, éventuellement où le trouble immunitaire est un trouble immunitaire chronique, de préférence
où le modèle animal est un modèle de souris, ou/et
où le mammifère est une souris ou un humain, de préférence
où le mammifère est un humain, de préférence
où l'humain est atteint d'un trouble immunitaire, éventuellement où le trouble immunitaire est un trouble immunitaire chronique.

5. Lymphocyte T modifié ou méthode selon la revendication 4, où le trouble immunitaire chronique est une infection chronique, de préférence
où l'infection est causée par un agent choisi dans le groupe constitué par le virus de l'immunodéficience humaine (VIH), le virus de l'hépatite C (VHC), le virus de l'hépatite B (VHB), l'adénovirus, le cytomégalovirus, le virus d'Epstein-Barr, le virus de l'herpès simplex 1, le virus de l'herpès simplex 2, l'herpèsvirus humain 6, le virus de la varicelle et du zona, le virus de l'hépatite B, le virus de l'hépatite D, le papillomavirus, le parvovirus B19, le BK polyomavirus, le JC polyomavirus, le virus de la rougeole, le virus de la rubéole, le virus de la leucémie humaine à lymphocytes T I, le virus de la leucémie humaine à lymphocytes T II, *Leishmania, Toxoplasma, Trypanosoma, Plasmodium, Schistosoma* et *Encephalitozoon,* de préférence
où l'infection chronique n'est pas une infection latente.

6. Lymphocyte T modifié ou méthode selon la revendication 4, où le trouble immunitaire chronique est un cancer, de préférence
où le cancer est un cancer hématologique ou un cancer solide, de préférence
où le cancer solide est choisi dans le groupe constitué par le cancer du poumon, le cancer du poumon non à petites cellules (CPNPC), le cancer de la peau, le mélanome, le cancer du col de l'utérus, le cancer de l'utérus, le cancer de l'ovaire, le cancer du sein, le cancer du pancréas, le cancer de l'estomac, le cancer de l'oesophage, le cancer colorectal, le cancer du foie, le cancer de la prostate, le cancer du rein, le cancer de la vessie, le cancer de la tête et du cou, le sarcome, le lymphome, et le cancer du cerveau.

7. Lymphocyte T modifié ou méthode selon l'une quelconque des revendications 1 à 6, où le lymphocyte T CD8+ épuisé exprime un biomarqueur d'épuisement des lymphocytes T choisi dans le groupe constitué d'un inhibiteur de point de contrôle, PD-1 (Pdcd1), TIM-3 (Havcr2), LAG-3 (Lag3), CTLA-4 (Ctla4), 2B4 (CD244), CD39 (Entpdl), CD160, éomésodermine (Eomes), T-BET (Tbx21), BATF, BLIMP-1 (Prdml), NFATC1, NR4A2, MAFB, OCT-2 (Pou2f2), Foxpl, récepteur alpha de l'acide rétinoïque (Rara), et leurs combinaisons, de préférence où le lymphocyte T CD8+ non épuisé est une cellule naïve, une cellule effectrice fonctionnelle ou une cellule mémoire.

8. Lymphocyte T modifié ou méthode selon l'une quelconque des revendications 1 à 7, où le lymphocyte T est un lymphocyte T primaire isolable du mammifère, modifiable et renvoyable au mammifère, ou
où le lymphocyte T peut être administré au mammifère ou est cultivé *in vitro.*

9. Méthode selon l'une quelconque des revendications 2 à 8, où l'épuisement est empêché dans un lymphocyte T CD8+ non épuisé, de préférence
où le lymphocyte T CD8+ non épuisé est une cellule naïve, une cellule effectrice fonctionnelle ou une cellule mémoire ou/et
comprenant en outre la formulation du lymphocyte T CD8+ non épuisé modifié dans une forme administrable à un sujet.

10. Méthode selon l'une quelconque des revendications 2 à 8, où l'épuisement des lymphocytes T CD8+ est inversé dans un lymphocyte T CD8+ épuisé, de préférence
où le lymphocyte T CD8+ épuisé exprime un biomarqueur d'épuisement des lymphocytes T choisi dans le groupe constitué d'un inhibiteur de point de contrôle, PD-1 (Pdcd1), TIM-3 (Havcr2), LAG-3 (Lag3), CTLA-4 (Ctla4), 2B4 (CD244), CD39 (Entpdl), CD160, éomésodermine (Eomes), T-BET (Tbx21), BATF, BLIMP-1 (Prdml), NFATC1, NR4A2, MAFB, OCT-2 (Pou2f2), Foxpl, récepteur alpha de l'acide rétinoïque (Rara), et leurs combinaisons, de préférence
comprenant en outre la formulation du lymphocyte T CD8+ épuisé modifié dans une forme administrable à un sujet.

11. Lymphocytes T modifiés selon l'une quelconque des revendications 1 à 8 pour le traitement d'un trouble immunitaire chez le sujet, de préférence
où les lymphocytes T modifiés sont administrés de manière focale ou systémique.

12. Lymphocytes T modifiés à utiliser selon la revendication 11, où l'administration systémique est intraveineuse, intramusculaire, intrapéritonéale ou intra-articulaire, ou où les lymphocytes T modifiés administrés au sujet sont autologues, syngéniques, allogéniques ou xénogéniques pour le sujet ou/et
où les lymphocytes T modifiés administrés au sujet sont administrés dans une formulation pharmaceutiquement acceptable ou/et
où les lymphocytes T modifiés maintiennent l'expression modulée d'au moins 5 % de *pdcdl* après l'administration au sujet ou/et
comprenant en outre l'administration au sujet d'une quantité thérapeutiquement efficace d'une composition pharmaceutique comprenant un ou plusieurs agents anti-immunitaires ou/et
comprenant en outre la mise en contact des lymphocytes T CD8+ avec un ou plusieurs agents qui empêchent ou inversent l'épuisement des lymphocytes T CD8+, de préférence
où le ou les agents sont des inhibiteurs de points de contrôle immunitaire, de préférence
où le point de contrôle immunitaire est choisi dans le groupe constitué de PD-1, PD-L1, PD-L2, LAG-3, TIM-1, CTLA-4, VISTA, B7-H2, B7-H3, B7-H4, B7-H6, 2B4, ICOS, HVEM, CD160, gp49B, PIR-B, récepteurs de la famille KIR, TIM-1, TIM-4, BTLA, SIRPalpha (CD47), CD48, 2B4 (CD244), B7. 1, B7.2, ILT-2, ILT-4, TIGIT et A2aR.

13. Lymphocytes T modifiés à utiliser selon la revendication 11 ou 12, où le sujet est un modèle animal d'un trouble immunitaire, éventuellement où le trouble immunitaire est un trouble immunitaire chronique, de préférence
où le modèle animal est un modèle de souris, ou/et
où le sujet est une souris ou un humain, ou
où le sujet est un humain, de préférence
où l'humain est atteint d'un trouble immunitaire, éventuellement où le trouble immunitaire est un trouble immunitaire chronique.

14. Lymphocytes T modifiés à utiliser selon l'une quelconque des revendications 11 à 13,
où le trouble immunitaire chronique est une infection chronique, de préférence
où l'infection est causée par un agent choisi dans le groupe constitué par le virus de l'immunodéficience humaine (VIH), le virus de l'hépatite C (VHC), le virus de l'hépatite B (VHB), l'adénovirus, le cytomégalovirus, le virus d'Epstein-Barr, le virus de l'herpès simplex 1, le virus de l'herpès simplex 2, l'herpèsvirus humain 6, le virus de la varicelle et du zona, le virus de l'hépatite B, le virus de l'hépatite D, le papillomavirus, le parvovirus B19, le BK polyomavirus, le JC polyomavirus, le virus de la rougeole, le virus de la rubéole, le virus de la leucémie humaine à lymphocytes T I, le virus de la leucémie humaine à lymphocytes T II, *Leishmania, Toxoplasma, Trypanosoma, Plasmodium, Schistosoma* et *Encephalitozoon,* ou où l'infection chronique n'est pas une infection latente.

15. Lymphocytes T modifiés à utiliser selon l'une quelconque des revendications 11 à 13, où le trouble immunitaire chronique est un cancer, de préférence où le cancer est un cancer hématologique ou un cancer solide, de préférence
où le cancer solide est choisi dans le groupe constitué par le cancer du poumon, le cancer du poumon non à petites cellules (CPNPC), le cancer de la peau, le mélanome, le cancer du col de l'utérus, le cancer de l'utérus, le cancer de l'ovaire, le cancer du sein, le cancer du pancréas, le cancer de l'estomac, le cancer de l'oesophage, le cancer colorectal, le cancer du foie, le cancer de la prostate, le cancer du rein, le cancer de la vessie, le cancer de la tête et du cou, le sarcome, le lymphome, et le cancer du cerveau.
